# EUROPEAN PATENT SPECIFICATION

(11) **EP 0 701 612 B1**
(45) Date of publication and mention of the grant of the patent: **21.01.1998**
(21) Application number: 94915562.6
(22) Date of filing: 29.04.1994
(51) Int. Cl.: C12N 15/31, C12N 15/70, C12N 15/74, A61K 39/02, C12N 5/10, C07K 14/00

(54) **IMMUNOGENIC FORMULATION OF OspC ANTIGEN VACCINES FOR THE PREVENTION AND TREATMENT OF LYME DISEASE AND RECOMBINANT METHODS FOR THE PREPARATION OF SUCH ANTIGENS**
OSPC-ANTIGEN-IMPFSTOFFE IMMUNOGENE ZUSAMMENSETZUNG ZUR VORBEUGUNG UND BEHANDLUNG VON LYME-KRANKHEIT UND REKOMBINANTE VERFAHREN ZUR HERSTELLUNG VON DERARTIGE ANTIGENE
FORMULATION IMMUNOGENE DE VACCINS A BASE D'ANTIGENES OspC, DESTINEE A LA PREVENTION ET AU TRAITEMENT DE LA MALADIE DE LYME ET PROCEDES DE RECOMBINAISON POUR LA PREPARATION DE CES ANTIGENES

(30) Priority: 29.04.1993 US 53863
(43) Date of publication of application: 20.03.1996
(73) Proprietor: IMMUNO Aktiengesellschaft, A-1221 Wien (AT)
(72) Inventor: LIVEY, Ian, A-1020 Wien (AT); CROWE, Brian, A-1180 Wien (AT); DORNER, Friedrich, A-1230 Wien (AT)
(74) Representative: Kolb, Helga, Dr. Dipl.-Chem.
(86) International application number: EP9401365
(87) International publication number: WO9425596

(56) References cited:
- EP-A- 0 522 560
- MEDICAL MICROBIOLOGY AND IMMUNOLOGY, vol. 182, no. 1 , March 1993, pages 37-50; S. JAURIS-HEIPKE ET AL: 'Genetic heterogenity of the genes coding for the outer surface protein C (OspC) and the flagellin of Borrelia burgdorferi'
- INFECTION AND IMMUNITY, vol. 61, no. 12 , December 1993 , WASHINGTON US, pages 5097-5105; STEVEN J. PADULA ET AL: 'Molecular characterization and expression of p23 (OspC) from a North American strain of Borrelia burgdorferi'

## Description

The present invention relates to the prevention and treatment of Lyme disease in mammals and in particular to immunogenic formulations comprising different serological forms of OspC to retard or prevent the development of Lyme disease. The invention also comprises recombinant methods for the preparation of novel antigens.

### Background of the Invention

Lyme disease or Lyme borreliosis are terms used to describe the diverse clinical symptoms associated with tick-borne spirochetal infections caused by Lyme Disease *Borrelia*. Common manifestations of Lyme disease include disorders affecting the skin [erythema migrans (EM) or acrodermatitis chronica atrophicans (ACA)], nervous system (neuroborreliosis), and joints (arthritis) but other organs and tissues may become infected and diseased. Lyme disease has a world-wide distribution and is the most prevalent tick-borne disease in both the United States and Europe. The range of clinical symptoms commonly associated with Lyme disease in Europe is broader than that in the United States, with skin and nervous system disorders being common in Europe but rare in the United States, whereas arthritis is more common in the United States than in Europe. The clinical symptoms in North America appear to be a subset of those observed in Europe.

Lyme disease is typically treated with antibiotics. Treatment may be delayed, however, due to the often complex clinical picture and the lack of widely available, reliable diagnostic tests. If the disease is allowed to proceed to a chronic condition, treatment with antibiotics is more difficult and is not always successful. Furthermore the prospect that permanent damage is induced is likely to be increased during the course of a prolonged infection. Accordingly, a vaccine to prevent Lyme disease is desirable.

Two antigens from Lyme disease *Borrelia* have been described that can protect against infection/disease by this organism as determined in animal models of Lyme disease. These antigens, OspA and OspC (or "pC"), therefore are likely candidates for inclusion in any vaccine designed to protect against Lyme disease. See Simon et al., European patent No. 418,827; Fikrig et al., Science 250: 553-56 (1990); Preac-Mursic et al., Infection 20: 342-49 (1992). OspA and OspC share many characteristics. Both are lipoproteins that are exposed at the cell-surface, (Howe et al., Science 227: 645-46 (1985) ; Bergstrom et al. Mol. Microbiol. 3: 479-486 (1989)), both are plasmid-encoded (Barbour et al., Science 237: 409-11 (1987); Marconi et al., J. Bacteriol. 175: 926-32 (1993)), the genes for these proteins are present in most strains (Barbour et al., J. Infect. Dis. 152: 478-84 (1985); Marconi et al., J. Bacteriol. 175: 926-32 (1993)), and both exist in multiple serologically distinct forms (Wilske et al., (1989)).

The existence of multiple, serologically distinct forms of these antigens is an obstacle to the development of an OspA and/or OspC vaccine to protect against most, if not all, forms of Lyme disease. For instance, it has been demonstrated by Fikrig et al., J. Immun. 148: 2256-60 (1992), that immunization with one serological form of OspA, such as recombinant OspA of strain N40, need not protect against a challenge with a strain expressing a different OspA, for example, strain 25015. Consequently, it is necessary to develop typing schemes to classify and group the different variants of the antigen (i.e., OspA and/or OspC) such that the optimal mixture of serologically distinct forms of the antigen(s) that are needed to give broad protection can be determined.

A serotyping system for OspA has been developed using a limited number of monoclonal antibodies as the typing tools and 7 serotypes of OspA have been described using this methodology. Wilske et al., Ann. N.Y. Acad. sci. 539: 126-43 (1988). Restriction fragment length polymorphism (RFLP) analysis of OspA genes from 55 different European and North American strains identified six distinct genogroups. Wallich et al., Infection and Immunity 60: 4856-66 (1992). OspA proteins from North American isolates seem to be reasonably uniform since twelve of fourteen OspA's belonged to OspA type I and two to OspA type III. By contrast, the OspA's from European isolates are much more heterogeneous and include representatives of OspA types I (18), II (17), IV (4) and V (1). Construction of a phylogenetic tree based on sequence data for twelve OspA proteins from individual strains of B. burgdorferi supports the findings of the RFLP analysis but sequence information from isolates from two of the six genogroups is still lacking. At present no typing system exists for OspC.

Another consideration when selecting the appropriate antigens for inclusion in a vaccine is whether they are derived from strains that are epidemiologically important for the disease. In the mid-1970's it was postulated that pathogenic bacteria arise from a limited number of clones of highly related bacteria that in some way have a selective advantage in causing disease. This clonal hypothesis has since been confirmed. See Achtman et al., J. Infect. Dis. 165: 53-68 (1992). Thus, it is highly likely that among the numerous strains of Lyme disease *Borrelia* found in nature, only a limited number of "clones" exist that are highly adapted to causing mammalian, and in particular human, disease. In developing a vaccine to protect against disease in mammals and hence also in humans, it is of paramount importance to identify disease associated clones so that efforts can be concentrated against them. Thus it is necessary to elucidate the population structure of the species Lyme disease *Borrelia* and identify disease associated clones.

To date, a number of methods have been used to resolve the population structure of Lyme disease Borrelia, including (A) RFLP analysis of genomic DNA or of specific genes (LeFebvre et al., J. Clin. Micriobiol. 27: 636-39 (1989); Marconi & Garon, J. Bacteriol 174: 241-44 (1992); Postic et al., Res. Micriobiol. 141: 465-75 (1990); Stahlhammar-Carlemalm et al., Zbl. Bak 274: 28-39 (1990); Adam et al., Infect. Immun. 549: 2579-85 (1991); Wallich et al., Infect. Immun. 60: 4856-66 (1992)), (B) DNA-DNA hybridization (LeFebvre et al., J. Clin. Micriobiol. 27: 636-39 (1989); Postic et al., Res. Micriobiol. 141: 465-75 (1990)), (C) analysis of 16S rRNA by hybridization to oligonucleotide probes (Marconi et al., J. Clin. Micriobiol 30: 628-32 (1992) or by sequencing (Adam et al., Infect. Immun. 59: 2579-85 (1991); Marconi & Garon, J. Bacteriol. 174: 241-44 (1992)), (D) fingerprinting by an arbitrarily primed polymerase chain reaction (Welsh et al., Int. J. System. Bacteriol. 42: 370-77 (1992)), (E) multi-locus enzyme electrophoresis (Boerlin et al., Infect. Immun. 60: 1677-83 (1992)) and (F) serotyping of isolates (Peter & Bretz, Zbl. Baktk. 277: 28-33 (1992)).

There is broad agreement between the results obtained by these different procedures. In general, it appears that Lyme disease Borrelia isolates can be divided into at least three major groups. Indeed, some investigators believe that the genetic distances between members of these groups is sufficient to merit differentiating them into three separate species: *B.burgdorferi* sensu stricto (type strain B31), *B.garinii* sp. nov. (type strain 20047) and a species designated *B.afzelii* or the "group VS461 *Borrelia."* See Baranton et al., Int. J. Syst. Bacteriol. 42: 378-383, 1992; Marconi & Garon, supra.

The significance of the existence of these different groups for vaccine development remains to be fully elucidated. It is clear from the data of Wallich et al., Infection & Immunity 60: 4856-66 (1992), that there is a strong association between the genogroup to which an isolate belongs and the type of OspA that is produced: isolates from the group containing strain B31 (genogroup AAA or *B.burgdorferi* sensu stricto) produce a type I OspA (all of thirty strains analyzed), isolates from the group containing strain 20047 (genogroup BBB or *B.garinii* sp. nov.) usually produce a type II (17/19) OspA but types V (1/19) and VI (3/39) were also noted, isolates from the clone containing strain B023 (genogroup BBA or group VS 461) produce a type IV OspA (4/4), the remaining two isolates (genogroup B, B/A, A) produce a type III OspA.

Lyme disease isolates from North America predominantly belong to one group (genogroup AAA or *B.burgdorferi* sensu stricto), represented by strain B31, and consequently produce a type I OspA. This suggests that a vaccine containing a type I OspA may be sufficient to protect against most isolates causing Lyme disease in North America at the present time. In Europe the picture is more complex, since all three major clones are found and there is correspondingly an increased diversity in the types of OspA present (genotypes I, II, IV, V, VI). Furthermore, OspA was found not to protect in two studies, conducted using Lyme disease isolates from Europe, which also demonstrated the utility of OspC as a protective antigen. See U.S. patent application No. 07/903,580; Preac-Mursic et al., Infection 20: 342-49 (1992).

It was not known heretofore whether OspC was clonally inherited, with specific types of OspC restricted to particular groups of Lyme disease isolates (that is, to *B.burgdorferi* sensu stricto, *B.garinii* sp. nov. or group VS461). As OspC is plasmid encoded, Marconi et al., J. Bacteriol. 175: 926-32 (1993), it was conceivable that there had been plasmid-mediated transfer of the OspC gene between the different species of Lyme disease isolates. If this were the case, then the different types of OspC which are known to exist but which have not been defined, would not necessarily be clonally inherited.

### Summary of the Invention

It is the object of the present invention to provide an effective OspC vaccine, with broad cross protective levels in relation to all strains, against Lyme disease in mammals, by selecting OspC formulations based on defined OspC families resolved by phenotypic typing (the OspC "serovar" typing) and RFLP typing analyses, and sequence analysis of a large variety of strains of worldwide origin.

Furthermore, the OspC gene has been discovered to be clonally inherited. Consequently, it now is possible to interpret the results of the OspC typing schemes in view of epidemiological information from a "Common Membrane Antigen Typing" (CMAT) scheme, described below, which can be used to elucidate the clonal population structure of Lyme disease Borrelia strains. By the same token, it also now is possible to select the most appropriate OspC vaccine formulations either (a) to enable one to design vaccines to protect against strains prevalent within defined geographical regions or (b) to protect specifically and preferentially against epidemiological important disease associated clones or clonal clusters of *B. burgdorferi.*

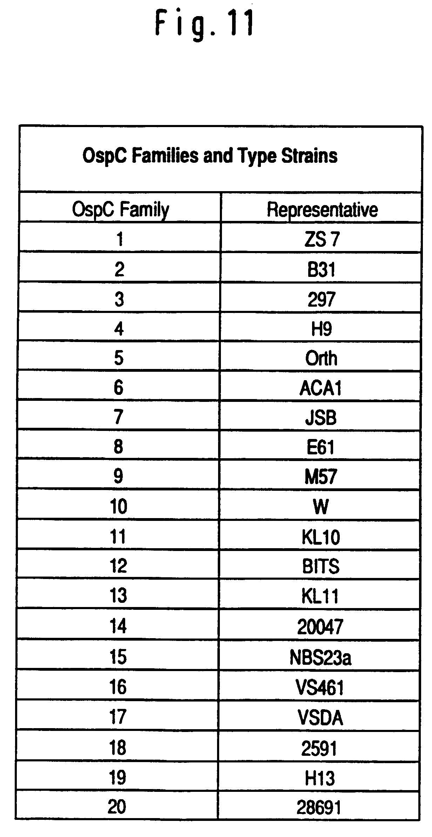

In accomplishing these and other objectives, there has been provided, in accordance with one aspect of the present invention, an immunogenic composition comprising
(a) an amount of material comprising (i) one or more OspC antigens of Lyme disease Borrelia substantially purified from each of the 20 currently recognized OspC families of Figure 11 or (ii) OspC variants or OspC mimetics of said OspC antigens, said OspC variants or OspC mimetics having a structure that is sufficiently similar to native OspC to induce the production of protective antibodies; and
(b) a physiologically-acceptable excipient therefore, wherein said amount is sufficient to elicit, in a mammal susceptible to Lyme borreliosis, an immune response that is protective against Lyme borreliosis.

According to a further embodiment, the above immunogenic composition comprises one or more, preferably two or more, OspC antigens of Lyme disease Borrelia of the 20 currently recognized OspC-families of Figure 11 or OspC variants or OspC mimetics of said antigens as defined above under (ii), and a physiologically-acceptable excipient as defined above under(b).

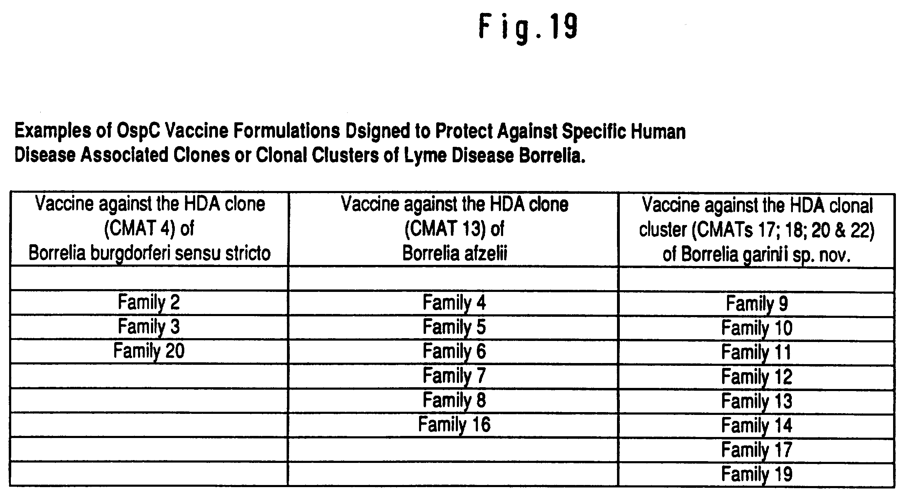

In accordance with another aspect of the present invention, an immunogenic composition is provided that comprises an amount of material comprising (i) one or more OspC antigens of Lyme disease Borrelia substantially purified from each of the OspC-families of Figure 19 expressed by the human disease associated (HDA) clones and clonal clusters or (ii) OspC variants or OspC mimetics of the OspC antigens, said OspC variants or OspC mimetics having a structure that is sufficiently similar to native OspC to induce the production of protective antibodies; and
(b) a physiologically-acceptable excipient therefore, wherein said amount is sufficient to elicit, in a mammal susceptible to Lyme borreliosis, an immune response that is protective against Lyme borreliosis.

As a preferred embodiment, the above immunogenic composition comprises one or more, preferably two or more, OspC antigens of the OspC-families of Figure 19, or OspC variants or OspC mimetics as defined above under (ii), and a physiologically-acceptable excipient as defined above under (b).

In a preferred embodiment, an immunogenic composition within the present invention is designed to protect against Lyme disease Borrelia strains prevalent within a particular geographic region, such as North America, Europe or Austria. As a preferred embodiment a combined OspA/OspC vaccine, which is superior to a vaccine formulated with either antigen alone is claimed.

The invention also comprises recombinant methods for the production of novel OspC antigens together with DNA sequences, expression vectors, and transformed host cells.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### Brief Description of the Drawings

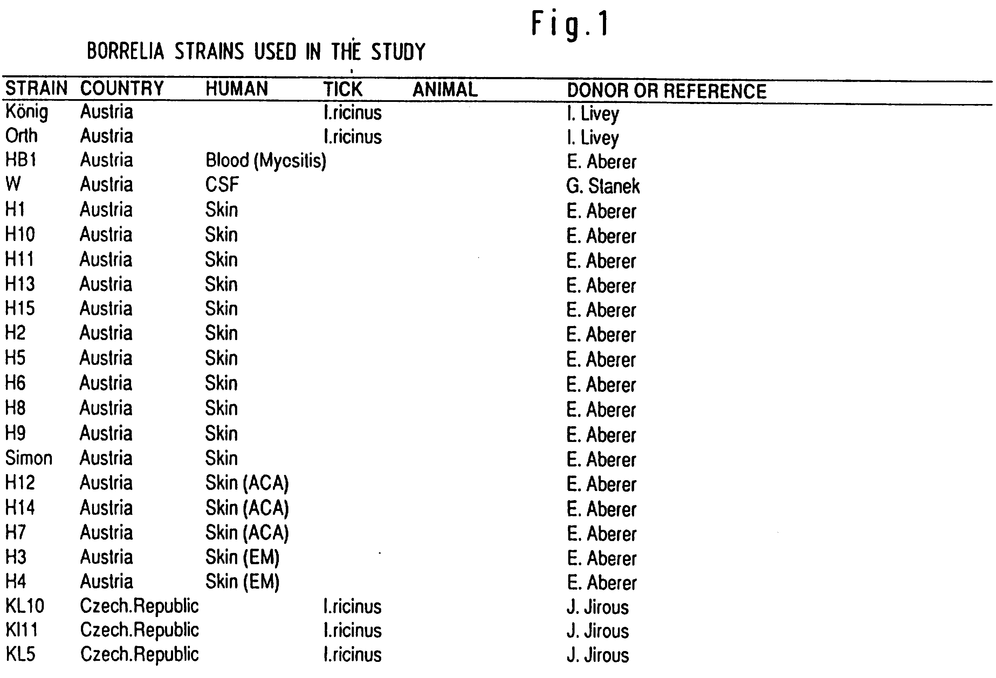

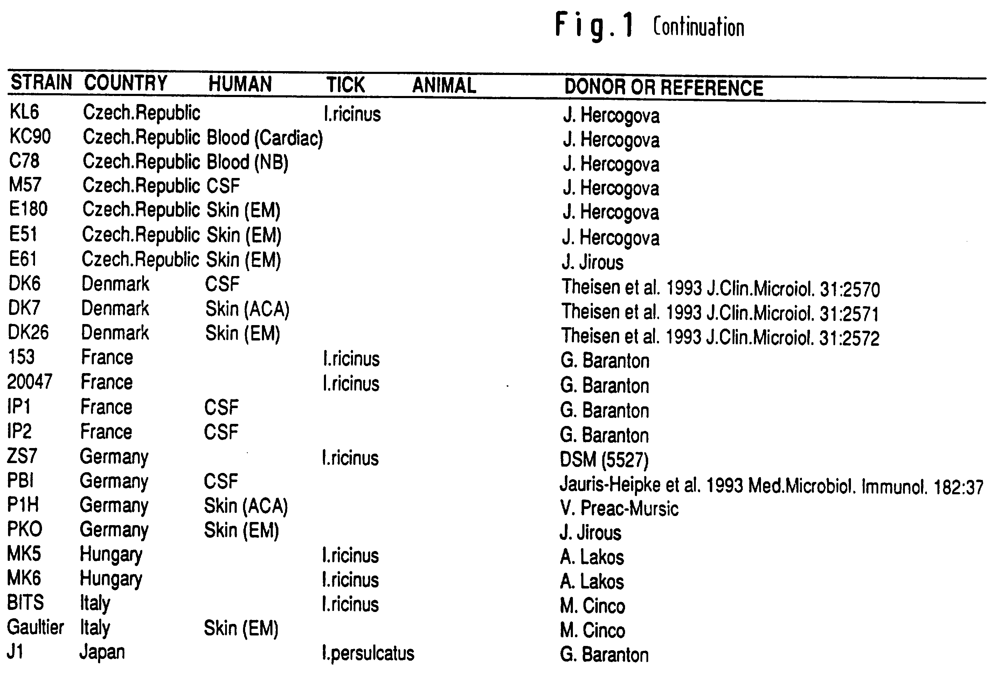

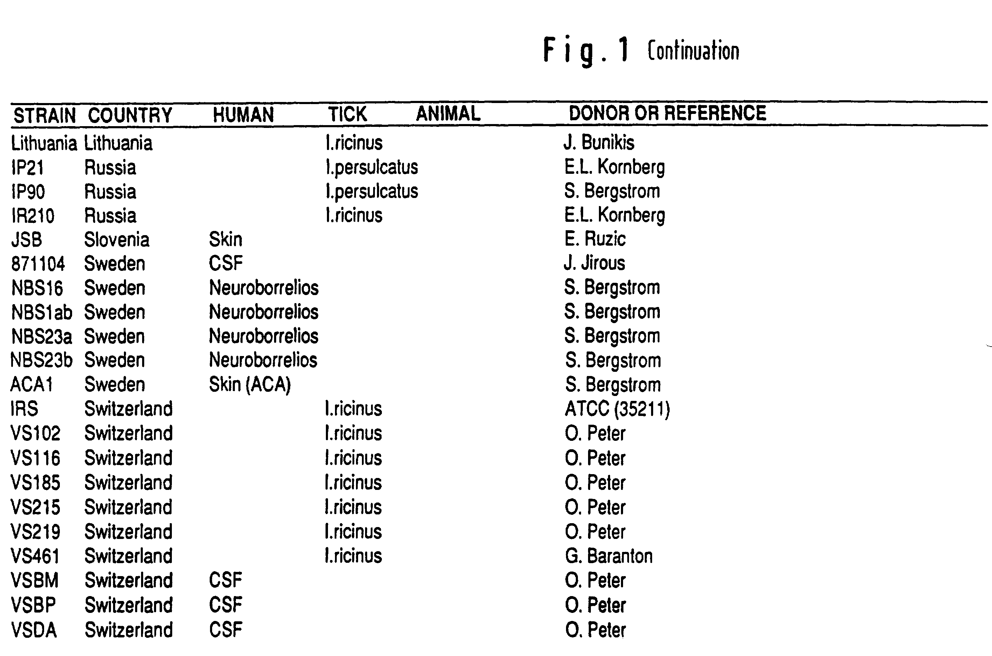

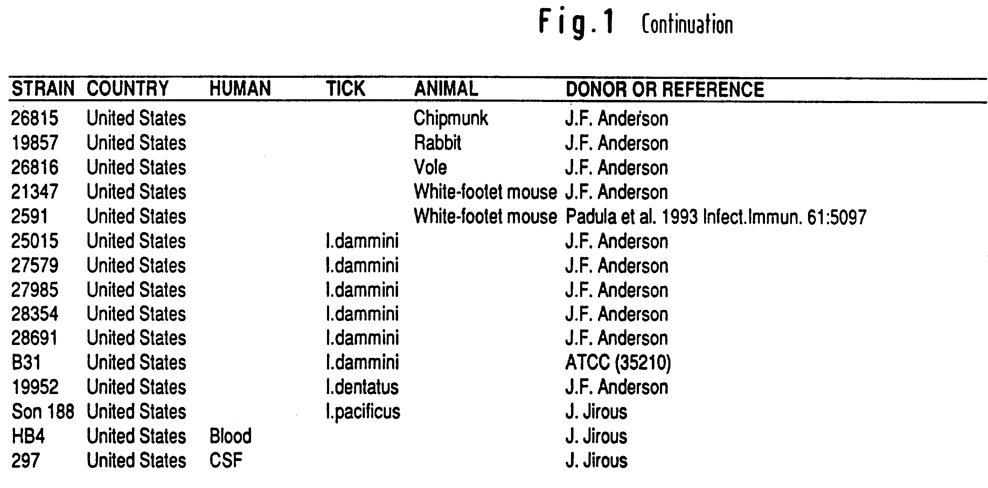

Figure 1 describes 77 *Borrelia* strains which were used in the experimental investigations. The country of origin and the biological source (i.e. human, tick or animal) from which these strains were isolated is described. The clinical material and disease syndrome from which the human isolates were obtained is shown too (Abbreviations: CSF, cerebrospinal fluid; ACA, acrodermatitis chronica atrophicans; EM, erythema migrans). The properties of 5 additional strains not experimentally studied are also included, since published information pertaining to these strains was used in some of the analyses.

Figure 2 lists the addresses of all strain contributors.

Figure 3 lists the monoclonal antibodies and their common membrane antigen specificities. The homologous reacting strain and the isotype of the monoclonal antibody are also indicated.

Figure 4 shows the individual scores and representative strains for each of the CMATs resolved by the CMAT typing scheme.

Figure 5 shows the dendrogram of the cluster analysis performed on the CMAT typing data. The frequency of occurrence of the CMATs among the strains tested also is indicated, as are the grouping of the individual CMATs into CMAT clusters (all CMATs with > 50% similarity) and CMAT subgroups (all CMATs with > 20% similarity).

Figure 6 gives the reaction pattern of a panel of 16 OspC-specific monoclonal antibodies with the various serovar type strains.

Figure 7 shows the sizes of the restriction fragments obtained when PCR amplified *ospC* genes (prepared as described in example 4 are digested with the enzymes *Dpn11, Dde1* and *Dra1.* The data presented shows the 35 unique patterns of restriction fragments (i.e. 35 *ospC* RFLP types) identified from an analysis of the restriction fragment data from the 82 strains listed in Type strains chosen to represent each of the *ospC* RFLP types are also given.

Figure 8 shows the Figure 1 aligned, partial nucleotide sequences of twenty-four *ospC* genes selected from strains belonging to *ospC* RFLP types 1-24.

Figure 8a shows the complete sequences of the novel ospC genes according to Figure 8 including the 3' end. Additionally Figure 8a includes the sequences for the ospC genes of strains H13 and 28691.

Figure 9 shows the aligned, partial amino acid sequences deduced from the nucleotide sequence data of Figure 8. The sequenced region corresponds to the first 92% of the mature OspC protein.

Figure 9a shows the complete amino acid sequences of the novel OspC antigens according to Figure 9 including the C-terminal. Additionally Figure 9a includes the sequences for the OspC antigens of strains H13 and 28691.

Figure 10 is a dendrogram of the OspC protein sequence data of Figure 9 showing the phylogenetic relationships between the sequences and the degree of sequence identity. This analysis has been used to assign the OspC proteins into OspC families. Members of an OspC family comprise related OspC sequences with >80% sequence identity. It is also shown that OspC proteins cluster in a species-specific manner indicating that the OspC protein is clonally inherited.

Figure 11 lists the 20 OspC families and indicates strains chosen as a representative of that family.

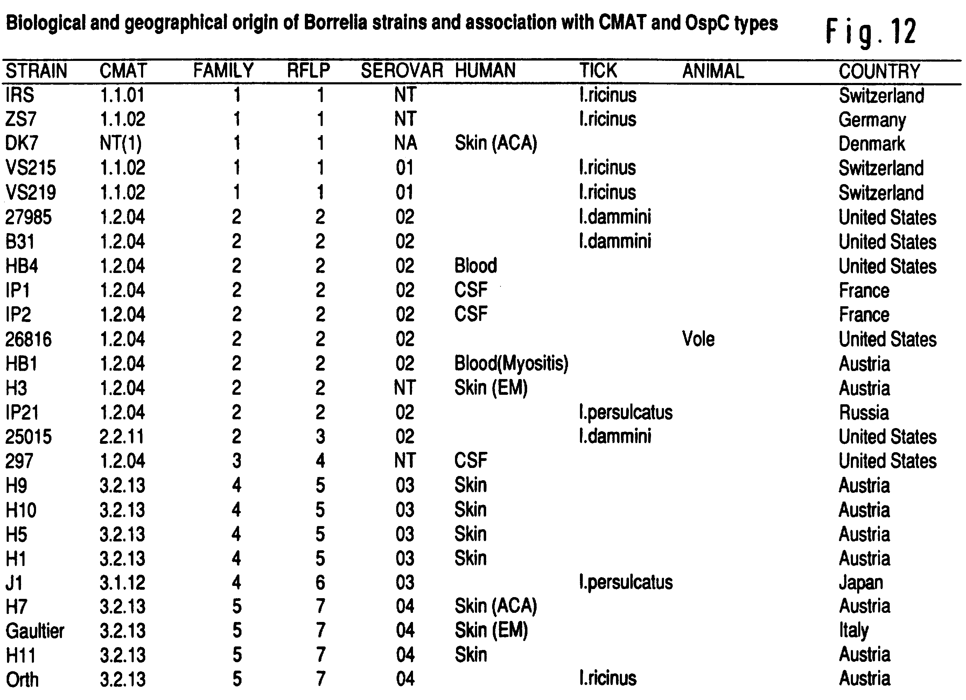

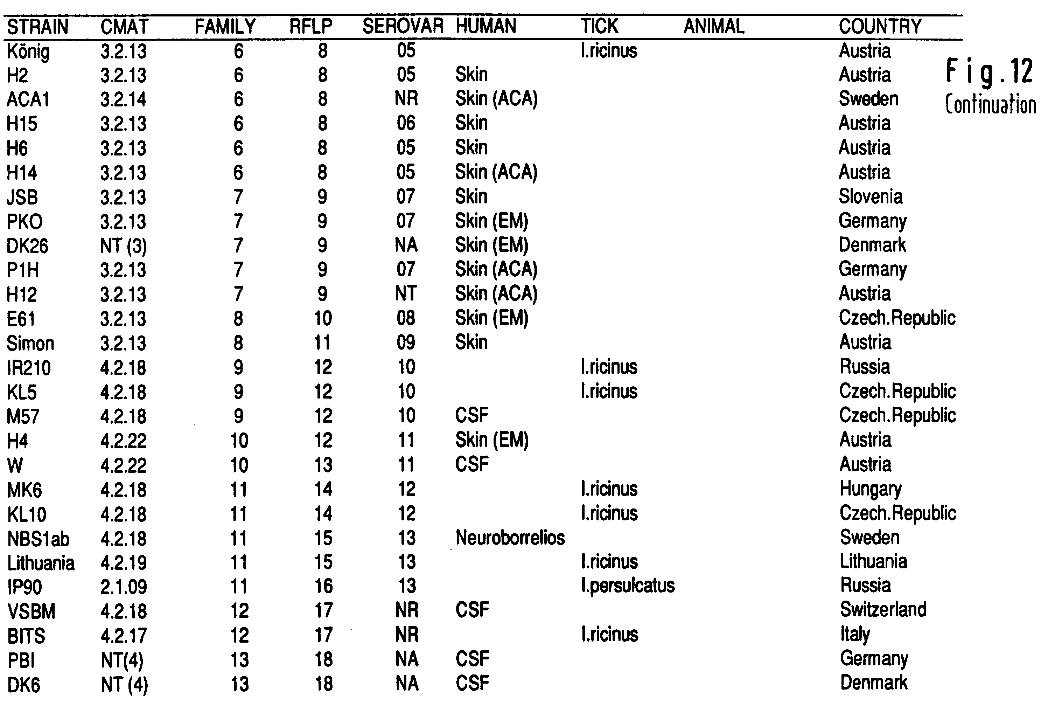

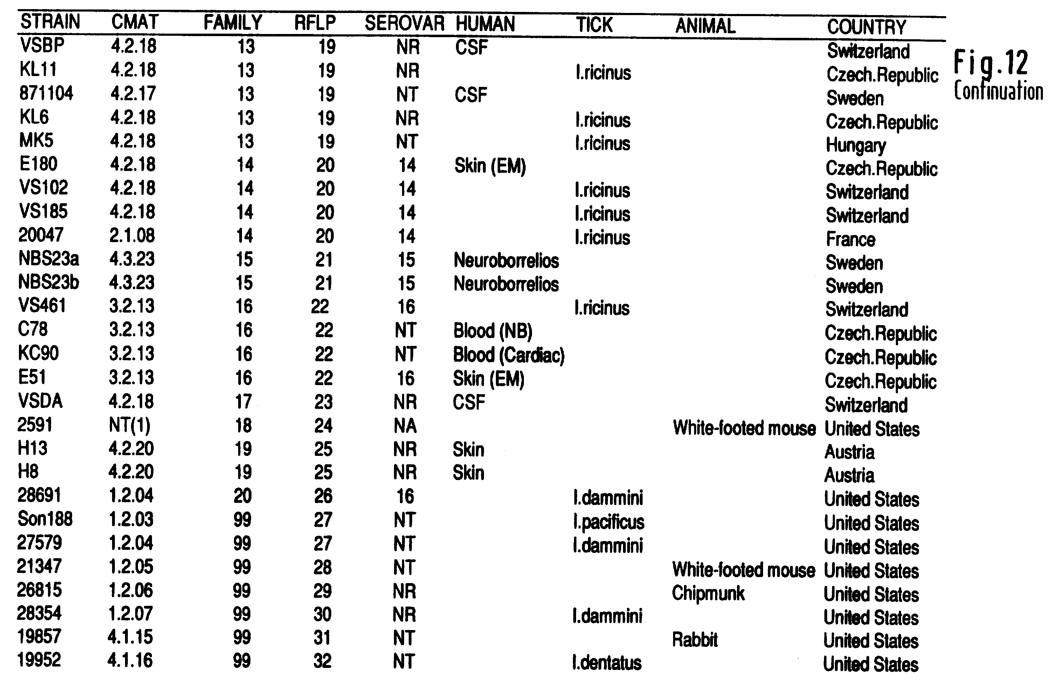

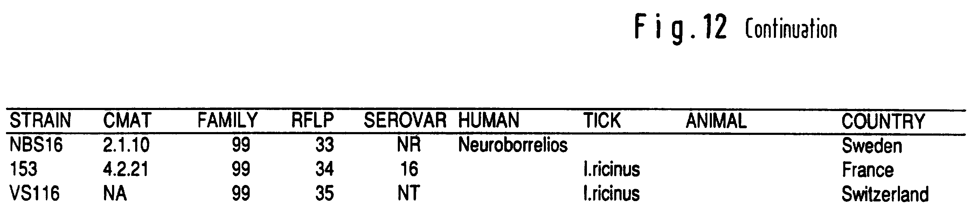

Figure 12 summarizes the results of the CMAT and OspC typing analyses of the 82 strains from Figure 1. The data are sorted by OspC family and RFLP-type to show the frequency with which strains belonging to a particular OspC family occur. Strains which have not been assigned an OspC family are designated 99. The biological and geographical origins of the strains is included to allow a comparison of these parameters with the OspC family. CMAT values have been assigned to 5 strains for which there was a published description but which were not tested (i.e. strains of *B.burgdorferi, B.afzelii* and *B.garinii* correspond to CMATs 1,3 and 4 respectively). OspC serovars have not been assigned to all strains; 5 strains were not available (NA), others were not tested (NT) since they expressed insufficient amounts of OspC for reliable typing and some were tested but were non-reactive (NR) with the panel of monoclonal antibodies.

Figure 13 lists the sequences of the mapped OspC epitopes together with the frequency of their occurrence among the strains analyzed. At the bottom of the table the monoclonals are grouped into categories according to the frequency with which they react with the seventy seven strains in the study.

Figure 14 shows the map of the generalized OspC protein marking the location of the epitopes of numerous BBM monoclonal antibodies indicated by their numbers.

Figure 15 shows the results of active immunization experiments using the gerbil model. Groups of animals were immunized with purified OspC protein variants of either the same (H7) or different OspC family (ZS7, PKO and W) to that of the challenge strain Orth. The results indicate that there is strong cross protection when one immunizes with a variant of the same family as that expressed by the challenge strain, but that there is little or no protection when one immunized with an OspC variant of a different OspC family to that of the challenge strain.

Figure 16 summarizes the OspC typing information and the distribution of human isolates among the various OspC types. The specificity and prevalence of OspC antibodies present in human Lyme disease sera form the Czech Republic is also shown. OspC antibody specificity was defined by testing against a panel of 18 different *Borrelia* strains representing 16 OspC families.

Figure 17 shows the yeast expression vector pPC-PP4 with the OspC coding sequence under transcriptional control of the methanol inducible AOX-1 promotor.

Figure 18 shows the results of active immunization experiments using the gerbil model. Animals were immunized with purified OspC protein derived from *B. burgdorferi* strain Orth and recombinantly produced from *P. pastoris* GS115/pPC-PP-4. The results indicate a strong protection with the *Borrelia* derived as well as with the Yeast-derived OspC protein.

Figure 19 shows examples of OspC vaccine formulations designed to protect against specific human disease associated clones or clonal clusters of Lyme disease Borrelia.

### Detailed Description of the Invention

By the serovar, restriction fragment length polymorphism (RFLP) and sequencing analyses described in greater detail below, it has been discovered that, despite the high degree of OspC protein heterogeneity across different Lyme disease isolates, OspC proteins can be grouped into a limited number of families on the basis of similarity in amino acid sequence, inter alia. In accordance with the present invention, the implications of this finding are realized in the design of vaccine formulations that provide a high level of cross-protectivity, relative to different strains, which has not been attained previously.

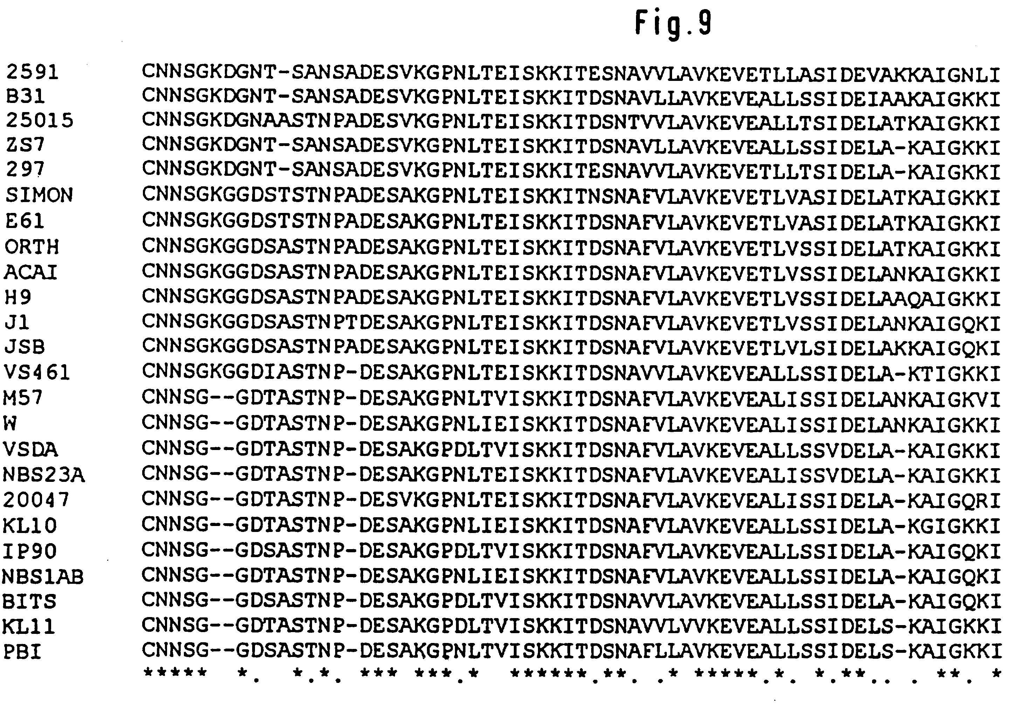

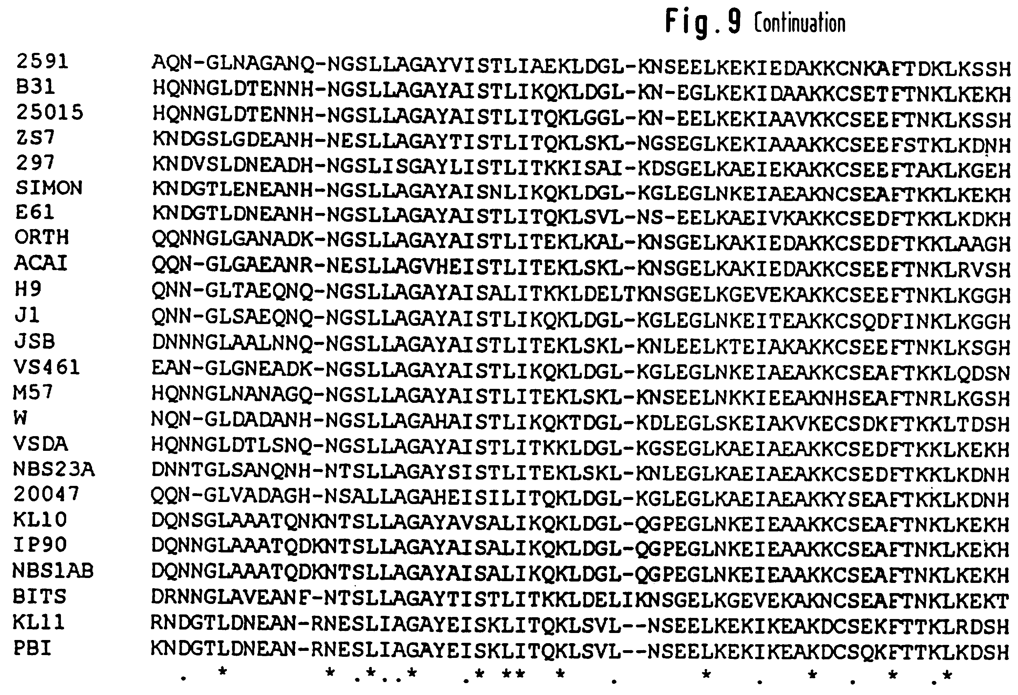

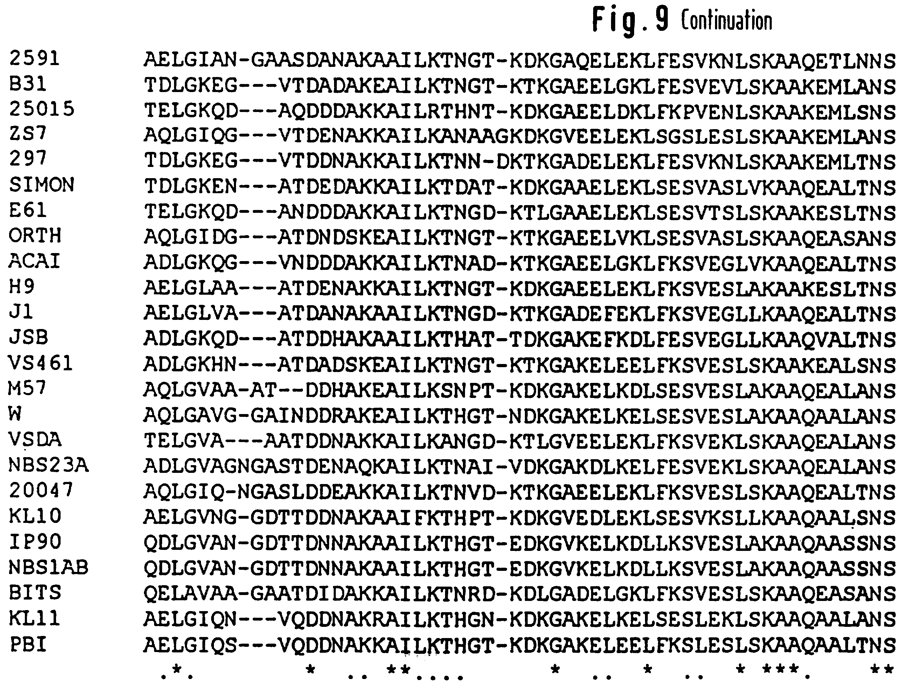

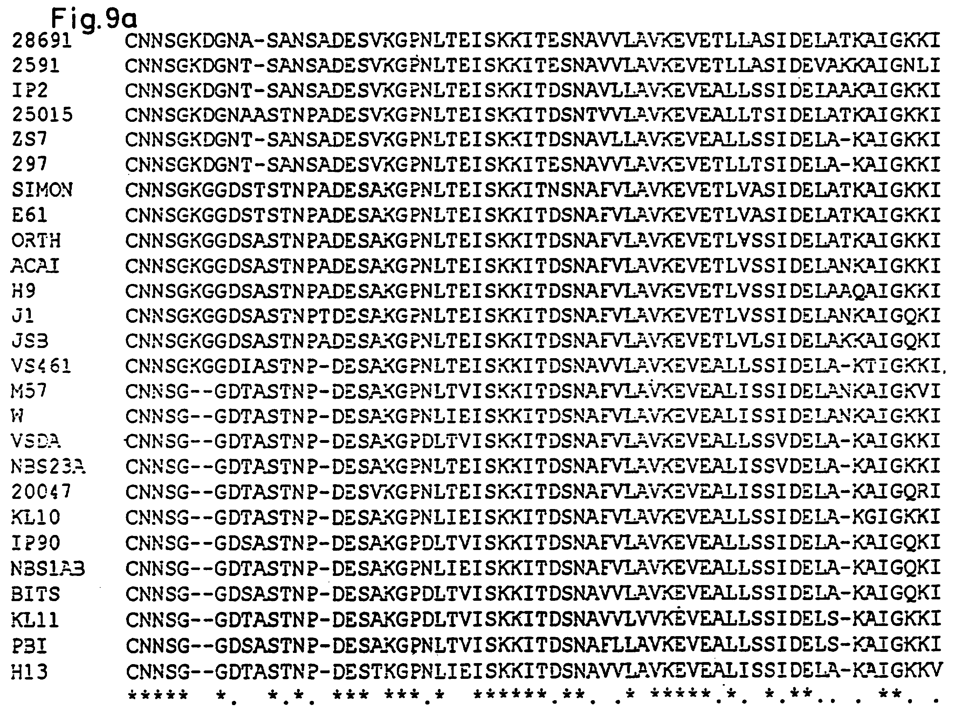

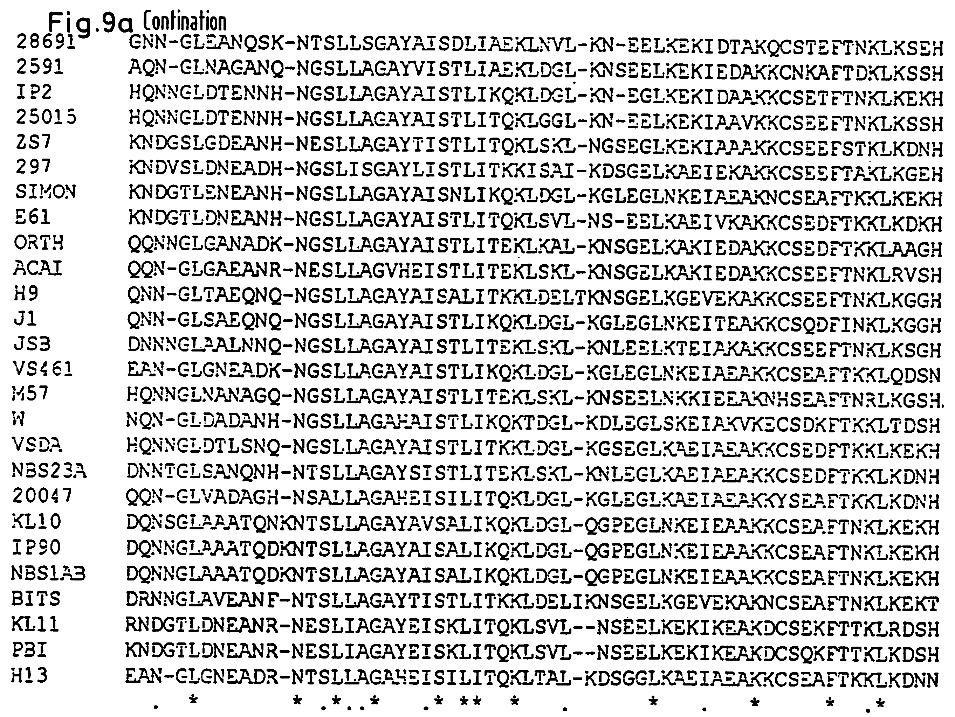

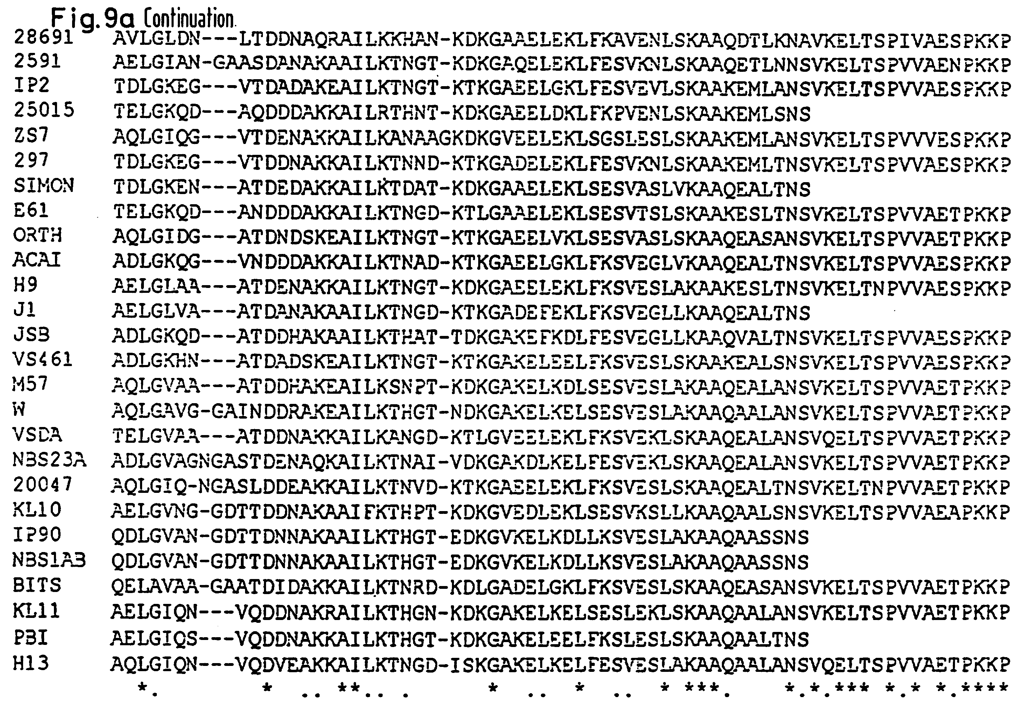

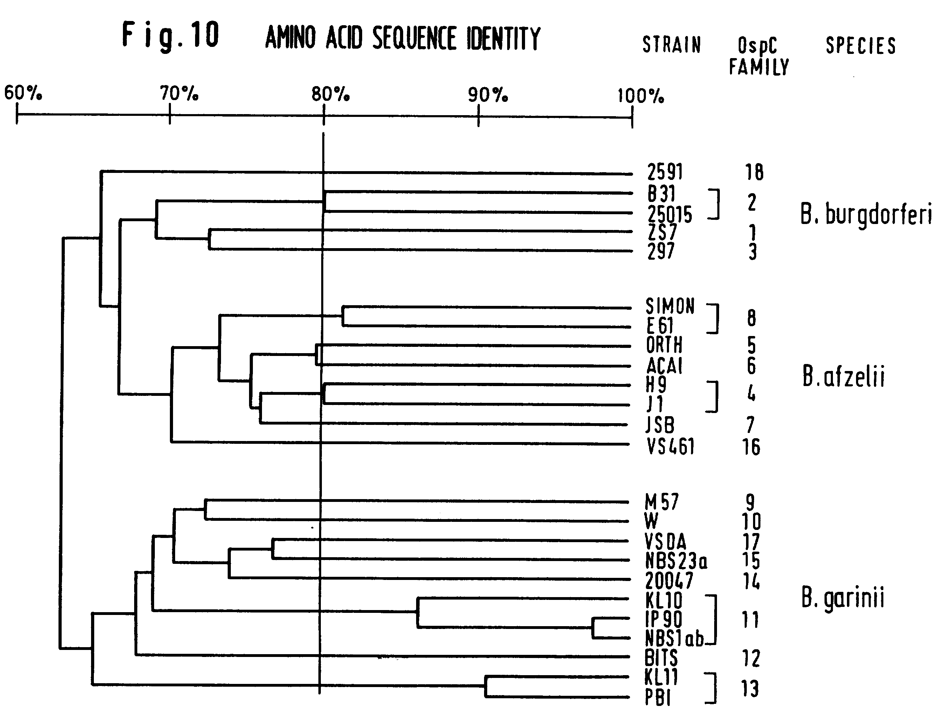

In order to achieve such cross-protection one must be able to predict the effectiveness of the components of a given vaccine in protecting against all possible disease-causing strains. Pursuant to the present invention, this problem is overcome by insuring that the heterogeneity of protective antigen components in a vaccine optimally reflects the heterogeneity found in nature. In particular, for the OspC protein this is achieved by formulating vaccines that contain representatives for all of the OspC families revealed by the sequence analysis described here. An example of such a formulation entails including in a vaccine the twenty OspC proteins that are representative of all of the aforementioned OspC families. An OspC family is defined as a group of OspC proteins that have more than 80% amino acid sequence identity over the first 92% of the mature OspC protein i.e. excluding the information for the 18aa leader sequence and the final 16aa as shown in Figures 9, 9a and 10.

The present invention thus relates in one aspect to an immunogenic composition comprising one or more OspC antigens substantially purified from each of the twenty OspC families which the present inventors have delineated for the first time. The use of twenty antigens, for example, is an improvement over the prospect of including all possible OspC variants found in nature (cf. 35 OspC RFLP types described here). In accordance with another aspect of the present invention, the formulation of a Lyme disease vaccine is simplified further by restricting the number of antigenic components, in a manner that does not reduce the protective efficacy of the vaccine, by the combined application of OspC typing data and epidemiological data. Exemplary of this approach, as described in greater detail below, are (A) the design of vaccine formulations for use in a particular geographic region, such as North America, Europe or a particular country such as Austria, and (B) the design of a vaccine that is targeted to protect specifically against only those clones, identified by CMAT analysis, which are associated with human disease. In one embodiment under rubric (A), a vaccine is formulated for use in North America and contains antigens representing only those OspC families observed for American strains, namely, families 2 and 3 (see Figure 12).

According to another embodiment said vaccine formulated for use in North America comprises strains from families 2, 3, 18 and 20.

In order to identify clones of Lyme disease *Borrelia,* a population structure analysis was carried out by CMAT analysis. A "CMAT (type)" is defined as a unique, nine-digit score resulting from the combined score of molecular weight variants of nine common membrane antigens detected, and in some cases differentially discerned, by a given set of monoclonal antibodies specific for these antigens. A "CMAT cluster" is a group of related CMAT (types) having at least 50% similarity in their CMAT score. A "CMAT group" is used here to denote a group of CMAT types having more than or equal to 20% similarity in their CMAT score. Consequently, a CMAT group may be comprised of several CMAT clusters which in turn may be comprised of several CMAT types.

A "clone" is defined as a CMAT type comprising more than one strain, or otherwise, a clone is a group of strains having the same CMAT type and thus are considered as arising from a common ancestral strain. A "clonal cluster" is a group of clones related at the CMAT cluster level (that is, where their CMAT types are more than 50% similar). A "human disease-associated clone" is a clone that, based on epidemiological and clinical data, can be shown to be associated frequently with human disease. Likewise, a "human disease-associated clonal cluster" is a clonal cluster that can be shown, from epidemiological and clinical data, to be associated frequently with human disease. Thus in an embodiment under rubric B (see above), an OspC vaccine is formulated against the human disease associated CMAT clones 1.2.4., 3.2.13, and the clonal cluster 4.2.17, 4.2.18, 4.2.20 and 4.2.22.

OspC is known to be a suitable immunogen for eliciting a protective immune response in animal models of Lyme disease when the challenge organism is the same Lyme disease isolate from which the OspC was derived. Due to the serological heterogeneity of OspC proteins among Lyme disease *Borrelia* strains, however, it was thought that immunization with OspC from one strain might not protect against infection with a wide range of Lyme disease *Borrelia* isolates and recognized a need to validate this assumption based upon cross-protection studies (Example 6). Based upon these studies, it became clear that an OspC based vaccine would have to contain several serologically distinct forms of OspC. A prerequisite to the formulation of such a multivalent OspC vaccine is knowledge of the degree of diversity among the different forms of OspC and of how these different forms are related. Accordingly, such information is applied, pursuant to the present invention, in the development of new vaccine formulations against Lyme disease *Borrelia.*

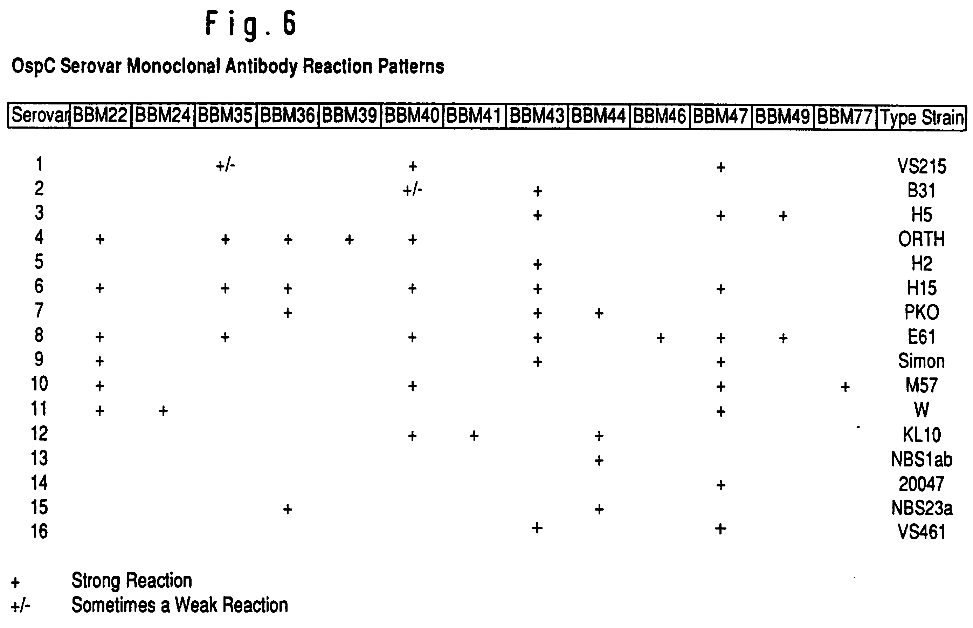

The first step towards acquiring the required information was the development of a monoclonal antibody based typing system (Example 1) for characterizing the OspC proteins from different Lyme disease *Borrelia* strains. In order to ensure that the widest range of OspC proteins would be analyzed a large number of Lyme disease *Borrelia* strains (i.e. 62 of the 82 strains depicted in Figure 1 were selected as producing sufficient OspC to allow a reliable characterization) from different geographical locations and isolated from humans (for example, skin, cerebrospinal fluid and blood), animal and ticks were studied. Another key aspect of this analysis was the use of a large number of OspC-specific monoclonal antibodies (25 in the present example) that had been produced not simply to one OspC protein but to six different OspC proteins, thereby increasing the diversity of OspC epitopes capable of being recognized and hence increasing the power to discriminate between different OspC proteins. The data collected in this analysis clearly showed that a large degree of serological heterogeneity exists among the OspC proteins from different sources. Examples of the reaction patterns of the 16 distinct types, or serovars, of OspC that were identified with a collection of 13 monoclonal antibodies are shown in Figure 6. In addition, 12 strains were non-typable as they did not react with any of the monoclonal antibodies.

Although highly effective, the typing of OspC by serological means was nevertheless incomplete, since it requires both that OspC is expressed as a major protein and that a set of antibodies with a wide range of specificities is available. It was clear from the results of the serovar analysis that the full-spectrum of antigenic diversity was not being detected with the monoclonal antibodies being used, even although they had been chosen to minimize this problem. Consequently, the heterogeneity of OspC was further studied by analysing the restriction fragment length polymorphism (RFLP) occurring within the *ospC* gene (Example 3). An analysis of the data from 82 strains (i.e. experimental data from all 77 strains in our culture collection plus information deduced from 5 published *ospC* sequences; see Figure 1) revealed the presence of 35 distinct RFLP *ospC* types. Although this method detects more variation than evident from the serovar typing, there is extremely good agreement between the results obtained with the two methods (Figure 12).

The classification of *Borrelia* strains into serovars and RFLP-types according to the OspC protein or gene that they possess, made it possible, pursuant to the present invention, to select for more detailed characterization a limited number of OspC variants which are representative of the population as a whole. Thus, a panel of 29 strains comprising one or more representatives from each of the most ubiquitious OspC types was selected, the OspC gene was amplified by PCR, and the nucleotide and deduced amino acid sequence determined (see Example 4). The amino acid sequence of the mature OspC protein (from cysteine 19; see U.S. Patent Application No. 07/903,580, previously incorporated by reference), less the last 16 amino acids, was used to determine the relationship between OspC proteins from the different OspC serovars /RFLP-types.

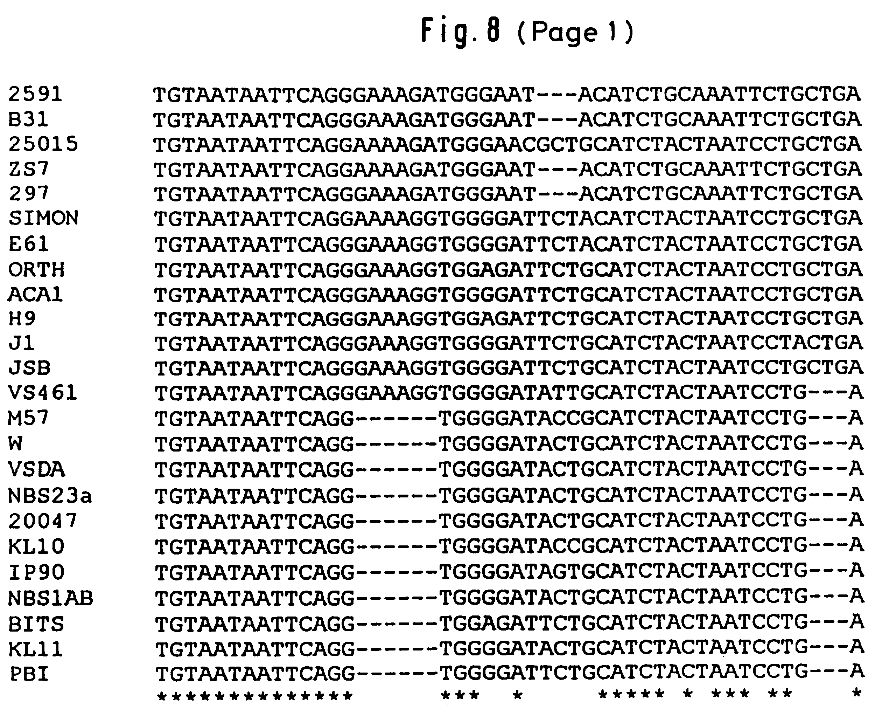

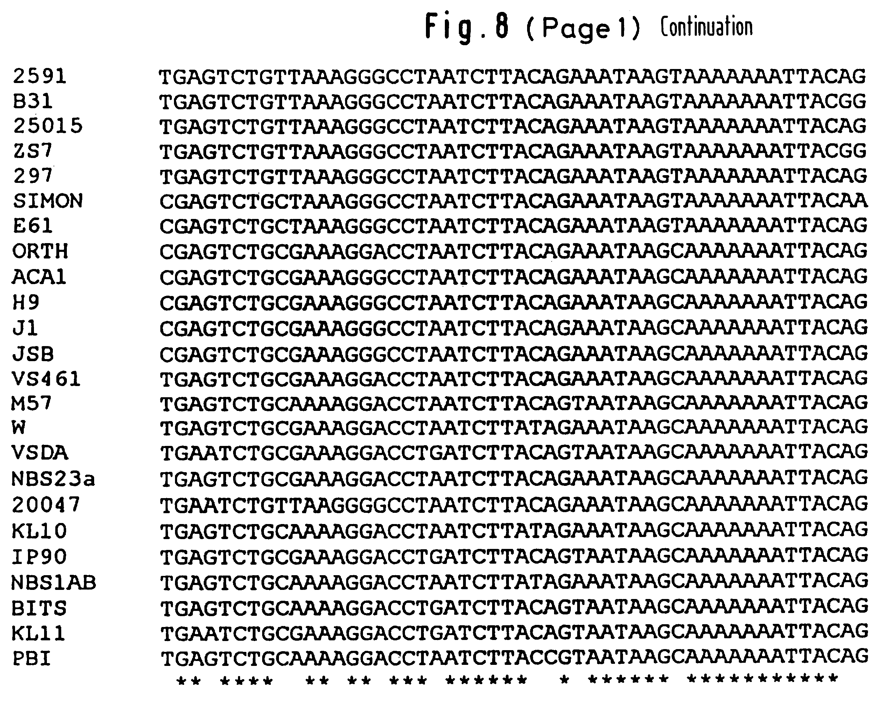

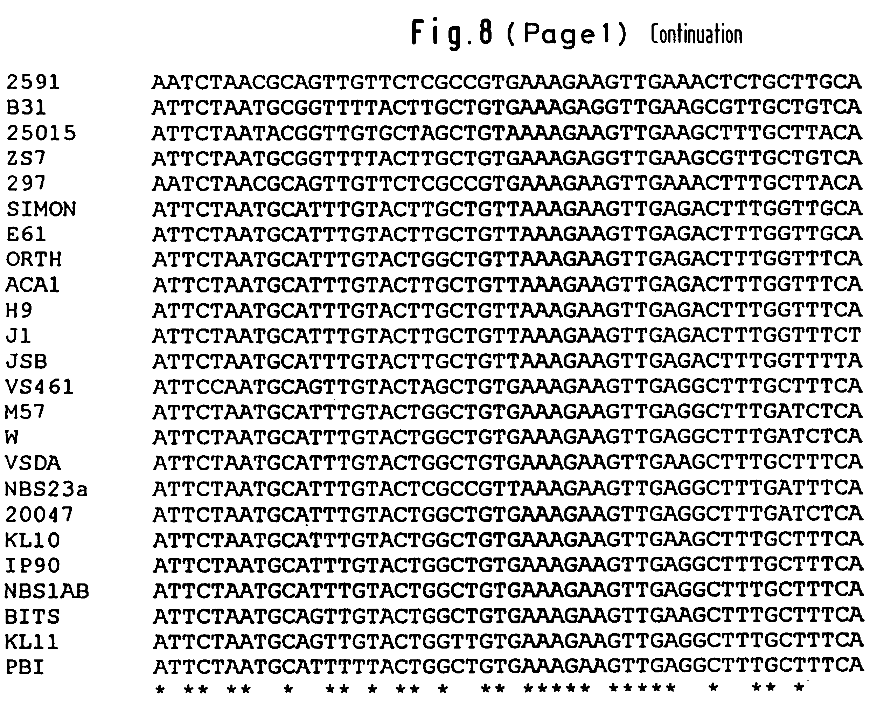

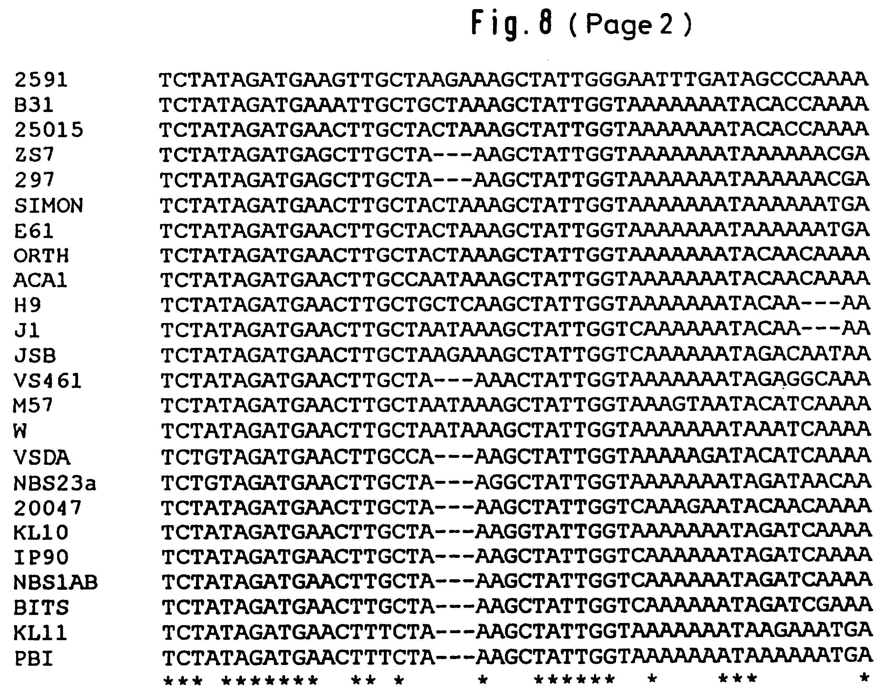

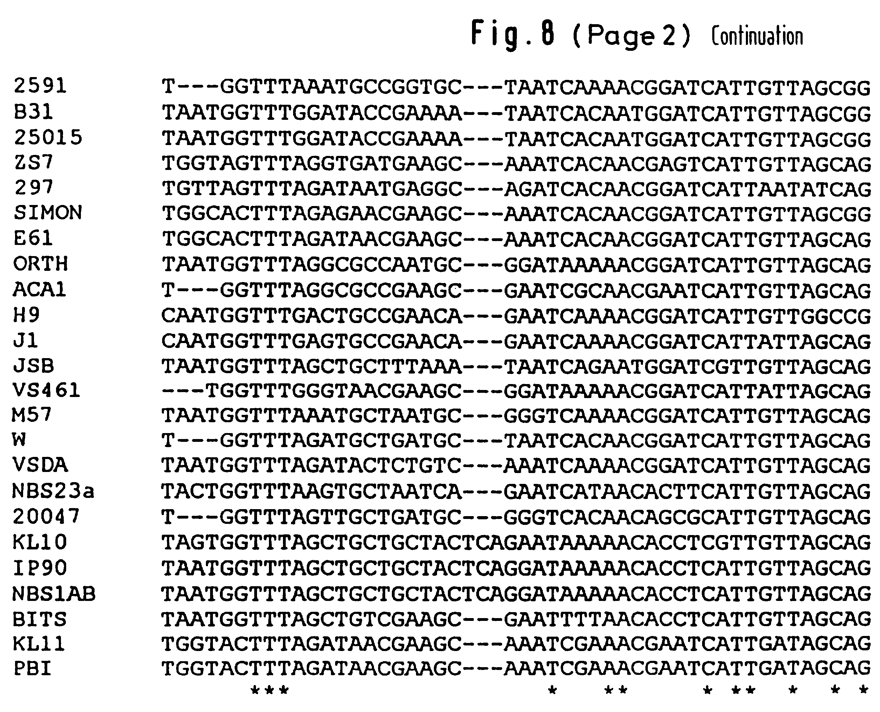

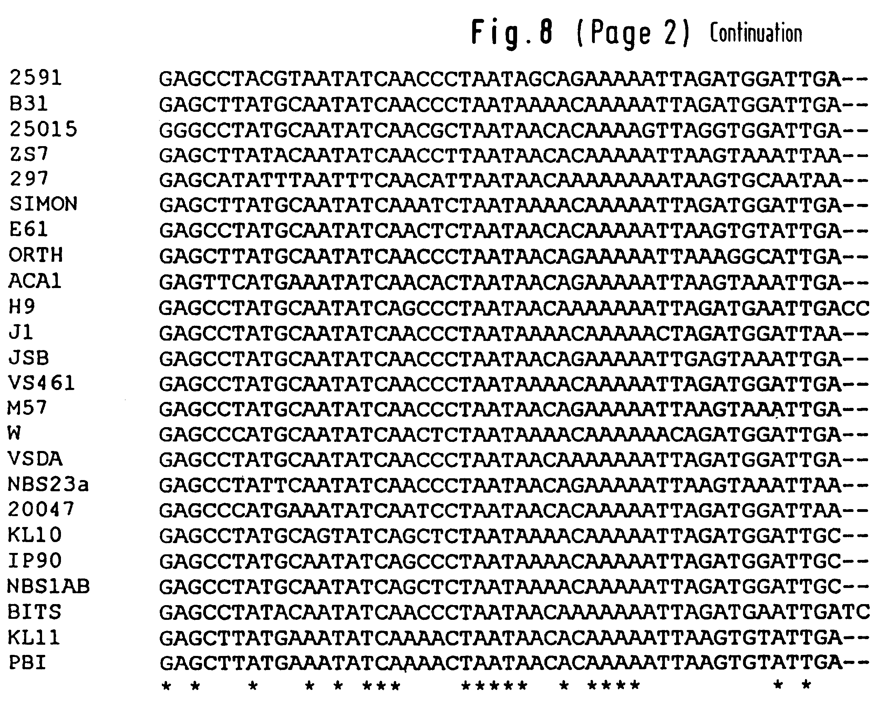

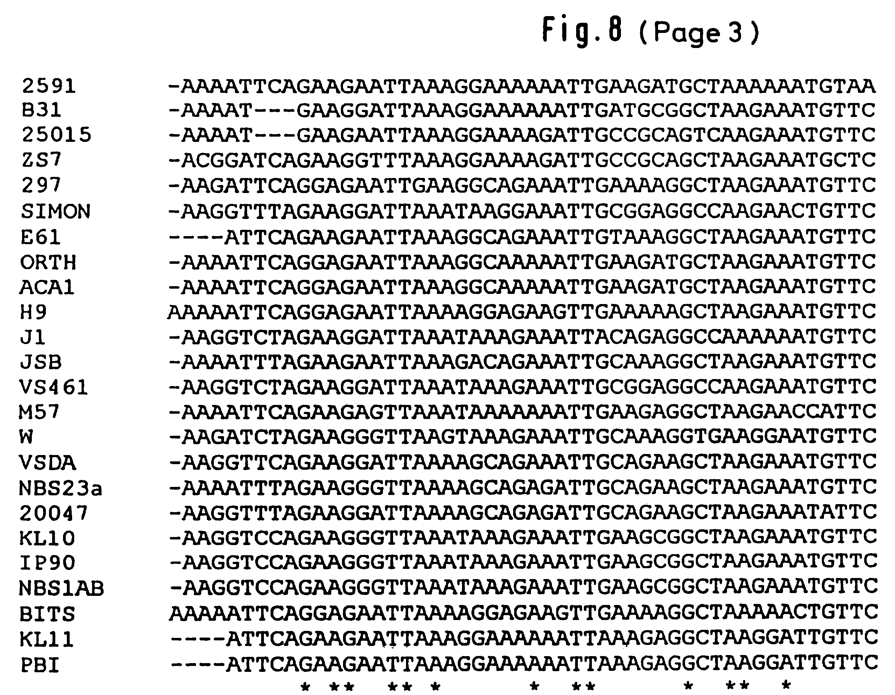

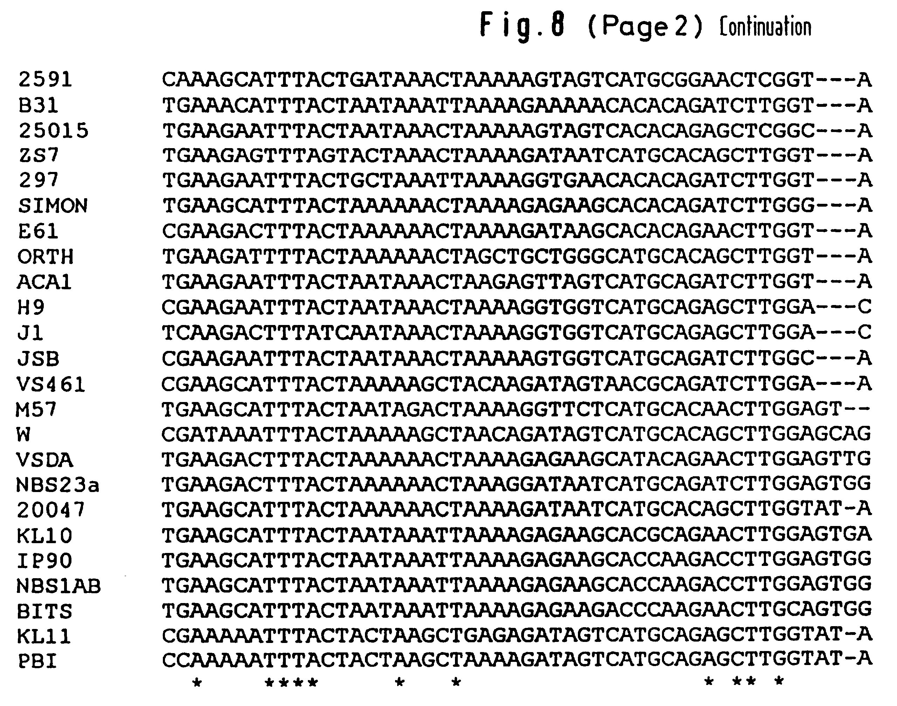

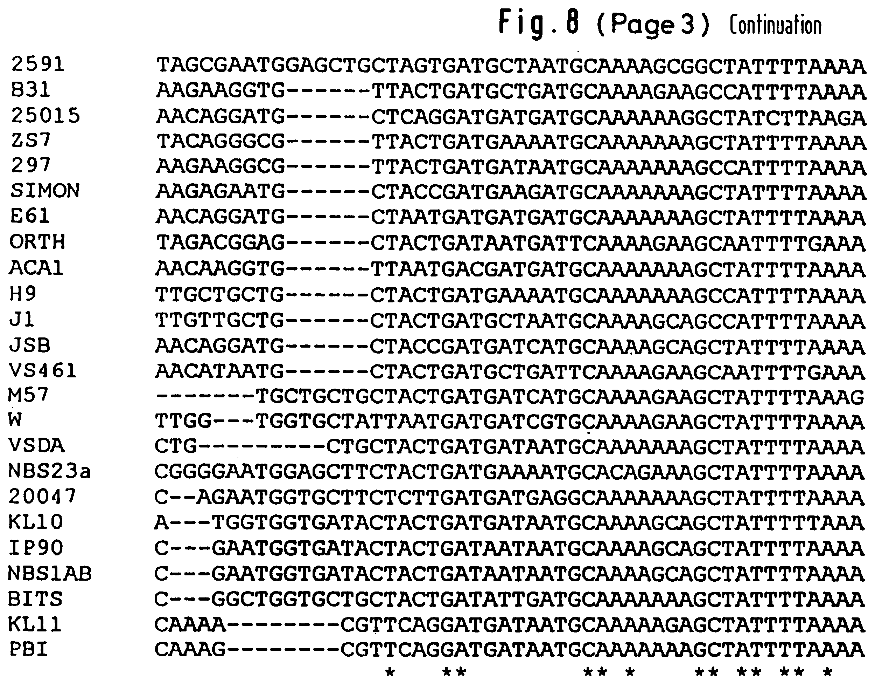

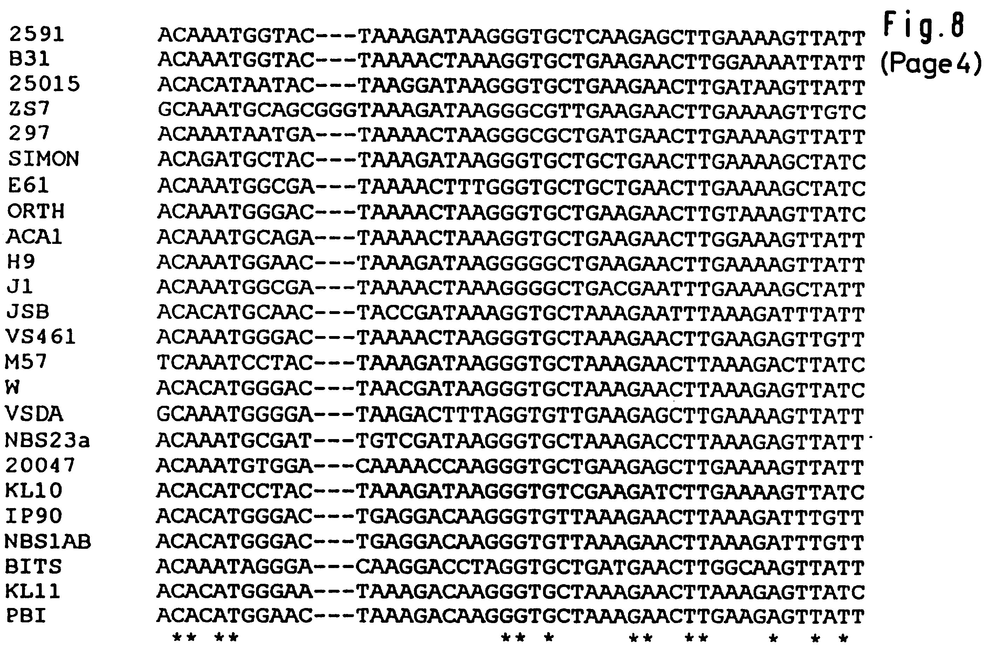

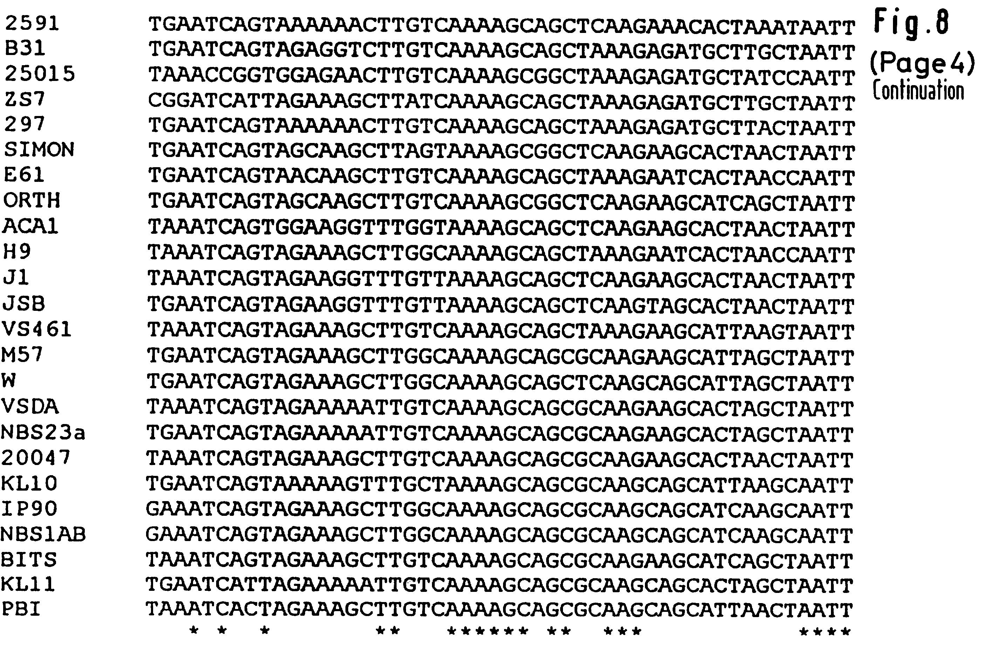

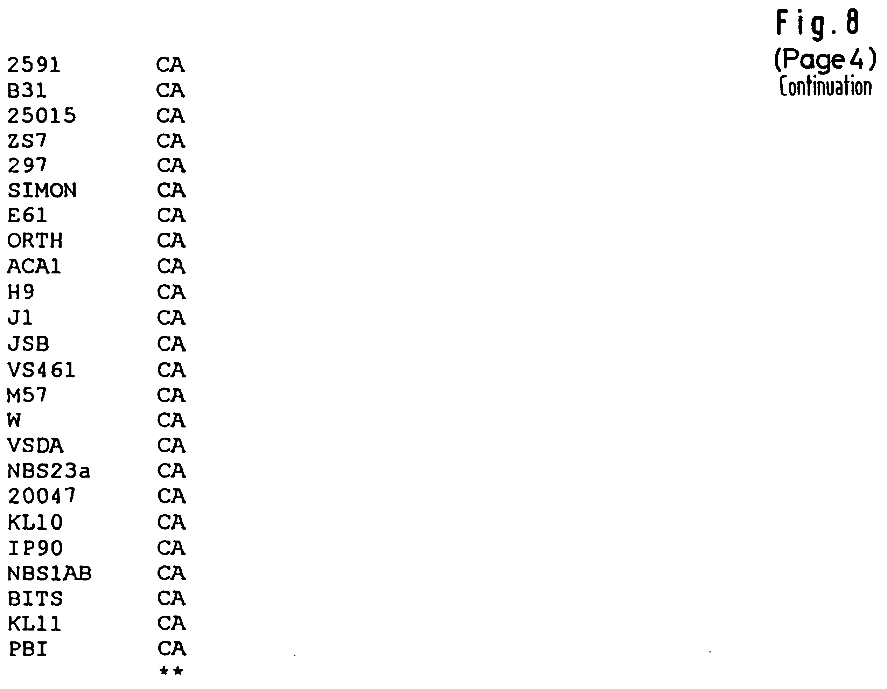

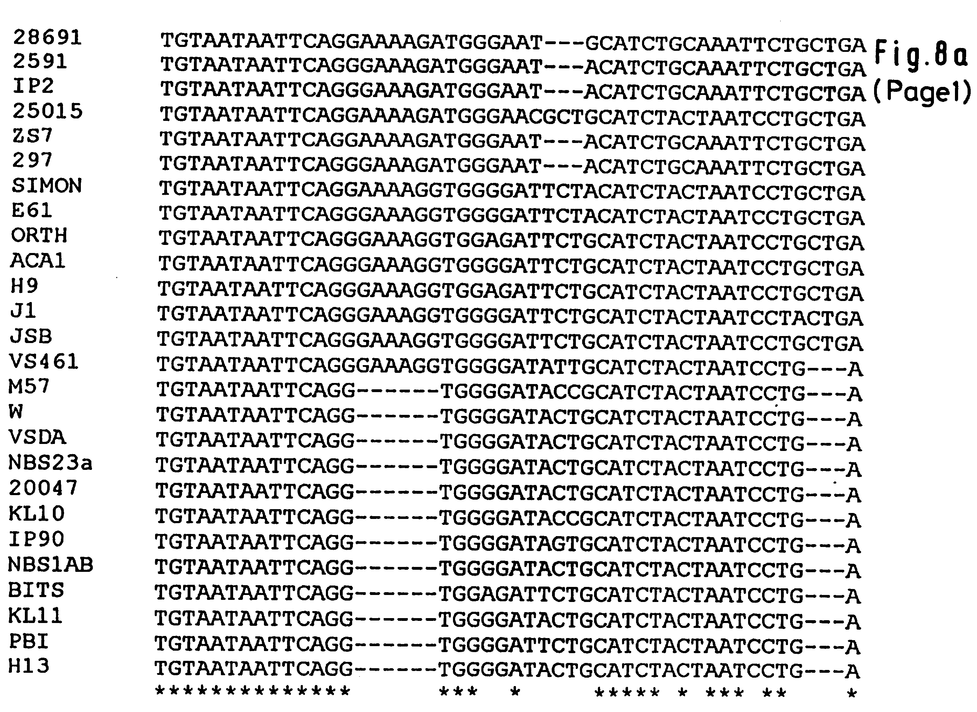

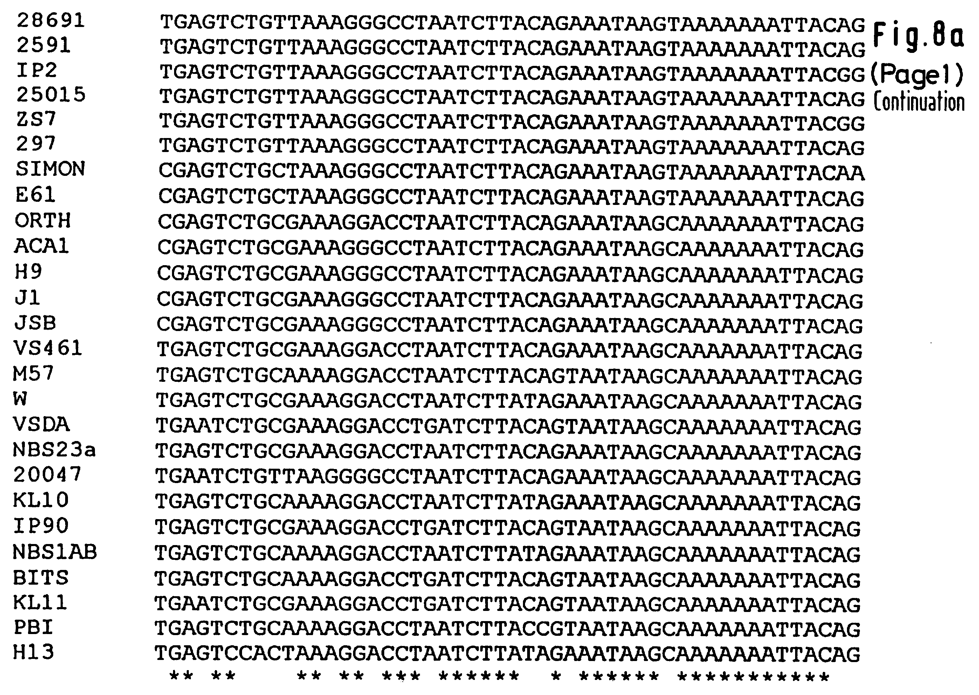

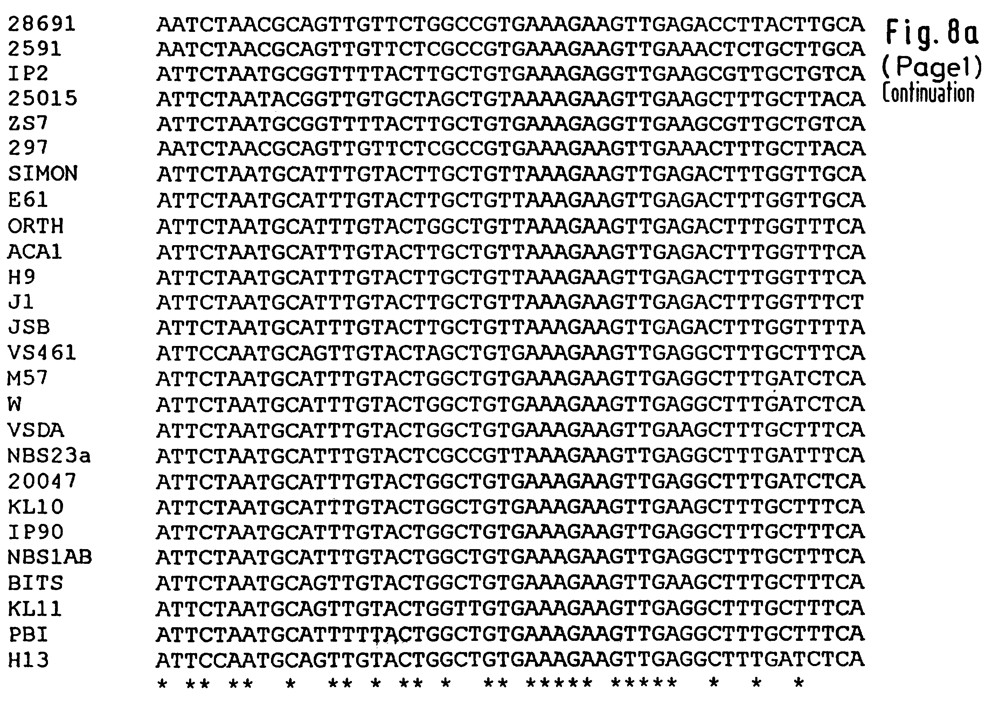

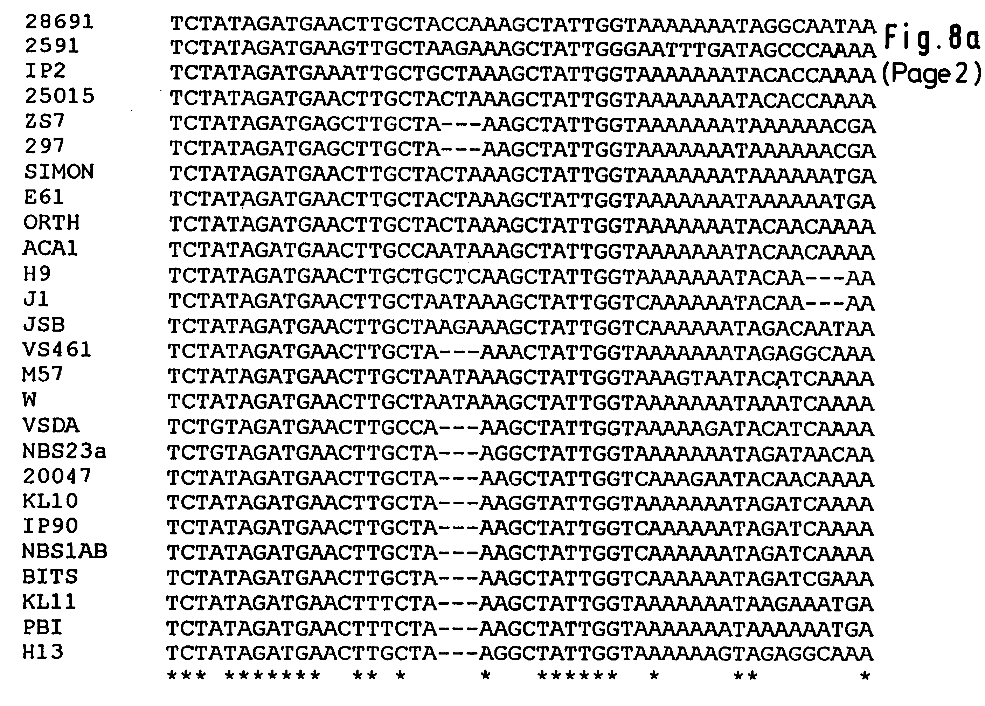

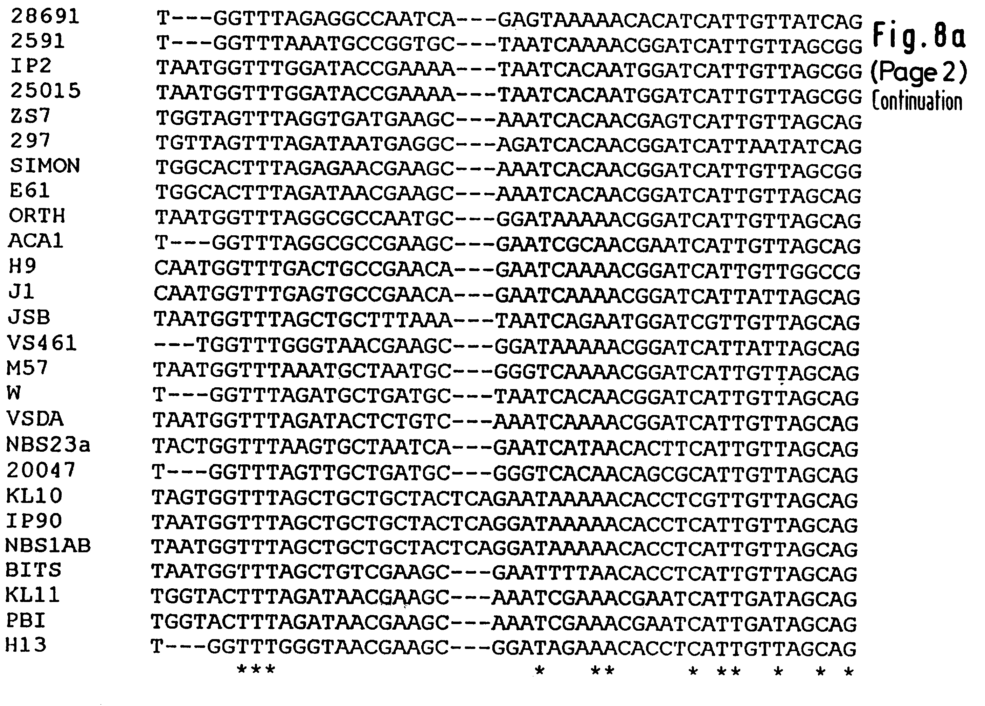

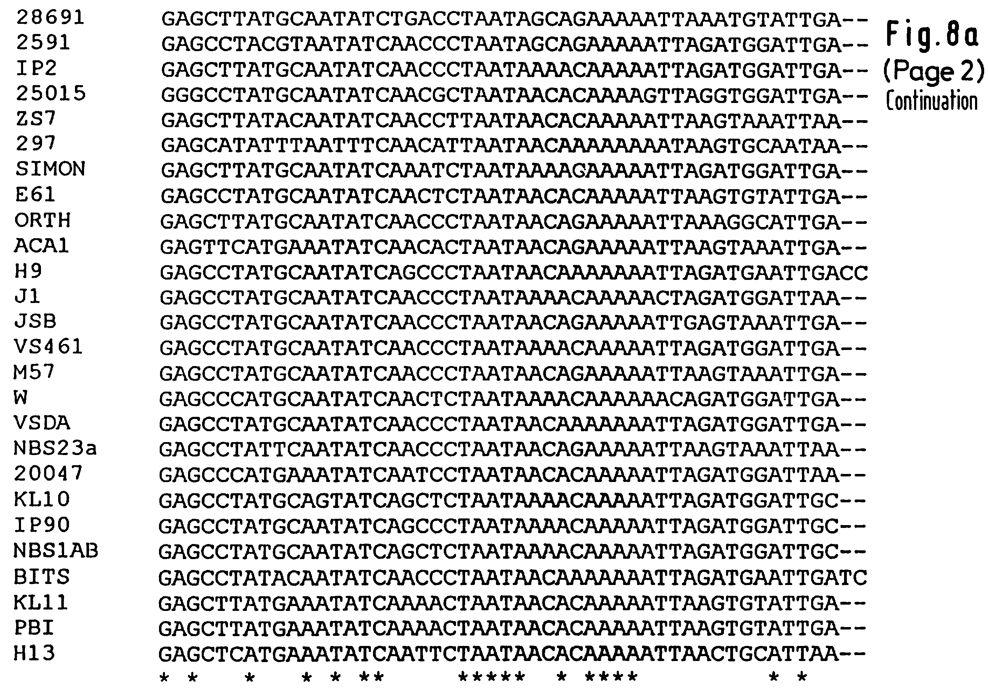

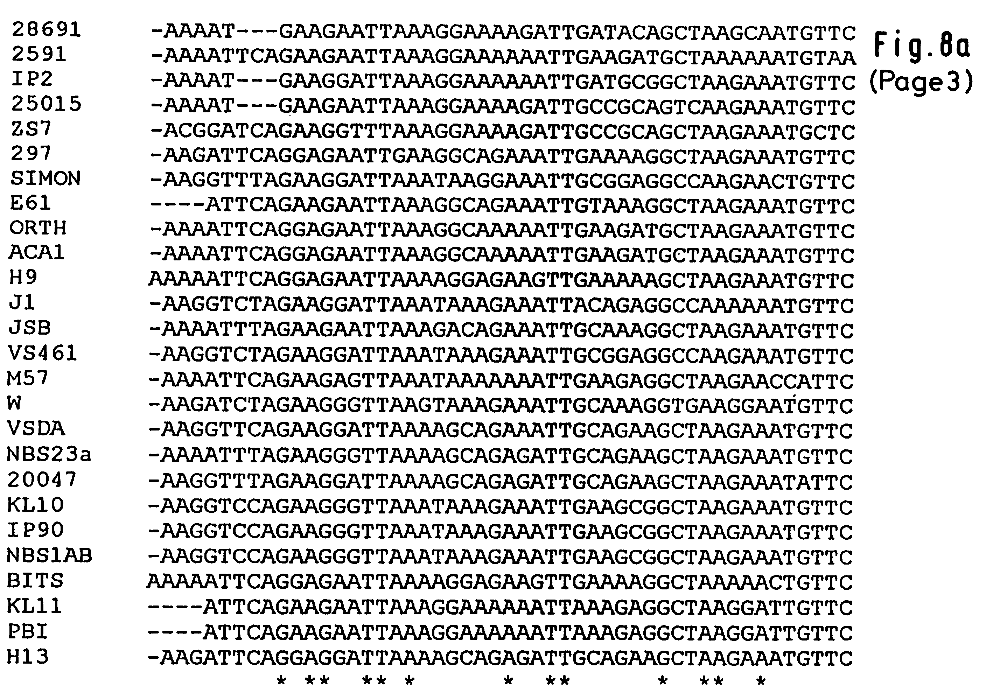

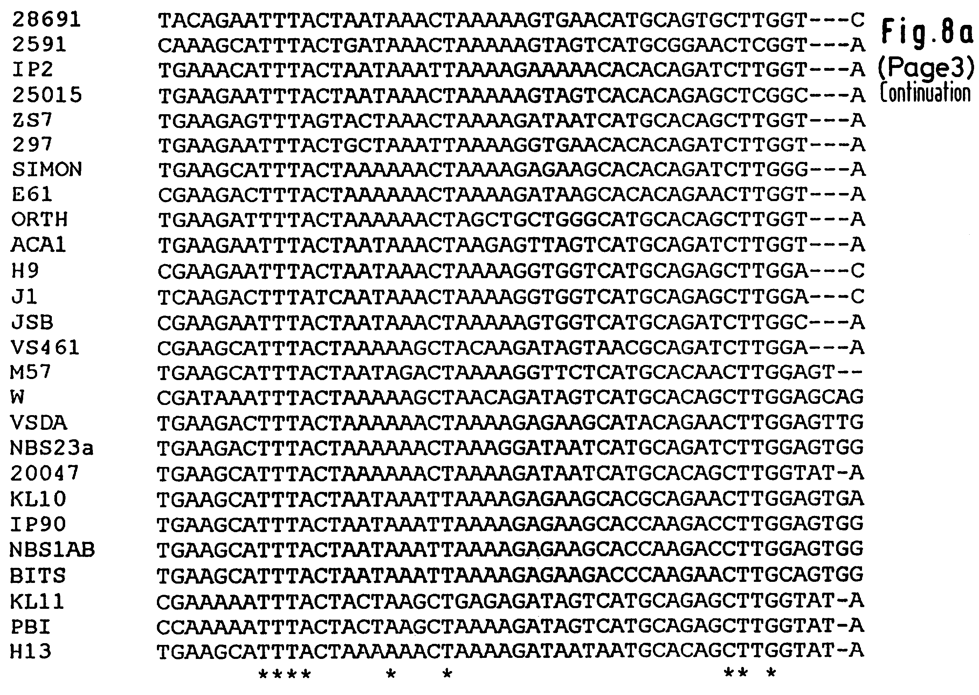

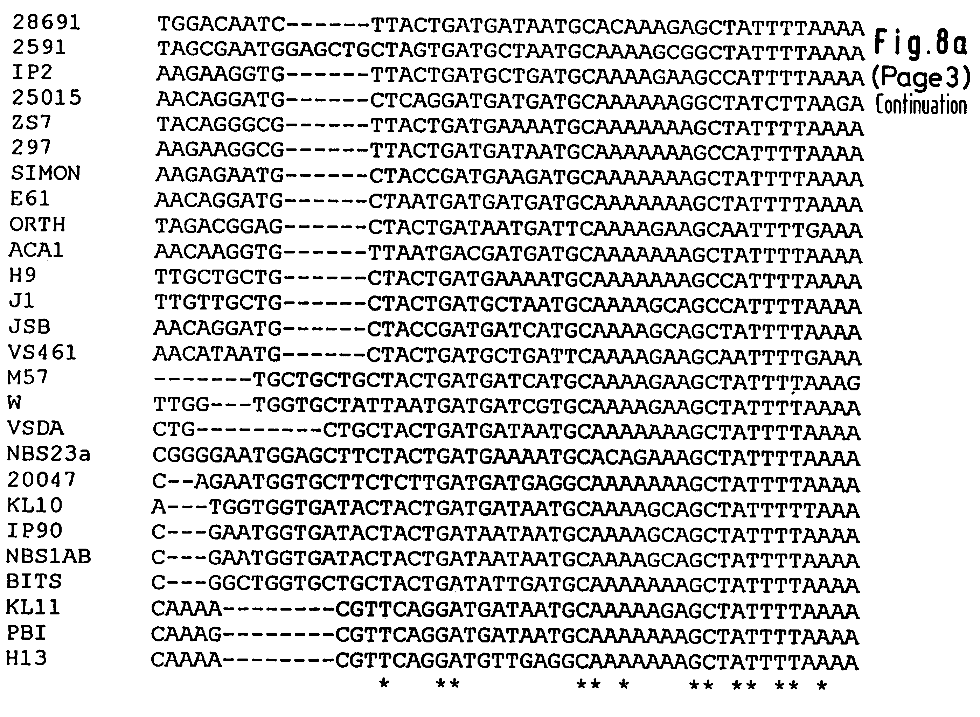

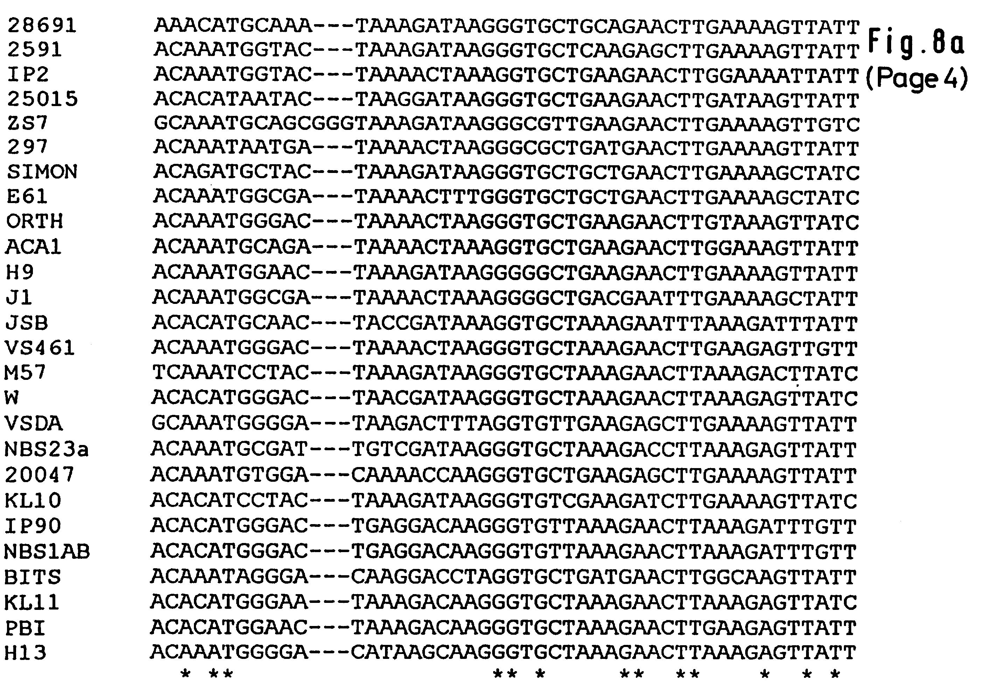

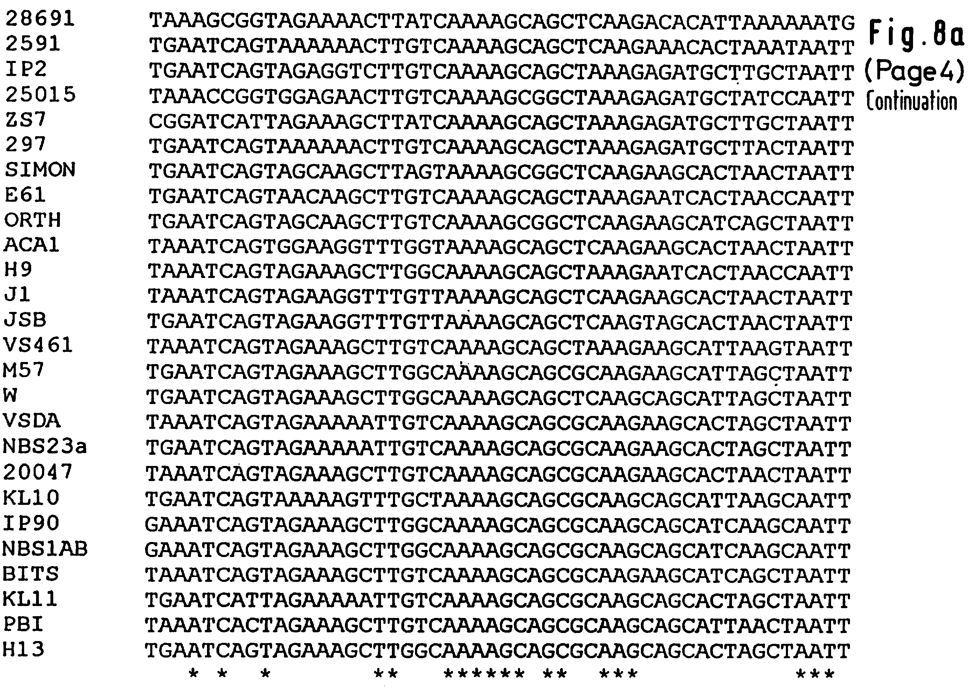

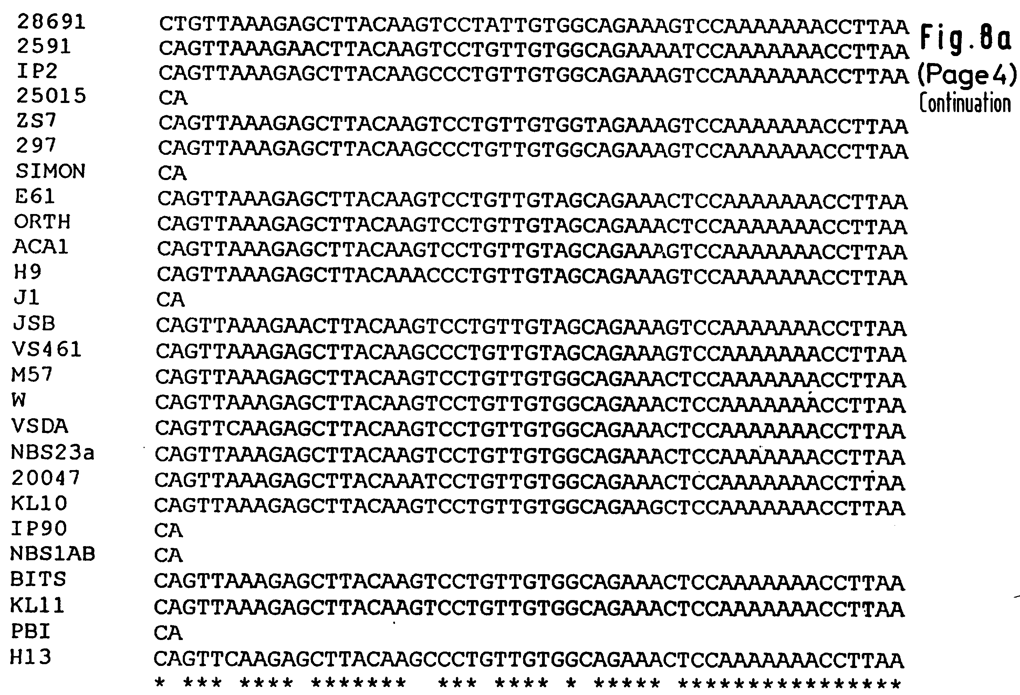

The relationship between closely related OspC proteins from the same OspC type was investigated as a further check on the validity of the typing systems and to establish whether within a given OspC type there was further heterogeneity. The nucleotide and deduced amino acid sequences for the OspC proteins from 24 strains are shown in Figures 8 and 9, respectively (i.e. 22 sequences from this study and 2 published sequences for strains 2591 and PBI). The dendrogram showing the phylogenetic relationship between the OspC protein is presented in Figure 10.

An OspC antigen-based immunogen of the present invention can comprise a mixture of different serological forms of naturally occurring OspC protein. In another embodiment of the invention, the immunogenic composition comprises OspC variants or OspC mimetics of OspC antigens. Thus, in addition to OspC protein obtained from Lyme disease *Borrelia* cells, as described hereinafter, recombinant OspC variants of the naturally-occurring molecule ("OspC variants") and "mimetics" -- compounds having mimotopes which mimic ospC epitopes -- can be employed.

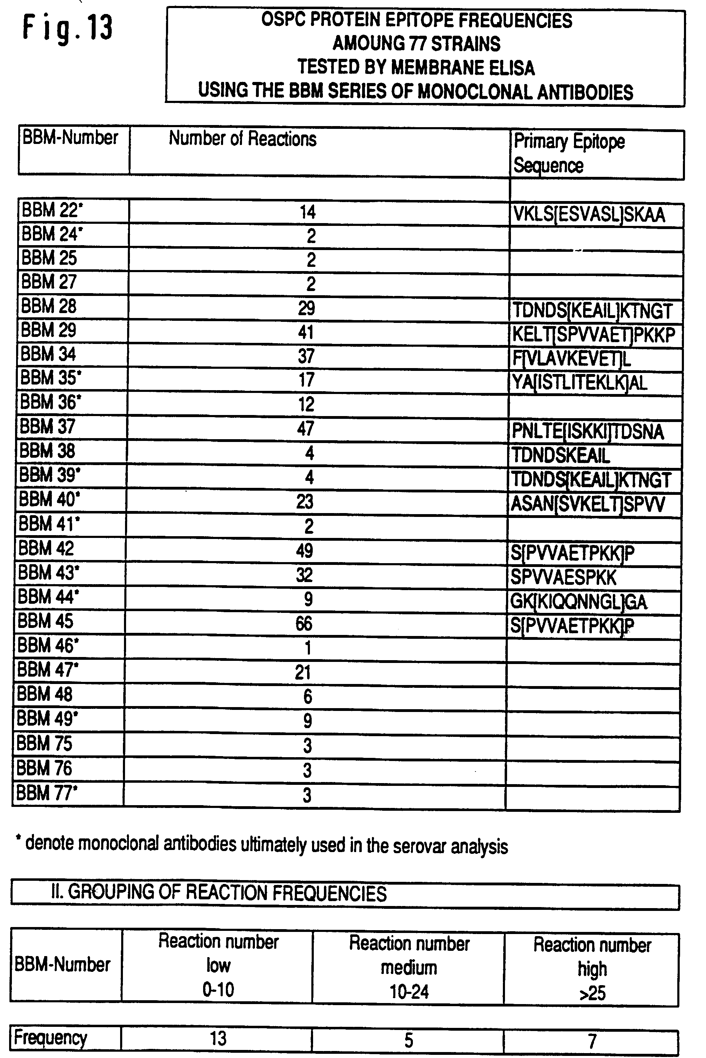

The category of OspC variants includes, for example, oligopeptides and polypeptides corresponding to immunogenic portions of the OspC molecule and any non-proteinaceous immunogenic portions of the OspC molecule. Thus, a variant is intended to include a polypeptide that is homologous to and retains the salient immunological features of the natural OspC molecule. In this regard, "homology" between two sequences connotes a likeness short of identity indicative of a derivation of the first sequence from the second. For example, a polypeptide is "homologous" to OspC if it contains an amino acid sequence which corresponds to an epitope recognized by OspC specific antibodies or T-cells. Such a sequence may be only a few amino acids long and may be a linear determinant or one which arises when amino acids from separated portions of a linear sequence are spatially juxtaposed after protein folding or after being subjected to covalent bond modification. The amino acid sequences which are antigenic determinants for purposes of this invention can be ascertained, for example, by monoclonal mapping analysis techniques which are known in the art. See Regenmortel, Immunology Today 10: 266-72 (1989), and Berzofsky et al., Immunological Reviews 98: 9-52 (1987). For instance, in the the present invention, the OspC antigen comprises one or more of the following amino acid sequence (Figure 13): 
(1) VKLSESVASLSKAA;    (2) TDNDSKEAILKTNGT;
(3) KELTSPVVAETPKKP;    (4) FVLAVKEVETL;
(5) YAISTLITEKLKAL;    (6) PNLTEISKKITDSNA;
(7) ASANSVKELTSPVV;    (8) SPVVAETPKKP;
(9) GKKIQQNNGLGA; and    (10) SPVVAESPKK
 or variants or mimetics of the above epitope sequences.
In the preferred embodiment, the vaccine would comprise peptides corresponding to serotype-specific epitopes selected from one or more of the OspC proteins from the OspC families described herein. Cross-protection studies (Example 6) indicate that protective immunity is induced by serotype-specific epitopes. An example of a serotype-specific epitope is sequence #2 from strain Orth (see above), which is recognized by monoclonal antibodies BBM38 and BBM39 which are specific for OspC proteins from OspC family 5 (serovar 4). This epitope corresponds to the putative epitope (DNDSKE) predicted from a hydrophilicity analysis of the Orth OspC. Potential serotype-specific epitopes can likewise be predicted to occur between amino acid residues 120-155 (starting from the first cysteine residue) in OspC proteins from other OspC families (example 4). Such a vaccine may include variants or mimetics of the peptide sequences, as described below. Assaying for this type of similarity can also be effected via a competitive-inhibition study in the case of antibodies or by T-cell proliferation.

Polypeptides which qualify as OspC variants according to these criteria can be produced, pursuant to the present invention, by conventional reverse genetic techniques, i.e., by designing a genetic sequence based upon an amino acid sequence or by conventional genetic splicing techniques. For example, OspC variants can be produced by techniques which involve site-directed mutagenesis or oligonucleotide-directed mutagenesis. See, for example, "Mutagenesis of Cloned DNA," in CURRENT PROTOCOLS IN MOLECULAR BIOLOGY 8.0. 3 et seq. (Ausubel et al. eds. 1989) ("Ausubel").

Other OspC variants within the present invention are molecules that correspond to a portion of OspC, or that comprise a portion of OspC but are not coincident with the natural molecule, and that display the immunogenic activity of OspC when presented alone or, alternatively, when linked to a carrier. An OspC variant of this sort could represent an actual fragment of the natural molecule or could be a polypeptide synthesized de novo or recombinantly.

To be used in recombinant expression of OspC or an OspC variant, a polynucleotide molecule encoding such a molecule would preferably comprise a nucleotide sequence, corresponding to the desired amino acid sequence, that is optimized for the host of choice in terms of codon usage, initiation of translation, and expression of commercially useful amounts of OspC or a desired OspC variant. Also, the vector selected for transforming the chosen host organism with such a polynucleotide molecule should allow for efficient maintenance and transcription of the sequence encoding the polypeptide. The encoding polynucleotide molecule may code for a chimeric protein; that is, it can have a nucleotide sequence encoding an immunological portion of the OspC molecule operably linked to a coding sequence for a non-OspC moiety, such as a signal peptide for the host cell.

In order to isolate a DNA segment which encodes an OspC molecule, total Lyme disease *Borrelia* DNA can be prepared, according to published methods. See, for example, Maniatis, et al., MOLECULAR CLONING: A LABORATORY MANUAL (Cold Spring Harbor Laboratories, NY 1982); Baess, Acta Pathol. Microbiol. Scand. (Sect. B) 82: 780-84 (1974). The DNA thus obtained can be partially digested with a restriction enzyme to provide a more or less random assortment of genomic fragments; an enzyme with a tetranucleotide recognition site, such as Sau3A (MboI), is suitable for this purpose. The fragments from such a partial digestion then can be size-fractionated, for example, by sucrose gradient centrifugation (see Maniatis, supra) or by pulsed field gel electrophoresis, see Anal, Trends in Genetics (November 1986), at pages 278-83, to provide fragments of a length commensurate with that of DNA encoding the OspC molecule.

According to well-known methods described, for example, in Ausubel at 5.0.1 et seq., the selected fragments can be cloned into a suitable cloning vector. A DNA thus obtained could be inserted, for example, at the BamHI site of the pUC18 cloning vector. Chimeric plasmids or phage, inter alia, produced by joining the size-selected fragments to the cloning vector can then be transformed into *E. coli* or other host cells, which are screened thereafter for expression of the encoded protein. A variety of methods can be used for screening libraries to identify a clone containing the OspC gene. These methods include screening with a hybridization probe specific for OspC, such as an oligonucleotide probe, or screening for OspC antigen expression using a OspC specific immunological reagent. The latter, for instance, may be accomplished by immunoblotting a library with anti-OspC monoclonal antibodies or with a specific polyclonal antibody prepared from animals immunized with purified OspC. Once a clone containing OspC encoding DNA is identified in the library, the DNA can be isolated, the region encoding OspC protein fully characterized (as by sequencing), and, subsequently, the DNA can be used to produce ospC expression vectors suitable to the production of OspC-active protein.

As noted previously, to provide an effective immunogen the structure of the recombinantly expressed pC protein should be sufficiently similar to that of native (non-denatured) OspC so that the protein induces the production of protective antibodies. To this end, it is preferable to express OspC-encoding DNA in such a way that intracellular proteolysis and aggregation of the expression product, in denatured form, are avoided. One way to avoid these problems is to recombinantly produce pC in a host-vector system that provides for secretion of pC from the host cell, preferably directly into the culture medium. One such system is provided by *Bacillus subtilis.* A suitable secretion vector can be constructed for *B.subtilis* by linking *the B. amyloliguefaciens* α-amylase signal sequence, see Young, et al., Nucleic Acid Res. 11: 237-49 (1983), to the *Bacillus* plasmid vector pUB110, as described by Ulmanen, et al., J. Bacteriol. 162: 176-82 (1985). According to this approach, the coding sequence for the foreign protein is cloned downstream of the promoter, the ribosome binding site and the signal sequence for α-amylase. Transcription and translation of OspC is under control of the α-amylase promoter and translation machinery in this construct, and secretion of pC from the host cell is provided by the α-amylase signal sequence. The present invention comprises expression vectors which are functional in procaryotes as well as eucaryotes. Similar vectors for use in yeast have been described and the expression secretion of OspC in yeast using these vectors could be achieved. A suitable expression vector can be constructed by linking the OspC coding sequence to an inducible promotor in a yeast replication plasmid. According to this approach, the coding sequence of the foreign protein is cloned downstream of e.g. the AOX-1 promotor and transcription and translation can be induced by the addition of methanol to the culture medium. Either intracellular expression or secretion of the foreign protein (by linking a signal sequence to the coding sequence of the mature protein) can be obtained. A preferred yeast strain is Pichia pastoris. In yeast, especially P. pastoris, high yields of the expression products were obtained.

Yet another approach for expressing OspC in a host vector-system which avoids proteolysis, aggregation and denaturation is the use of vaccinia virus as a vector capable of expression in a variety of mammalian host cells susceptible to vaccinia infection. This approach would entail preparing a recombinant vaccinia virus-derived vector in which the pC gene is placed under the control of a promoter, along with translation and secretion signals, suitable for expressing OspC protein in a vaccinia-infected host. As described in U.S. patent No. 4,603,112, the contents of which are hereby incorporated by reference, the plasmid also would comprise, 5' to the transcription control regions and 3' to the 3' termination and polyadenylation signals, flanking sequences which are conducive to homologous recombination into a wild-type vaccinia genome. When a construct of this sort is introduced into a vaccinia infected host cell, the flanking sequences direct recombination between the plasmid vector and the vaccinia virus, with the result that a cloned structural sequence (here, encoding OspC) becomes part of, propagates with and is expressed with the vaccinia virus. Preferably, the region between the flanking sequences also contains a selectable marker, such that in the presence of selection medium only those cells containing recombined vaccinia virus (and, in the present context, the sequence encoding a OspC-active polypeptide), will survive.

A recombinant vaccinia strain produced in this manner can be used to infect mammalian cells, such as Vero cells or CV1 cells, suitable for high density fermentative growth. The OspC-active protein expressed in these cells during fermentation would be secreted into the fermentation medium, from which it would be purified via conventional methodology.

In addition to natural OspC and OspC variants, the present invention comprehends compounds ("mimetics") which mimic OspC epitopes ("mimotopes"). One example of a mimetic is an anti-idiotype antibody, that is, an antibody that is produced by immunizing an animal with an antibody which specifically binds to an epitope on an antigen. The anti-idiotype antibody recognizes and conforms to the combining site on the first antibody. Therefore, the shape of its combining site closely resembles the epitope which fits into the combining site of the first antibody. Because an anti-idiotype antibody has a combining site whose shape mimics the original antigen, it can be used as a vaccine to generate antibodies which react with the original antigen. See Fineberg & Ertl, CRC Critical Reviews in Immunology 7: 269-84 (1987). Appropriate mimetics could be identified by screening with a OspC antibody to detect which compounds bind thereto or could be produced by molecular modelling. See Morgan et al., "Approaches to the Discovery of Non-Peptide Ligands for Peptide Receptors and Peptidases," in ANNUAL REPORTS IN MEDICINAL CHEMISTRY (Academic Press 1989), at pages 243 et seq.

The vaccine of the present invention is intended for the immunization of a susceptible mammal, including a human being, against Lyme disease. The term "immunogen" means an antigen which evokes a specific immune response leading to humoral or cell-mediated immunity, in this context, to infection with *Borrelia.* "Immunity" thus denotes the ability of the individual to resist or overcome infection more easily when compared to individuals not immunized, or to tolerate the infection without being clinically affected.

The immunogen of the present invention may be further comprised of an acceptable physiological carrier. Such carriers are well-known in the art and include macromolecular carriers. Examples of suitable carriers in mammals include tuberculin PPD, bovine serum albumin, ovalbumin or keyhole limpet hemocyanin. The carrier should preferably be non-toxic and non-allergenic.

The immunogen may be further comprised of an adjuvant such as an aluminum compound, water and vegetable or mineral oil emulsions (for example, Freund's adjuvant), liposomes, ISCOM (immunostimulating complex), water-soluble glasses, polyanions (such as poly A:U, dextran sulphate or lentinan), non-toxic lipopolysaccharide analogues, muramyl dipeptide, and immunomodulating substances (for example, interleukins 1 and 2) or combinations thereof. The preferred adjuvant is aluminum hydroxide. Immunogenicity can also be enhanced in mammals which have received live attenuated bacterial vectors, such as *Salmonella* or *Mycobacteria,* or viral vectors like vaccinia, which express an OspC-active polypeptide.

Techniques for formulating such immunogens are well-known in the art. For instance, the immunogen of the present invention may be lyophilized for subsequent rehydration in a physiologically acceptable excipient such as saline or other physiological solution. In any event, the vaccine of the present invention is prepared by mixing an immunologically effective amount of OspC with the excipient in an amount resulting in the desired concentration of the immunogenically effective component of the vaccine. The amount of immunogenically effective component in the vaccine will depend on the mammal to be immunized, with consideration given to the age and weight of the subject as well as the immunogenicity of the immunogenic component present in the vaccine. In most cases, an amount of the immunogenic component of the vaccine will be in the range of 1 to 100 micrograms per antigen per dose, and preferably will be in the range of 10 to 50 micrograms per antigen per dose.

In yet another embodiment of the present invention, the immunogenic composition is comprised of one or more OspC antigens or OspC variants or OspC mimetics or the OspC antigens substantially purified from each of the OspC-families of Figure 11 expressed by the human disease associated clones and clonal clusters, as described in Example 1.

Thus, the invention comprises according to claims 1-3 immunogenic compositions of OspC antigens of Lyme disease Borrelia consisting either or one or more, preferably two or more, OspC antigens of the 20 currently recognized OspC-families as shown in Figure 11 or of one or more OspC antigens from each of the 20 currently recognized OspC-families of Figure 11. Instead of the above OspC antigens, the combination may comprise OspC variants or OspC mimetics of said OspC antigens, said OspC variants or OspC mimetics having a structure that is sufficiently similar to native OspC to induce the protection of protective antibodies.

Figure 11 shows the sequences of essential epitopes. According to the invention, OspC antigens are included which comprise one or more of such epitopes. Consequently, these antigens or polypeptides according to the invention comprise at least the epitopic sequence as shown in Figure 13. This epitopic sequence may be as short as the amino acid sequence given in brackets in Figure 13.

As a further embodiment the invention also comprises immunogenic compositions which contain either one or more, preferably two or more, OspC antigens of the OspC-families of Figure 19 or one or more OspC antigens from each of the OspC-families of Figure 19. These immunogenic compositions correspond to patent claims 3 and 4.

In another embodiment, the immunogenic composition is formulated to protect against Lyme disease Borrelia strains prevalent within a particular geographic region. Thus, in one embodiment, a vaccine is formulated which is preferentially protective against Lyme disease *Borrelia* strains most prevalent in North America. In another embodiment, a vaccine is formulated for Lyme disease Borrelia strains most prevalent in Europe. In a third embodiment, a vaccine is formulated for Lyme disease Borrelia strains most prevalent in Austria. Vaccine formulations for each of these geographical locations are shown in Example 7.
In addition, to OspC vaccine formulations a combined OspA/OspC vaccine is considered since this could be superior to a vaccine formulated with either antigen alone;
firstly, because Lyme disease *Borrelia* strains may not always express one or the other of these antigens but they always express either OspA or OspC;
secondly, it has been reported that there is reciprocal regulation of these two antigens such that if the expression of one is down regulated (e.g. in response to the immune response) the expression of the other is enhanced.
thirdly, at least for *in vitro* studies with OspA it has been demonstrated that vaccine escape mutants could arise, a problem that can be circumvented by the inclusion of a second antigen since a double mutational event in two independent antigens is extremely improbable.
fourthly, the immune response of vaccines to a given antigen is not uniform. The inclusion of two antigens enhances the probability that an individual, who responds poorly to either OspA or OspC, would nevertheless be protected by making a protective response to the other antigen.

Finally, it is to be expected that there could be a synergetic effect and that a more solid immunity would be obtained with a vaccine comprising OspA and OspC.

In one embodiment one or more OspC proteins from the OspC families 1-20 would be combined with one or more OspA proteins as expressed by *Borrelia* strains B31, Orth, H4 and KL11.
In another embodiment a combined OspA/OspC vaccine for the United States comprises an OspC from OpsCs family 2 and 3 together with an OspA as expresssed by strains B31. In a further embodiment a combined OspA/OspC vaccine for use in Europe comprises 14 OspCs from OspC families 2, 4-7, 9, 10, 12, 13 and 14, 15-17, 19 together with OspAs as expressed by strains B31, Orth, H4 and KL11. A further embodiment of a combined OspA/OspC vaccine for Austria comprises OspCs from families 2, 4-7, 10, 13 and 19.

The invention also comprises the use of a combination of antigens as comprised by the above-described immunogenic compositions for the manufacture of a vaccine for the treatment or prevention of Lyme borreliosis in a mammal. As a preferred embodiment this vaccine is useful for humans.

The methods for preparing of vaccines according to the present invention are designed to ensure that the identity and immunological effectiveness of the specific molecules are maintained and that no unwanted microbial contaminants are introduced. The final products are distributed and maintained under aseptic conditions. The method of immunizing a mammal against Lyme disease involves administering -to the mammal an effective amount of the foregoing immunogen. Administration may involve any procedure well-known in the art. For instance, a suitable administration strategy may involve administering the above described vaccine to mammals which are known to be exposed to ticks bearing Lyme disease *Borrelia ,* approximately 6 months to 1 year prior to the time of anticipated exposure. Any immunization route which may be contemplated or shown to produce an appropriate immune response can be employed, in accordance with the present invention, although parenteral administration is preferred. Suitable administration forms include subcutaneous, intracutaneous or intramuscular injections or preparations suitable for oral, nasal or rectal administration.

By "substantially purified" is meant a homogenous protein free of any toxic components, thereby reducing the likelihood of an adverse reaction. "Homogenous" in this context means that at least 80% (w/v) of the protein is fully intact OspC, with nearly all of the remainder represented by OspC breakdown products. Thus, impurities in the form of media constituents and other *Borrelia* proteins are present, if at all, only in trace amounts. Homogenous OspC may be comprised of more than one serological form of OspC.

In this way the present invention enables the removal of unwanted, potentially immunogenic proteins which could induce autoantibodies and cause harmful autoimmune reactions in the immunized mammal. By the same token, the above-described purification method also ensures lot-to-lot reproducibility during vaccine production.

The preferred method of purification comprises the following steps:
(a) disruption of Lyme disease *Borrelia* cells and fractionation by centrifugation into "membrane" and "cytoplasmic" components;
(b) extraction of the membrane fraction with a non-denaturing detergent followed by centrifugation to obtain a supernatant comprising solubilized protein and to remove insoluble material as a pellet; and
(c) fractionation of solubilized antigens by ion-exchange chromatography (diethylaminoethyl or "DEAE"), adsorbed antigens being eluted with a NaCl gradient.

The purification method can include concentration and further purification of the antigens by:
(a) hydroxylapatite chromatography, adsorbed antigens being eluted by increasing the phosphate content of the buffer; and/or
(b) immobilized metal-affinity chromatography, adsorbed antigens being eluted with imidazole.

Other elution methods known in the art include elution by a reduction in pH or by increasing concentrations of ammonium chloride, histidine or other substance with affinity for the chelated metal.

Cell disruption can be accomplished by lysing cells by shaking them in suspension in a cell mill with tiny glass beads, by sonication or in a French-press. Alternatively, antigens may be extracted directly from the cell-surface of the organism by exposing the cell to a detergent, by changing the ionic strength of the cell's environment or by slightly shifting the temperature. Alternatively, a starting material comprised of membrane blebs which are shed from cells may be used.

The extraction of the membrane fraction may be accomplished with a detergent which preferably has good solubilizing power, is non-denaturing and is compatible with ion-exchange chromatography. The preferred detergent is zwitterionic detergent 3-14 by Calbiochem, although any detergent or organic solvent may be used which has the above characteristics. The detergent is typically used at a concentration of 1% (w/v) but would be effective to varying degrees in the range of 0.01-10% (w/v). Detergent extraction is carried out at a temperature in the range of 0 to 60°C, preferably at 37°C and should take from ten minutes to 8 hours, preferably one hour. Chaotropic agents such as urea could be used in addition to the detergent to improve the solubilization process.

The detergent solubilized antigens are then fractionated by DEAE-chromatography. Preferably, a DEAE ion-exchange resin is used but other anionic or cationic exchange resins may be used instead or in conjunction with one another. In accordance with the present invention, an ion-exchange resin comprises an insoluble matrix to which charged groups have been coupled. Functional groups used for anion exchangers include amino ethyl (AE), diethylaminoethyl (DEAE) and quaternary aminoethyl (QAE) groups. Cation-exchangers may have carboxymethyl (CM), phospho- or sulphopropyl (SP) groups. Although samples are applied to the column in a Tris buffer containing zwitterionic detergent 3-14 (1%), and the antigens are eluted with a gradient of NaCl, other formulations may be equally effective.

Antigens may be concentrated by binding them onto hydroxylapatite, according to methods well known in the art. An alternative or complementary procedure by which antigens can be further concentrated/purified is by immobilized metal-affinity chromatography. This latter method is preferred to hydroxylapatite chromatography for the purification of OspC since a better separation from OspA and OspB is achieved.

The advantage of the above described non-denaturing purification process is that the three-dimensional conformation of the protein is maintained, thereby keeping all the antibody combining sites found on the native protein, including those involved in protection. If a protein is denatured, the binding sites may be partially or completely destroyed and the capacity of the antigen to induce antibodies to the antigenic sites will be correspondingly diminished. Proteins thus altered therefore would be undesirable for use in vaccines.

Further, the invention comprises the recombinant preparation of novel OspC antigens as shown in Figure 9a. The invention also includes novel DNA sequences encoding OspC antigens according to Figure 9a. These DNA sequences are shown in Figure 8a. Also comprised by the invention are those DNA sequences which have at least 80% homology to any of the sequences of Figure 8a.

The invention also comprises the recombinant expression vectors in procaryotic or eucaryotic host cells, especially expression vectors useful in yeast, preferably Pichia. pastoris. According to a preferred embodiment of the invention the expression vector is inducible by methanol.

The invention comprises novel OspC antigens as (i) encoded by any of the sequences according to Figure 8a or (ii) having a homology of at least 80% with any of the amino acid sequences of Figure 9a.

The present invention is described in more detail in the following examples, which are illustrative and in no way intended to limit the scope of the invention.

### Example 1: CMAT typing of Borrelia burgdorferi strains and cluster analysis of the results which thereby permits the elucidation of CMAT clusters, CMAT families, "human disease associated" clones and clonal clusters

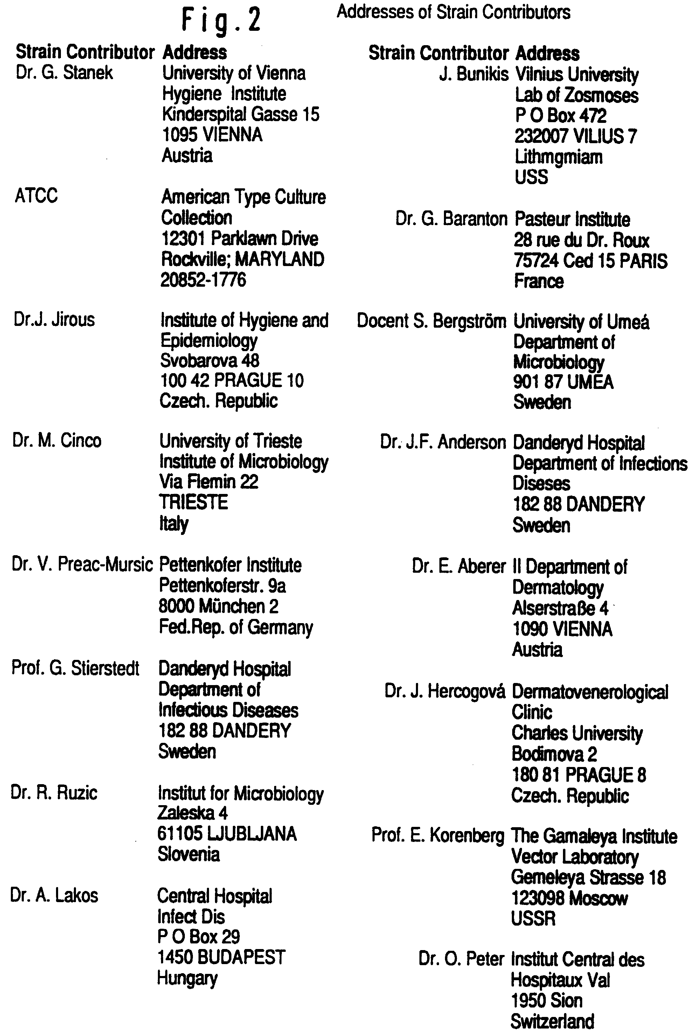

Seventy Seven strains (see Figure 1) were obtained from numerous sources listed in Figure 2. Care was taken that the collection contained strains of widely differing geographical origin and from as many differing epidemiological and clinical situations as possible.

Membrane fractions were then prepared from all strains as follows :
Lyme disease Borrelia cells were harvested by centrifugation (7000 x g, 20 minutes, 4°C), the cell pellet was washed twice in PBS containing 5 mM MgCl₂ and the cell wet-weight was determined. The washed cells were then lysed by shaking the mixture in a Vibrogen cell-mill (Model V14, Buhler). Three minute cycles of shaking with cooling (4°C) were repeated until lysis was greater than 99% complete, as assessed by dark-field microscopy. The lysate was then filtered on a sintered glass filter to remove the glass beads and the retained beads were washed with buffer to improve the yield of bacterial antigens in the filtrate. The lysate was centrifuged for 20 minutes at 7500 x g at 4°C to produce a crude membrane fraction termed membrane 2 or the "lsp" (low speed pellet) fraction. The supernatant was further centrifuged for 30 minutes at 100,000 x g at 4°C to produce a more purified membrane fraction, termed membrane 1 or "hsp" (high speed pellet). Both membrane fractions were washed twice in 100 mM Tris-HCL buffer pH = 7.4, using the original centrifugation conditions. The membrane 2 fraction was used for typing purposes whereas the membrane 1 fractions were reserved for antigen purification.

Membrane proteins present in the membrane 2 fractions of each strain were then analyzed by SDS-PAGE, as described in Laemmli, U.K., Nature (London) 227: 680-85 (1970). Variations in molecular weight were determined by reference to the electrophoretic mobility of a set of standard proteins covering the molecular weight range of the full spectrum of membrane antigen markers used in the analysis.

The antigens are transferred to a nitrocellulose filter and are identified using a panel of monoclonal antibodies, for example, by immunoblotting methods well known in the art. Ausubel, et al., 2 CURRENT PROTOCOLS IN MOLECULAR BIOLOGY 10.8.1 et seq. (1992). Monoclonal antibodies are produced by well-known hybridoma technology, Kohler and Millstein, Nature 256: 495-97 (1975). In the preferred embodiment of the present invention, the strains set forth in Figure 1 are analyzed. In general, the selection of a membrane antigen for inclusion in the analysis is governed by (a) the availability of monoclonal antibodies to detect it and to distinguish it from the numerous other antigens present in the SDS-PAGE membrane antigen profiles of bacterial strains; (b) by the antigen in question being present in a significant proportion of the strains analyzed i.e. a common antigen; (c) by the fact that it can be reproducibly detected in multiple, separately prepared, membrane fractions of the same strain, i.e., there is no evidence for intra-strain variation for the particular antigen in question; (d) by the fact that the antigen marker is stably expressed in the same isolate after multiple cultivation and passage and after storage for considerable time (up to 2 years); and (e) by lack of evidence by published scientific literature that the genes encoding the markers are extrachromosomally inherited or that variations of the antigens were under specific antigen variation mechanisms which can lead to intra-strain variation.

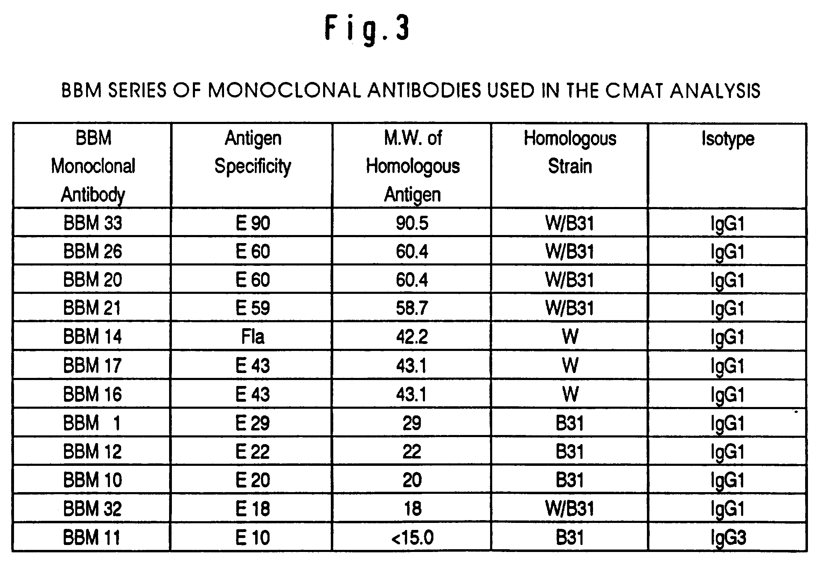

Figure 3 indicates the monoclonal antibodies used to identify the 9 common membrane antigens used in the analysis (E90, E60, E59, E43, Fla, E29, E22 antigens, the E18 + E20 antigen combined and the E10 antigen). The antigens are scored according to ascending molecular weight and, in the case where more than one monoclonal antibody exists, by their slightly differing reactivity (for example, E60 and E43), according to the reaction pattern of the antigen with those monoclonal antibodies.

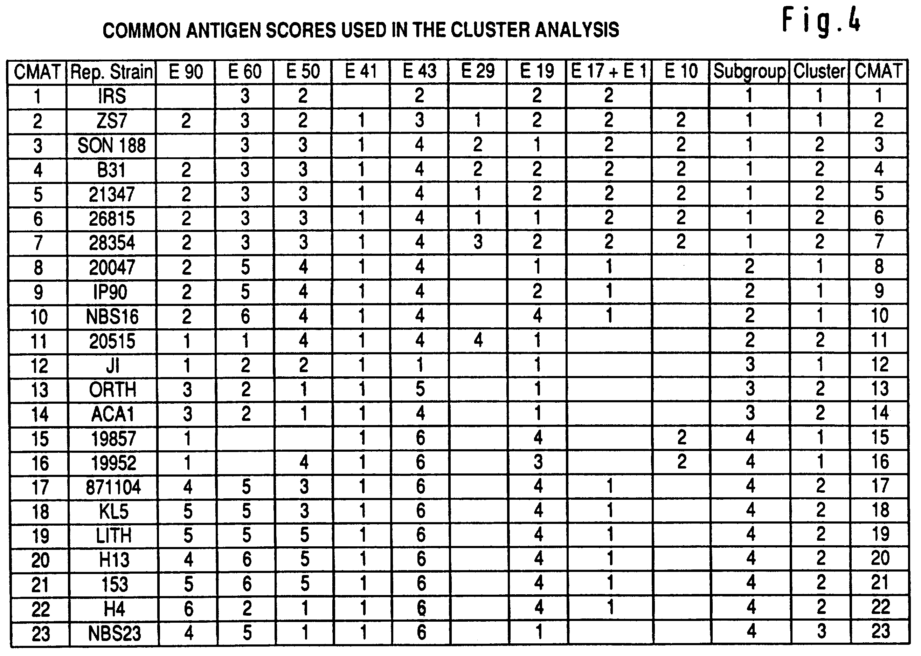

Figure 4 lists all unique combinations of 9 scores found for the strains analyzed which are represented as a 9-digit number. This 9-digit number then is designated as the common membrane antigen type (CMAT) of that strain. A cluster analysis was then performed to establish the relationship between all the CMATs observed. This was performed by determining the genetic diversity of each antigen and establishing an unweighted dissimilarity matrix calculated from the data of all unique CMATs. Absence of an individual common antigen score was treated as if it were missing data. The matrix formed was then subjected to cluster analysis with linkage by a weighted pair group method using arithmetic averages (Sneath and Sokal, supra) on an IBM compatible personal computer running CSS Statistica Statsoft software.

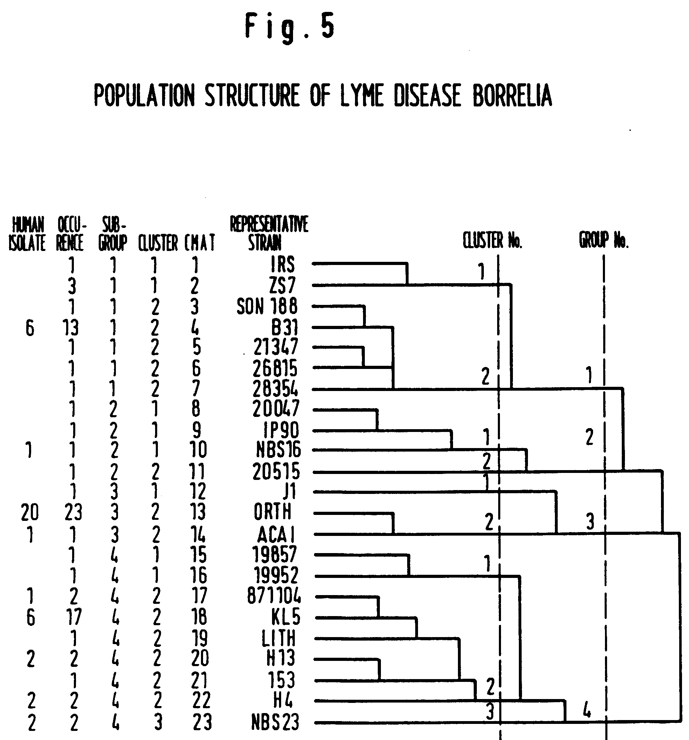

The resulting dendrogram of the cluster analysis is shown in Figure 5. In general, the dendrogram indicates that the Lyme disease *Borrelia* population is indeed well structured. By plotting the Eigen values at which each of the clustering steps occurs, it is possible to choose the most appropriate level at which to segregate the bacteria into categories.

The most ideal position to make divisions occurs at points in the curve where there are major jumps in the Eigen values for successive clustering steps. As shown in the dendrogram of Figure 5, this occurs at a level where there is between 68% and 88% difference in CMAT scores. This division divides the population into four major groups termed CMAT groups. A second major jump in the Eigen values for successive clustering occurs between the 40% and 52% difference between CMAT scores. This segregation divides each CMAT group into two to three clusters of individual CMATs. For convenience the 80% difference between scores was taken as the level at which CMAT grouping occurs, and 50% difference in scores at the level at which CMAT clustering occurs.

From the foregoing, it is apparent that the population of B. burgdorferi can be divided into four major CMAT groups, each of which is composed of two to three CMAT clusters. Each CMAT cluster was itself composed of between one and five individual CMATs. Comparison of these results with those of other population structure analysis taxinomic studies (Marconi & Garon, J. Bacteriol 174: 241-44 (1992); Boerlin et al., Infect. Immun. 60: 1677-83 (1992) Baranton et al., Int. J. Syst. Bacteriol. 42: 378-383, (1992)) indicates that the CMAT group 1 corresponds to the genospecies *Borrelia bugdorferi* sensu stricto, that CMAT group 3 is equivalent to *Borrelia afzelii* also known as "group VS461" and that CMAT groups 2 and 4 correspond to *B. garinii* sp. nov. The reason why the CMAT analysis divided the *B. garinii* genospecies into 2 CMAT groups is unclear but it may be due to the fact that fewer markers were used than for example in the multi locus isoenzyme electrophoresis study (Boerlin et al., Infect. Immun. 60: 1677-83 (1992)).

When looking at the occurrences of particular strains within each CMAT (Figure 5) it is apparent that 7 CMATs have more than one representative and thus can be considered as clones, i.e. strains having a common ancestry. Indeed 67% of all strains fell just within three distinct CMAT clones, for example, CMAT 4 (Clone 1:2:4) comprised 12 strains, CMAT 13 (Clone 3:2:13) comprising 23 strains, and CMAT 18 (Clone 4:2:18) with 15 strains. Notably, 76% (31 of 41, ) of the human isolates analyzed were found to be distributed among these three major clones. Futhermore, if one considers CMATs 17, 18, 20 and 22 together as part of a related clonal cluster, all belong to CMAT cluster 4.2, then the human disease associated goes up to a 87%. Due to this strong association with human disease, they can be considered as "human disease associated" (HDA) clones or clonal clusters (see definitions above). If one further looks at the types of isolates found among these three major clones, it becomes evident that CMAT 13, for example, appears to be associated with the chronic skin syndrome ACA (5 strains ) and that in general this clone is associated with syndromes of the skin (17 out of 19). In contrast, the other two HDA clones seem to be more prevalent in disseminating disease, that is, they are isolated form the blood or CSF from patients suffering from neuroborreliosis or Lyme arthritis. In the case of CMAT cluster 4.2 (i.e.CMATs 17,18,20 and 22) the epidemiological data seems to suggest a strong association with Neuroborreliosis. Of the 10 human isolates 6 were isolated from CSF material or from Neuroborreliosis patients, and four were isolated from patients with ECM, a syndrome normally associated with acute disease which can also be associated with neuroborreliosis. The syndrome association of CMAT 4 strains is not quite so clear cut as 2 of human isolates were isolated from blood, 3 from CSF and 1 from a patient with EM.

A further analysis of epidemiological data pertaining to the various strains reveals that the three major clones or clonal clusters have distinctive geographic distributions, and that this feature in turn correlates with general differences in the primary syndrome of Lyme disease observed within these regions. For example, CMAT 4 is the most predominant CHAT observed in North America, an area of the world where arthritic syndromes predominate. CMAT 13 and CMAT 18 are found predominantly in North Central Europe, where neurological syndromes and chronic skin syndromes predominate. Of the 3 major clones, only CMAT 4 is found in both North America and Europe (France, Austria and Russia), being widely distributed on both continents. Interestingly in areas of Central Europe, in particular Austria and Switzerland, where Lyme disease is endemic, all 3 major human disease associated clones co-exist.
The realization that human disease is predominanly caused by just one clone(CMAT 4) in CMAT group 1 (Borrelia bugdorferi sensu stricto) and one clone (CMAT 18) of CMAT group 3 (Borrelia afzelii) and a clonal cluster (CMAT cluster 4.2) in CMAT group 4 (B. garinii sp. nov.) allows one, in accordance with the present invention, to focus on these clones/clonal clusters with the aim of designing vaccines, such as an OspC vaccine or a combined OspC/OspA vaccine, which specifically protects against them. This vaccine then could be used in geographical regions where these clones are extremely prevalent. Furthermore, because of the differing clinical syndromes asociated with the differing clones, one can target a vaccine against these clones and, hence, in effect design vaccines to protect against specific syndromes.

### Example 2:

### Development of an OspC serovar typing scheme to analyse the serological variation of the OspC antigen of Lyme Disease Borrelia

### Preparation of anti-OspC antibodies

A panel of 25 monoclonal antibodies was produced against (a) four purified OspC proteins derived from (1) the Austrian strain Orth (BBM 34-39); (2) the German strain PKO (BBM 42-45); (3) the Czechoslovakian strains E61 (BBM 46, 47, and 49) and (4) KL10 (BBM 40-41); (b) an OspC protein enriched cocktail of antigens derived from the Austrian strain W (BBM 22, 24, 25, 27, 28, and 29); and (c) a membrane 2 fraction of the Czech strain M57(BBM 75-77).

The anti-OspC protein specificity of various monoclonal antibodies were confirmed by surfblot analysis against a membrane fraction of strain W or M57 in the case of BBM22, 24, 25, 27-29, and BBM 75-77, respectively, and in the case of the others by line blot analysis against the appropriate purified protein.

### Membrane ELISA Method

Serotyping of the OspC proteins was performed using a standard membrane ELISA technique. Membrane 2 fractions of all strains, prepared as describe in Example 1, were diluted to 0.1 mg/ml in phosphate buffered saline (PBS) pH 7.4 dispensed into individual wells of microtiter plates. The plates were allowed to dry out overnight at 37°C. Prior to use, the plates were washed twice in PBS, and then 50 ml of the diluted antibody solution in PBS containing 1% human albumin were added to each well and the plates incubated for one hour at 37°C. The plates were washed four times before the addition of the anti-mouse IgG alkaline phosphates conjugated antibody. The plates were incubated at 37°C for another hour before being washed four times prior to the addition of substrate, in order to estimate the amount of bound antibody.
Initially all strains were tested against all 25 monoclonal antibodies in order to see which monoclonals were most appropiate for serotyping purposes. Attempts were made to establish uniform positive and negative test criteria, however it became clear that this was not possible due to the vastly different levels of expresssion of the Osp C antigens within differing strains. To overcome this problem membrane 2 fractions were analysed be Western blot method, transfered to nitrocellulose and stained using Aurogold. Those strains which expressed no or low amounts of Osp C proteins (15 strains in all), as judged by the absence of a major protein in the 22 to 28 Kd molecular weight range, were removed from the study. Despite this, in a number of cases (for example, when using BBM 28, 29, 37 43, and 45), there was still no readily observed distinction between postive and negative results and thus these monoclonal antibodies were deemed unsuitable for typing purposes. All these antibodies recognise common epitopes and are thus of little discrimatory value anyway. Based on the strain coverage data of the initial analysis, a number of monoclonal antibodies were also found to recognise similar epitopes, e.g. BBM 24, 25 and 27, BBM 38 and 39 and BBM 75, 76, and 77, and thus only only one from each group (BBM 24, BBM 39 and BBM 77, respectively) were used in the final serovar typing scheme.
By removing these strains and monoclonal antibodies it was then possible to establish the criteria of a positive reaction as being one in which the optical density value obtained was significantly (three times) higher than the background level of negative strains. In practice this meant that a positve result had an Optical Density (OD) value greater than 0.6. All positive reactions were also confirmed by western blot analysis of the same membrane preparations. As a result of the western blot analysis it was discovered that BBM 48 strongly cross-reacted with a protein of approximately 60 kilodaltons, and gave rise to numerous false positive results in the ELISA analysis. Thus BBM 48 it was also omitted from the serovar analysis. Consequently the serovar analysis was somewhat simplified using only 13 of the inital 25 anti-OspC antibodies available.

The reaction pattern of each strain with the complete panel of 13 monoclonal antibodies then was collated, and each unique pattern designated as a "serovar" (see Figure 6). In the collection of 62 strains ultimately analyzed, 16 unique serovars were observed, thereby demonstrating the enormous degree of serological heterogeneity displayed by this membrane protein. The number of positive reactions observed among individual serovars ranged from between 1 to 7.

The full listing of serovar found for each strain is presented in Figure 12. As can be seen 12 strains (19 % of the strains analysed) did not react with any of the panel of 13 monoclonal antibodies (denoted by "NR" non-reactive) and were deemed non-typable. Taken together with the strains that were ommitted because of lack of or low level of expression (15 strains), a total of 22% of the strains available could not be typed. The frequency of occurrence of the serovars among the 78% of strains that could be unequivocably typed varied considerably. Only single representatives of serovars 6, 8 and 9 were observed whereas 10 strains were of serovar 2, the most common serovar. There were strong correlations between the family and genotype of the Osp C protein and its serovar. Indeed in most cases there seemed to be a one to one relationship e.g for families 1, 2, 4, 5, 7, 9, 10, 14, and 15. Families 3, 12, 13, 17, 18 and 19 could either not be tested or were non typable using the monoclonal antibodies currently available. Families 6, 8 and 11 could be futher subdivided serologically into 2 or 3 serovars, however it is interesting to note that the genotypes of these families also showed some diversity. One serovar (serovar 16) was observed in more that one family (families 16 and 20). This-might have occurred because there are only two positive reactions in this serovar and thus the current monoclonal antibodies were not able to discriminate between the two families.

### Example 3: Restriction Fragment Length Polymorphism (RFLP) analysis of ospC heterogeneity

The *ospC* gene from strain Orth was cloned and the nucleotide sequence determined as previously described (U.S. application, serial No. 07/903,580). Oligonucleotides corresponding to the proximal (coding strand, ATGAAAAAGAATACATTAGTGC, start codon underlined) and distal (non-coding strand, TAATTAAGGTTTTTTGGAGTTTCTG, stop codon underlined) ends of the *ospC* gene from strain Orth were then used in the polymerase chain reaction (see example 4) to amplify the *ospC* genes from 77 strains in our culture collection. All strains tested, including 14 strains from the United States, yielded PCR fragments of the predicted size (627-642bp) indicating that the plasmid-encoded *ospC* gene is not only stably maintained but is much more prevalent than previously supposed. The failure to detect the OspC antigen in *in vitro* grown cultures is unlikely to be due to the absence of the *ospC* gene but rather to the absence or low level of antigen being expressed.

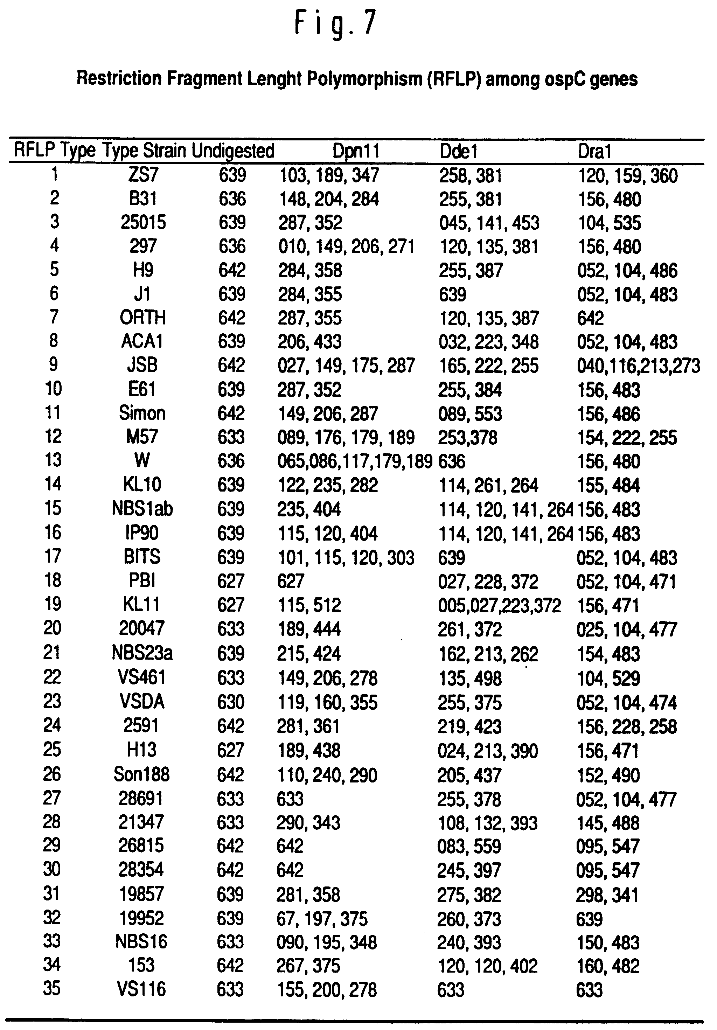

The polymorphism among *ospC* genes from different strains was determined by analysis of the restriction fragment patterns obtained after digestion of the PCR amplified *ospC* gene (prepared as described above) with the restriction enzymes *Dpn*11, *Dde*1 and *Dra*1. An analysis of the data from the 82 strains (i.e. experimental data from all 77 strains in our culture collection plus information deduced from 5 published *ospC* sequences; see Figure 1) revealed the presence of 35 distinct RFLP *ospC* types. The number and sizes of the fragments experimentally determined using standard procedures, was confirmed in many instances by sequencing i.e. for at least one representative of RFLP types 1-23, type 24 is based on the sequence data of Padula et al. The RFLP patterns associated with each RFLP type are shown in Figure 7. Where available, the fragment sizes deduced from sequence information has been presented (RFLP types 1-24) in preference to the measured values. A complete listing of the RFLP-types for each strain analysed is given in Figure 12.

### Example 4: PCR amplification and nucleotide sequencing of different alleles of the ospC gene and cluster analysis of the deduced amino acid sequences

As described in Examples 1 and 2, and summarized in Figure 12, it was possible to classify *Borrelia* strains into OspC serovars and *ospC* RFLP-types. Strains representing *ospC* RFLP-types 1-17 and 19-23 were selected, the *ospC* gene was amplified by the polymerase chain reaction and the nucleotide and deduced amino acid sequence determined. In several cases, the relationship between closely related OspC proteins was investigated as a further check on the validity of the typing systems and to check for further undetected heterogeneity within OspC types. A total of 27 *ospC* genes were PCR amplified and sequenced as described below. The sequence information has been used to classify OspC proteins into OspC families.

### Materials and Methods

A frozen *Borrelia* stock cell-suspension was thawed and 2µl (5x10⁶ -1x10⁸ cells/ml) was centrifuged for 5 minutes at top speed in a Heraeus Biofuge A microfuge. The cell pellet was resuspended in 10µl of 1x TAQ-buffer (Boehringer Mannheim), overlaid with 50µl mineral oil (Pharmacia), then incubated in a boiling water bath for 8 minutes and placed immediately on ice. To the cell-lysate was added 90µl of a reagent mixture [9µl 10x Taq polymerase buffer, Boehringer Mannheim; 2µl 10mM dNTP solution, Boehringer Mannheim; 5µl primer 1 (ATGAAAAAGAATACATTAAGTGCG), 10mM stock; 5µl primer 2 (ATTAAGGTTTTTTTGGAGTTTCTG), 10mM stock; 0,5µl 5,000U/ml Taq polymerase, Boehringer Mannheim; and 68.5µl H₂O]. DNA amplification was performed in a LKB Thermocycler (95°C for 36 seconds, 53°C for 60 seconds, 70°C for 84 seconds, 30 cycles). Amplification was monitored by analyzing 5µl of the product on a 1%(w/v) agarose gel in Tris-Acetate buffer (40mM Tris acetate, 2mM EDTA, pH 8.0), staining with ethidium bromide and visualization under UV light. Amplified products were concentrated using Spin Bind microcentrifugation cartridges (PMC). DNA then was collected in 30 µl H₂O and recovery was monitored by running 2µl of the purified product on an agarose gel as described above.

Amplified DNA fragments (2-7µl) were prepared for sequencing on a LKB Thermocycler (25 cycles at 95°C for 36 seconds, 53°C for 30 seconds, 70°C for 80 seconds) using the Auto Cycle Sequencing kit (Pharmacia) with the fluorescein labeled primers 5'-ATGAAAAAGAATACATTAAGTGCG-3' and 5'-ATTAAGGTTTTTTTGGAGTTTCTG-3'. Samples were electrophoresed on a 6% polyacrylamide sequencing gel using an automated laser fluorescent [ALF] sequencing apparatus (Pharmacia LKB) as specified by the manufacturer. The nucleotide sequence data files from the ALF were collated and analyzed using the software package DNASIS and the deduced protein sequences with PROSIS (Pharmacia-LKB).

The amino acid sequences for the OspC proteins from 24 different strains of the Lyme disease spirochetes (i.e. 22 sequences from this study and 2 published sequences for strains 2591 and PBI) were aligned by the fast/approximate method of Wilbur and Lipman, PNAS USA 80: 726-30 (1983), and the similarity scores thus generated were used to construct a dendrogram by the UPGMA method (a form of cluster analysis) of Sneath and Sokal, supra. These analyses were performed using the software package Clustal V (Higgins and Sharp, CABIOS 5: 151-53 (1989); Higgins et al., CABIOS (1991).

### Results and Discussion

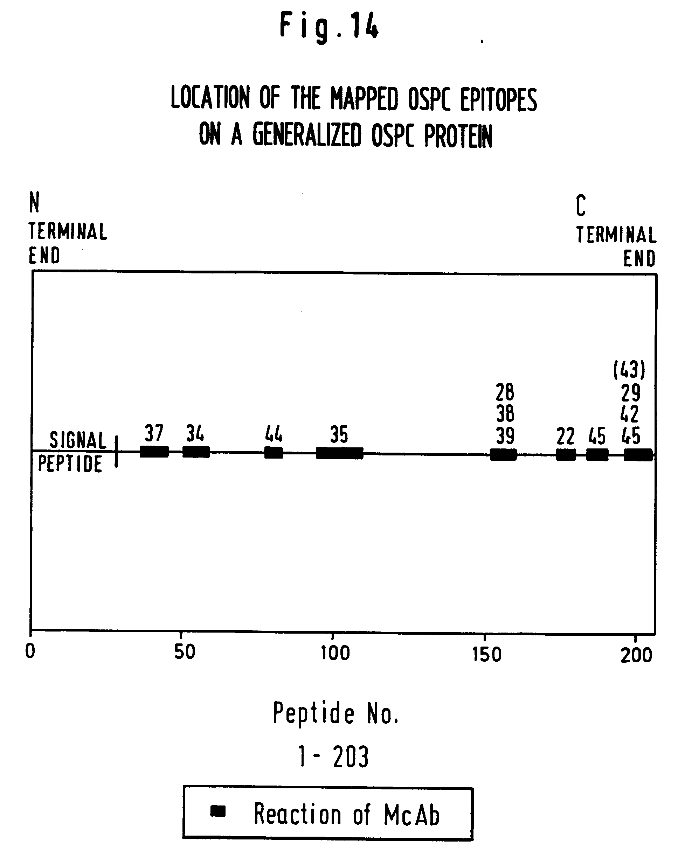

The aligned, nucleotide and deduced partial amino acid sequences for the *ospC* genes and proteins from 24 strains, representing 24 different RFLP-types, are shown in Figures 8 and 9. Since the amino acids preceding the first cysteine residue (amino acid 19 in the Orth sequence) in the OspC protein are the leader sequence and not present in the mature protein (the sequence FISC is a putative signal peptidase cleavage site) they were not included in the sequence comparison. At the carboxy terminal end of the protein, the last 16 amino acids were excluded. This includes the region corresponding to the binding site of primer 2 (equivalent to last 7 amino acids) and then a gap of 9 further amino acids until the first sequence data were obtained. This terminal portion of the OspC genes appears to be highly conserved and of minor importance in generating the diversity observed among the OspC proteins as indicated by the ability to amplify and sequence the *ospC* gene from all strains tested using primer 2. Moreover, monoclonal antibodies which bind to this region of OspC are broadly reactive (for example, BBM 29, 42 and 45 in Figures 13 and 14.

The OspC sequences are highly variable with the most distantly related amino acid sequences *(B.burgdorferi* strain 297 and *B.garinii* strain IP90) showing only 59% amino acid sequence identity (80% similarity). However, no sequence differences were detected between members of the same RFLP-type indicating that this typing method very accurately represents the heterogeneity among *ospC* genes (i.e. the OspC sequences for RFLP-type 1 strains VS215, VS219 and DK7 are identical to that of ZS7; RFLP-type 2 strains IP2 and 26816 are identical to B31; RFLP-type 6 strains H15 and ACA1 are the same; RFLP-type 7 strains PKO and DK26 are identical to JSB; RFLP-type 10 strains H4 and W are the same; RFLP-type 13 strains 871104 and KL11 are identical; RFLP-type 14 strains 20047 and VS185 are the same).

The degree of relatedness between the partial OspC amino acid sequences determined by cluster analysis is presented as a dendrogram in Figure 10. OspC proteins from strains of the same species are more closely related to each other than to OspC proteins from different species. Nevertheless, even within one species, considerable variability is evident. The OspC sequence diversity is particularly high among the *B.garinii* strains, as indicated by the deeper branching observed within this part of the phylogenetic tree and the larger number of OspC variants associated with *B.garinii* than with the other two *Borrelia* species. The clonal structure of *ospC* inheritance suggests that there has been no significant exchange of genetic material, either by intragenic recombination or horizontal transfer of the plasmid-encoded *ospC,* between the different Lyme disease *Borrelia* species.

OspC proteins have been assigned to OspC families, an OspC family being defined as a group of OspC proteins that have more than 80% amino acid sequence identity over the first 92% of the mature OspC protein i.e. excluding the information for the 18aa leader sequence and the final 16aa. Eighteen different OspC families are depicted in Figure 1 but two further OspC families (19 and 20) have been identified from incomplete sequence information for the OspC proteins from strains H13 and 28691 which has not been included in the dendrogram.

Despite the great diversity among the OspC proteins, the first third of the mature OspC protein is conserved (Figure 10), with strains of the same species showing around 80-90% sequence identity in this region. However, the sequence identity between OspC proteins from different species is not so high in this part of the protein due to the presence of species-specific sequence motifs at the amino-terminal end of the OspC protein. As indicated above, the carboxy-terminal portion of OspC, which has not been shown, is also apparently highly conserved. The intervening region (i.e. the lower two blocks of Figure 9 between amino acid residues KKI and NS) is highly variable and the major source of diversity associated with OspC. It is to be expected that serotype-specific epitopes would lie within this variable region. Analysis of the hydrophilicity profiles of the individual protein sequences, by the method of Hopp and Woods, found that the highest hydrophilic peak, highly predictive of the existence of an epitope, lies within this region. More specifically, despite the great variability between the OspC sequences in this region a putative epitope invariably lay between amino acid residues 120-155 of the mature protein. In the OspC of strain Orth, the hydrophilic peak occurs at residues 136-141 (DNDSKE), a region of high flexibility and a predicted β-turn, parameters which would also be indicative of an epitope (analyses done using PC/GENE).

### Example 5: Epitope Mapping of the Anti-Ospc Monoclonal

The epitopes of certain of the anti-OspC monoclonal antibodies were mapped using a commercial available Custom Designated Epitope Scanning kit from Cambridge Research Biochemicals Ltd., Gradbrook Park, Northwich, Cheshire, England which uses either the pin technology method described by Geysen et al., J. Immunol. Methods 102: 259-74 (1987) or an biotinylated peptide ELISA or Dot Blot method described by the manufacturer. The 2026 custom synthesized peptides tested were single step, overlapping 10mers of the OspC proteins sequences shown in Figure 9. Overlapping peptides of the signal peptide sequence of strain Orth and the C-terminal ends of the OspC proteins of Strains Orth PKO and B31 were also included in the analysis.

The combined sequence of sequential peptides reacting with a monoclonal antibody is described as a "full epitope sequence". Figure 13 lists the full epitope sequence of those monoclonal antibodies for which epitopes could be discerned. The sequence enclosed within brackets, [ ], includes the amino acids common to all reacting peptides and therefore form an important part of the epitope. The location of each full sequence within a generalized OspC protein the protein is shown in Figure 14. In one instance, e.g. with, a number of epitopes could be discerned, however, only that for the primary epitope, i.e., the most highly reactive, is given (Figure 13) and shown (Figure 14). In cases where the monoclonal reacted with peptides corresponding to similar regions in more than one Osp C protein only that for the Orth strain is given. Conversely, where the monoclonal antibody does not react with the Orth protein (e.g. BBM 43) the reacting sequence given is that for the homologous strain (PKO). At the bottom of Figure 13 the monoclonal antibodies are grouped into categories based on the frequency of occurrence of the epitope they recognize which are shown in the upper part of the figure. As can be seen, over half of them recognize highly-specific epitopes, in that they occur in fewer than ten of the strains analyzed. Five of the monoclonal antibodies recognize epitopes of intermediate occurrence, while the seven remaining can be considered to recognize common epitopes because they occur in more than twenty five of the of the 77 strains analyzed. The monoclonal antibodies which were found to be suitable for the serovar analysis are denoted in Figure 13 by an asterix.
It is interesting to note that it was primarily the monoclonal antibodies that recognize common epitopes (BBM 28, 29, 34, 37, 42, 43, and 45) or those of intermediate occurrence (BBM 22, 35, and 40) which could be unequivocally mapped. Indeed only three monoclonal antibodies which could be considered as type specific (BBM 38, 39 and 44), i.e. reacting with fewer than 10 strains, could be mapped. Both BBM 38 and 39 have the same strain reaction pattern and mapped to the same region (amino acid 155 and 170) . Based on hydrophilicity plots of the amino acid sequence of the Orth protein, a hydrophilic peak and predicted β turn coincides with this region, parameters highly indicative of an epitope. The epitope of BBM 44 lies between amino acid 79 to 90, also an area of considerable variation. Unfortunately none of the epitopes of the other type specific monoclonal antibodies could be mapped, suggesting that they are dependent on the confirmation of the molecule. However, since all 3 type specific antibodies map to regions that are among the most variable of the protein, it is highly likely that it is also involved in other type specific epitopes. Interestingly, BBM 28 which reacts with an epitope of high frequency also maps to same regions as BBM 38 and 39. The reasons for this is unknown however there may be slight differences in the number and the actual amino acids involved at the binding site which bring about this ambiguity.
Four of the antibodies (BBM 29, 42, 43 and 45) which react with common epitope map at the distial C-terminal end of the protein (amino acids 200 to 212), where as two others react close to the N terminal end of the protein (amino acids 41 to 67), regions which have been shown to be highly conserved. The monoclonal antibodies recognizing epitopes with intermediate occurrence mapped within the semi- conserved regions (amino acids 103 to 114 and amino acids 176 to 196) of the molecule.
These result were also confirmed in a further experiment where polyvalent rabbit sera specific for membrane 2 fractions of strains expressing each of the 16 serovar variants of the Osp C protein were screened against the 203 overlapping peptides of the Orth protein. All common, cross reacting epitopes were found within the conserved and semi-conserved regions outlined above. Interestingly sera from strains of CMAT group 1 (B. burgdorferi sensu stricto) did not cross react as frequently as those sera from strains of CMAT groups 3 (*B. afzelii)* and 4 *(B. garinii)*

### Example 6: Cross-Protection Studies in Gerbils

To ascertain whether cross-protection between different OspC families was possible, OspC proteins were purified from *B.burgdorferi* strain ZS7 (OspC family 1), *B.afzelii* strain PKO (OspC family 7) and *B.garinii* strain W (OspC family 10) and used as immunogens in the gerbil model of Lyme borreliosis against a challenge with *B.afzelii* strain Orth (OspC family 5). To test protection within a family, the OspC from strain H7 (OspC family 5) was used as an immunoqen against strain Orth. The OspC from strain H7 belongs to the same serovar and RFLP-type as the OspC from strain Orth.

Gerbils were given either a single, subcutaneous immunization of purified OspC (20µg protein/200µl, adjuvanted with TiterMax #R-1 (CytRx), that is, were prepared as a water-in-oil (squalene) emulsion with a synthetic immunomodulator (copolymer CRL89-41) or two intraperitoneal injections of ospC (10µg protein/500p1) adjuvanted with aluminium hydroxide. The purified antigens were prepared from strains by methods described in U.S. patent application No. 07/903,580, the contents of which were previously incorporated above by reference. Three and a half weeks after the first immunization, blood samples were taken from the eye and plasma prepared so that the antibody response to the immunogen at the time of the challenge could be ascertained (unimmunized control animals were likewise treated). Four weeks after the first immunization, the animals were challenged intraperitoneally with 10⁴ cells (25-100 ID₅₀) of strain Orth, as were a group of unimmunized control animals. The challenge suspension was also titrated in unimmunized gerbils to determine the dose required to infect 50% of the animals. After a further two weeks, the animals challenged with the 10⁴ dose were killed and the bladder, heart, kidneys and spleens cultured in BSK medium. Cultures were inspected for spirochetes at weekly intervals, from the second to the sixth week post-inoculation, by dark-field microscopy. Blood also was taken and the resultant plasma analyzed by western blotting for sero-conversion, i.e. the development of antibodies post-challenge to antigens from strain Orth other than the immunogen. There was good agreement between the cultural and serological tests used to ascertain which animals were infected. Only serological testing was used for the ID(50) determinations but in this instance the animals were bled three weeks post-challenge, the extra time being given to ensure that the antibody response in infected gerbils was sufficiently strong to be easily detected.

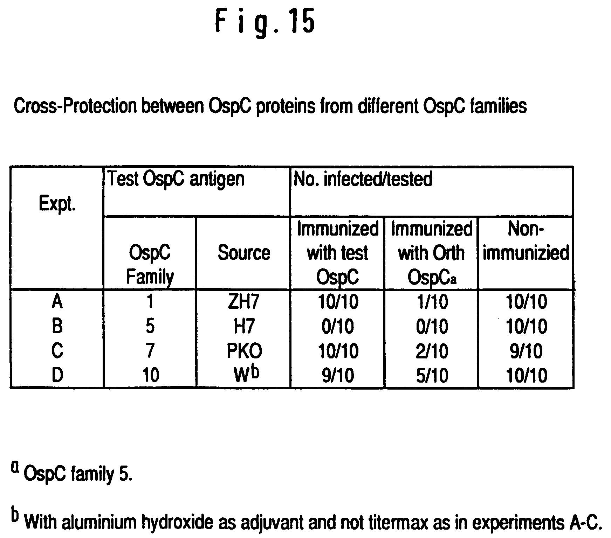

There were no signs of cross-protection between species i.e. OspC proteins from OspC families 1 (*B.burgdorferi*) and 10 *(B.garinii)* were ineffective as immunogens against the challenge strain Orth *(B.afzelii).* Likewise there was no sign of cross-protection between different OspC families of the same species i.e. the OspC protein from an OspC family 7 isolate *(B.afzelii strain* PKO) was ineffective as an immunogen against a challenge with strain Orth which expresses an OspC protein from OspC family 5. By contrast, immunization with the OspC protein from strain H7 (OspC family 5) was effective against a challenge strain Orth (OspC family 5). These data (Figure 15) indicate that cross-protection between the OspC families is unlikely and that protection within a family is possible. A multivalent vaccine comprising one or more types of OspC proteins from each of the OspC families should be sufficient to protect against most Lyme disease *Borrelia* strains.

### Example 7: Frequency of occurence geographical distribution of various families of OspC proteins associated with human disease

Due to the high degree of variability of the OspC protein, it is extremely difficult to design vaccine formulations which give good protective coverage, yet do not require the inclusion of excessively large numbers of variants in order to achieve this goal. One way of optimizing the selection of OspC variants would be to determine which OspC variants are associated with human disease and occur with a high frequency. Rare OspC variants or OspC variants rarely associated with human disease would thus be excluded from any vaccine formulation with minimal loss of vaccine efficacy. Furthermore, if the vaccine is designed only for use in a particular geographic region, it would be unneccessary to include those OspC variants not prevalent among the Lyme disease *Borrelia* of that region. Using the epidemiological and OspC typing information on the *Borrelia* strains used in this study (Figure 12) it has been possible to make selections on the OspC variants should be included in an OspC vaccine(s).
An analysis of the *B.burgdorferi* isolates (CMAT group 1 including strain 25015; total 23 strains) shows that the most prevalent OspC variants among those strains are those belonging to families 1 and 2. Family 1 strains are all European isolates and may cause human disease, although only 1 of the 5 strains was a human isolate which may be an indication that these strains are not highly virulent. Family 2 strains are the single most common type of OspC family with 10 members. Unlike family 1 strains, these strains are widely distributed with isolates from the United States, Europe and Russia. Family 2 strains are clearly associated with human disease with 50% of the isolates being clinical specimens. The remaining 8 *B.burgdorferi* isolates tested are all United States isolates and they are very diverse in terms of the OspC that they express, since each strain expresses a different OspC RFLP type. Only one of these strains (strain 297, family 3) was isolated from a case of Lyme disease. The family 2 and 3 strains belong, with the exception of strain 25015, to one of the 3 major human disease related clones CMAT type 1.2.4. (Figure 5).
The 26 strains of *B.afzelii* (i.e. Group VS461, CMAT group 3) fall into 6 discrete families; OspC families 4-8 and family 16 with 5, 4, 6, 2, or 4 members respectively. Except for one Japanese isolate, all the strains were from Europe; Austria (14), Czech Republic (4), Denmark (1), Germany (2), Italy (1) Slovenia(1), Sweden (1) and Switzerland(1). Eighty-eight percent of the European isolates were of human origin, with 80% of the isolates being from skin biopsies and 8% from blood samples. These data reflect a strong association between *B.afzelii* strains and the development of dermatological forms of Lyme disease. The human isolates were distributed evenly throughout the various OspC families. The Austrian isolates belonged predominantly to families 4-6 (86%) but single representatives were also found in families 7 and 8. The low incidence of Austrian isolates among OspC family 7 (i.e. 1/5) and the absence of isolates from family 16 (0/4) suggests that within Europe there are geographical variations in the prevalence of the various *B.afzelii* OspC families. Nevertheless, *B.afzelii* strains from the OspC families 4-8 and 16 are widely scattered throughout Europe. These strains are almost exclusively members of another major human disease related clone, namely 3.2.13. (Figure 5).

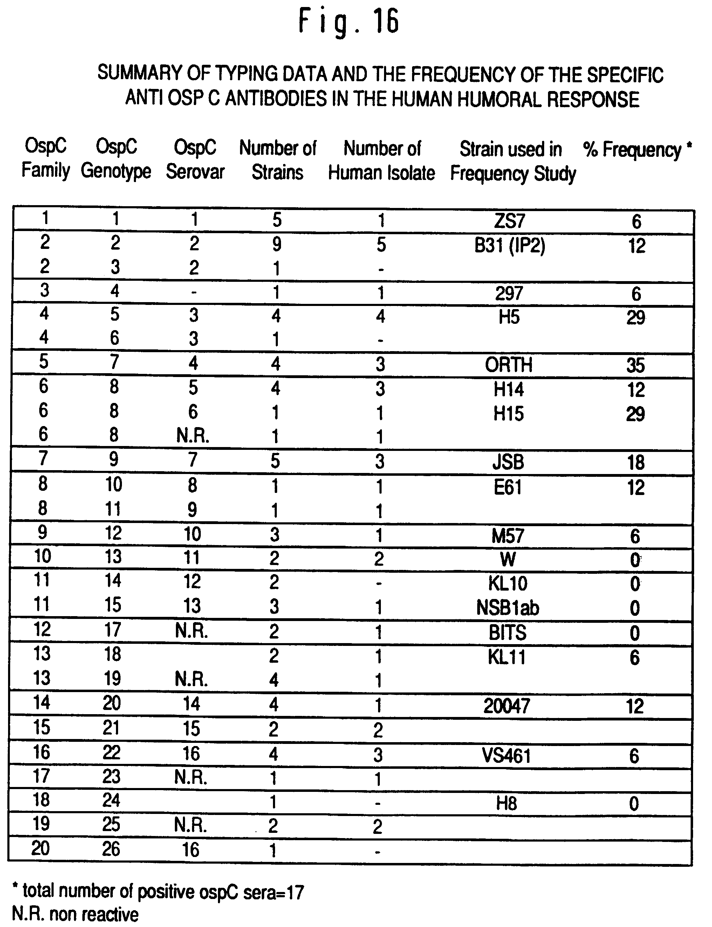

Thirty *B.garinii* strains (i.e CMAT group 4, excluding atypical strains 19857 and 19952, and CMAT 2 strains 20047, IP90 and NBS16) can be sub-divided into 9 OspC families plus 2 RFLP types for which there is no family assignment. Fifty-three percent of the *B.garinii* strains tested were human isolates and these strains are distributed throughout all but one (RFLP 34) of the OspC types. Seventy-five percent (12/16) of these *B.garinii* strains were isolated from cases of neuroborreliosis with the remainder being skin isolates. Therefore, *B.garinii* is primarily associated with neuroborreliosis, although the occurence of skin isolates is to be expected since the development of a skin-lesion (EM) is a manifestation common to all forms of Lyme disease irrespective of the causative agent. Strains of OspC family 13 were the most commonly isolated OspC type, accounting for 23%(7/30) of the total *B.garinii* OspC types and 25% (4/16) of the human isolates. Strains of this OspC family are widespread within Europe and include isolates from 6 different countries. OspC family 11 is also widely distributed and occurs reasonably frequently (17% or 5/30) but the association with human disease is less clear, since only one isolate was of human origin, but this may reflect sampling error and the small numbers of strains analysed. These latter comments are also applicable to strains of OspC family 14 (4 strains but only 1 human isolate). Isolates from the other OspC families ( 9,10,12,15,17,19 and RFLP types 33 and 34) were found at a lower frequency. However, for a vaccine against Austrian *B.garinii* strains OspC families 10 and 19 should be considered, in addition, since all 4 *B.garinii* isolates from Austria belonged to these 2 OspC families.
In addition, to the direct analysis of *Borrelia* strains, as described above, it is also possible to determine the prevalence of the various OspC variants and their association with human borreliosis indirectly. This can be achieved by testing the specificity of OspC antibodies in the serum from Lyme disease patients. Fifty sera taken from patients in the Prague area (Czech Republic) suffering from EM (15), ACA (15), neurological disorders(10) or joint and muscle associated syndromes (10) have been tested for antibodies to OspC. When screened against a panel of 18 strains, 17 (34%) of the sera (3 EM, 10 ACA, 3 Lyme arthritis and 1 neuroborreliosis) were found to react with one or more of the 16 OspC families tested (Figure 16). Twelve of the sera reacted specifically with just one OspC [family 5 (4x), family 7 ( 3x), family 6 (2x), family 8, 13 and 14 (1x)] Three sera reacted with both families 4 and 6 while 2 other sera were broadly cross reactive reacting with 6 and 8 of the families tested. Therefore, *Borrelia* strains expressing OspC variants from OspC families 5-7, 13, 14 and probably 4 are present in the Czech Republic. This serological data is consistent with, and therefore substantiates, the results obtained from the strain analysis for the neighbouring country Austria.

Based upon the results described above, vaccines have been formulated for use in;
1) United States; OspCs from OspC families 2 and 3
2) Europe; 14 OspC variants from OspC families 2, 4-7, 9, 10, 12, 13, 14, 15-17 and 19
3) Austria; 8 or more OspCs from OspC families 2,4-7,10,13 and 19.

### Example 8: Expression of recombinant OspC in P.pastoris

### Construction of the Pichia pastoris OspC expression vector

The recombinant *P.pastoris/E.coli* shuttle vector pHIL-A1 (provided from Phillips Petroleum) was used to clone the OspC coding sequence of *B.burgdorferi.* A panel of strains comprising one or more representatives from each family was selected and the OspC gene was amplified by the polymerase chain reaction. The coding sequence of the mature OspC protein starting with the first cysteine amono acid (amino acid 19 in the OspC protein sequence from strain Orth) was amplified by using the strain specific primers deduced from the OspC nucleotide sequences as disclosed USSN 07/903,580 (EP 0 522 560).
To create the 5' and 3' end of the *B.burgdorferi* Orth OspC gene, the polymerase chain reaction (PCR) was carried essentially as described in Example 4 using the amino-terminal primer PC-F with the sequence 5'-AAATGTGTAATAATTCA GGGAAAGG-3', and the carboxy-terminal primer PC-B with the sequence 5'-ATTAAGGTTTTTTTGGAGTTTCTG-3'. The underlined sequence in primer PC-F is identical to the translation start codon of the mature OspC protein and in primer PC-B to the translation stop codon, respectively.

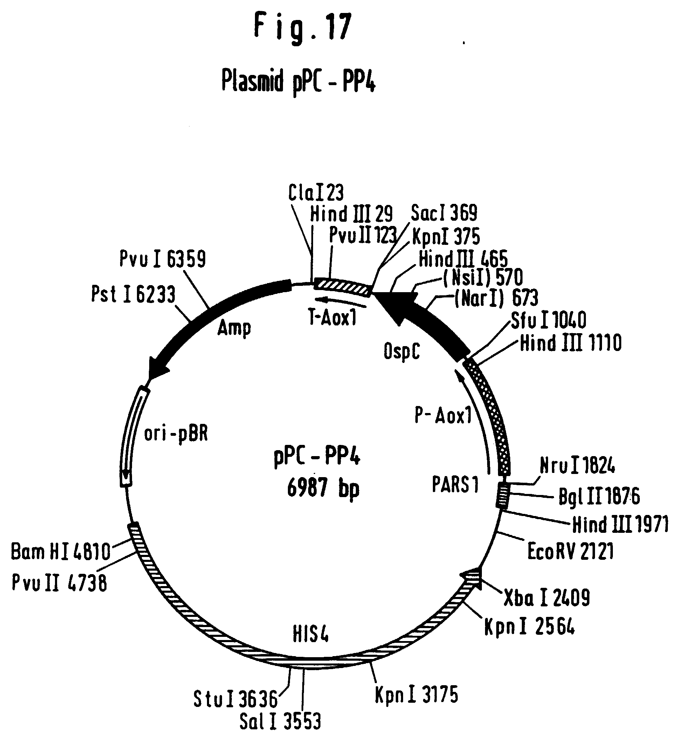

Cloning strategies (restriction site in the vector and primers used for PCR) for inserting OspC coding sequence of *B.burgdorferi* strain B31, PKO, ZS7, KL10 and E61 in pHIL-A1 are summarized in the Table 1 below.
The vector pHIL-A1 was digested with SfuI and overhanging ends were filled in with Klenow polymerase to create blunt ends. The purified PCR fragment containing the OspC coding seuqence was ligated with the vector overnight. The ligation mixture was used to transform competent *E.coli* DH5α and amicillin resistant colonies were selected on LB-Amp-plates for further plasmid amplification. Mini-preparations (Maniatis et al. 1982) were screened and analysed by restriction fragment length and the plasmid having the OspC gene was labelled pPC-PP4 (Figure 17). Purified pPC-PP4 DNA was prepared and the sequence was confirmed by DNA sequencing. The purified plasmid pPC-PP4 was transformed in *P. pastoris* strain GS115 NRRL-Y 11430 (Cregg et al. Mol. Cell. Biol. 5: 3376-3385 (1985)) by the method described by Dohmen et al. 1991 (Yeast 7: 691-692) and transformants were selected on MD plates.

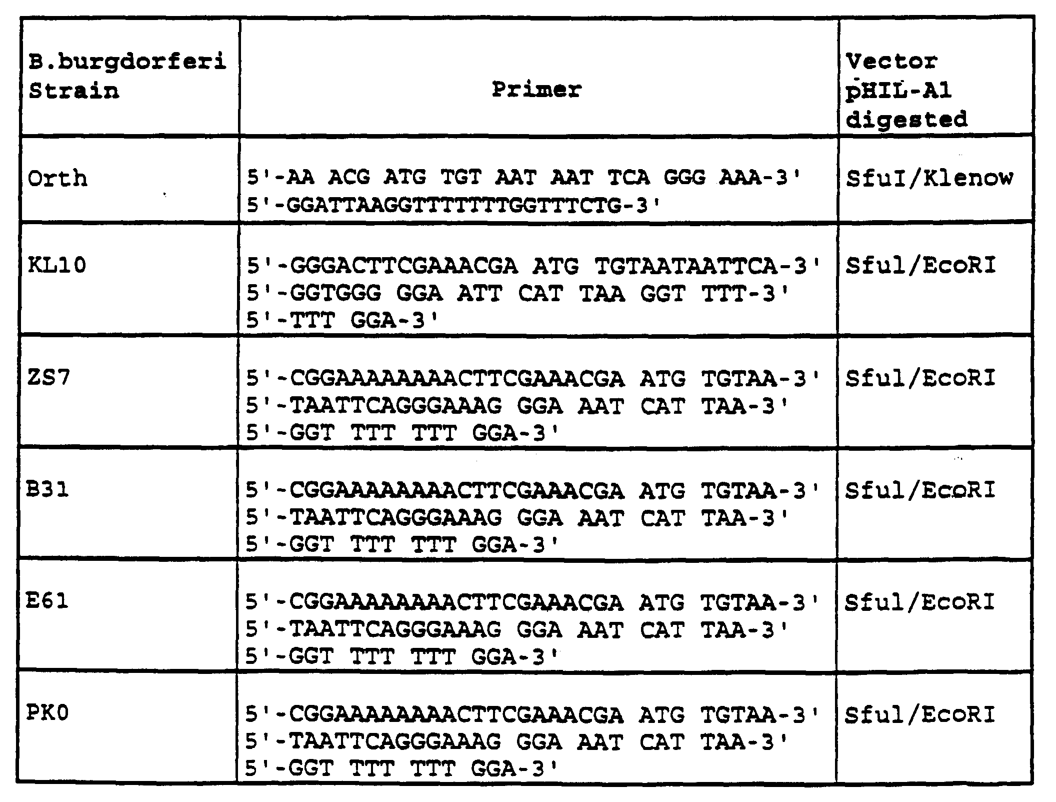

Table 1 shows the oligonucleotide primers used for the PCR amplification of the coding sequence of the mature OspC protein of different Lyme disease Borrelia strains. The restriction enzymes used for insertion of the OspC coding sequence in the pHIL-A1 vector are also given.

### Expression of recombinant OspC⁻in P.pastoris

*P.pastoris* GS115/pPC-PP4 transformants were picked from MD plates and grown in 3 ml MG medium at 30°C with constant agitation to an optical density (OD 600) of 2-10. For induction of OspC synthesis, one ml of the culture was spun down, washed once with MM, resuspended in 3 ml of MM or MMY¹-medium and incubated for 2 days at 30°C with constant agitation. Expression of OspC was induced by the presence of methanol in the growth medium. Aliquots of the culture were removed and lysates were Western blot analysed using OspC specific monoclonal antibody BBM 45
[¹ all of the following media are expressed in terms of quantity /1
MD: Yeast nitrogen base (YNB, 13.4 g), ammonium sulfate (5 g), biotin (400 mg), glucose (2%)
MG: YNB, ammonium sulftate, biotin, glycerol (10ml/l), potassium phosphate (100 mM)
MM: YNB, ammonium sulftate, biotin, methanol (5 ml), potassium phosphate (100 mM)
MMY: MM, yeast extract (10 g), casein (20 g)]

### Purification of recombinant OspC

*P.pastoris* GS115/pPC-PP4 cell were harvested by centrifugation (3000g, 5 min, 4°C). The washed cells were resuspended in 150 mM Tris/HCl buffer, pH 7.4 and the suspension was added to glass beads. The cells were then lysed by shaking the mixtures in a Vibrogen® cell-mill (Model V14, Bühler). The lysate was then filtered on a sintered glass filter to remove the glass beads. The lysate was centrifuged for 5 min at 3000g at 4°C. The supernatant was further centrifuged for 1 hour at 100,000g at 4°C. The "high speed supernatant" was used for further purification of the OspC antigen.
OspC antigens were fractionated by DEAE-chromatography, as exemplified below:
Column: Protein-PAK DEAE 5PW from Waters
Sample: 45 ml dialysed antigen preparation
Equilibration buffer (A): 10 mM Tris/HCl pH 7.5
Eluation buffer (B): 10 mM Tris/HCl, 1 M NaCl, pH 7.5
Flow rate: 4 ml/min
Gradient: 0% B for 70 min, 0-100% for 50 min
The column was equilibrated with buffer A and the antigens eluted with increasing amounts of NaCl. To identify fractions containing the antigen of interest, aliquots of fractions were precipitated with acetone and the pellets were analyzed by SDS-PAGE and/or immunoblotting.
Fractions from the DEAE ion-exchange chromatography separation enriched for OspC antigen were further purified by immobilized metal-affinity chromatography as described in EP 0 522 560.

### Large scale production of OspC protein in the fermenter

The production of OspC was examined in continuous fermentation run. Each run was performed using a fermenter equipped with monitors and controls for pH, dissolved oxygen, agitator speed, temperature, air flow and oxygen flow. Temperature was held at 30°C. Cell yield was determined from washed cell wet weight.
Inocula for the fermenter runs were grown in 2 1 Erlenmeyer flasks containing 500 ml of modified FM21 medium as disclosed in EP 0 263 311. The fermenter cultures grown in the batch mode were propagated with glycerol as sole source of carbon and energy. Continous cultures were established with constant glycerol feed until a biomass concentration of 500-700 g wet cell weight/litre was reached. Once baseline control samples were taken, methanol was added to the culture as methanol-salts-biotin feed over a period of several days to keep the methanol concentration between 0,05 and 1,5 %. Produced biomass were removed every day. *P.pastoris* cells were collected by centrifugation and resuspended in buffer (150 mM Tris/HCl, 2 mM EDTA, 1 mM benzamidine hydrochloride, 0,1% NaN₃, pH 7.4). Cell lysates were obtained by using French press. OspC protein concentration was determined by the method of Bradford. Preliminary results showed an antigen production of about 100 fold increased yield of OspC antigen (per unit volume culture)derived from *P.pastoris* compared to the yields obtained from the *B.burgdorferi* strains.

### Immunization and challenge (protection) studies of the P.pastoris derived OspC antigen

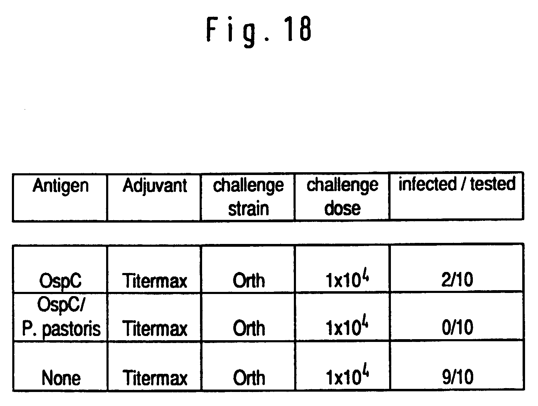

Protection studies were performed in gerbils as described in example 5. Twenty microgram amounts of OspC from *P.pastoris* (cloned from strain Orth)or *Borrelia* strain Orth were tested for their protective efficacy as shown in Figure 18. All gerbils immunized with *P.pastoris* derived OspC were protected against a challenge with the homologous strain Orth and results are comparative to protection studies obtained with *Borrelia* derived OspC antigen.

## Claims

1. An immunogenic composition comprising
(a) an amount of material comprising (i) one or more OspC antigens of Lyme disease Borrelia substantially purified from each one of the 20 currently recognized OspC families of Figure 11 or (ii) OspC variants or OspC mimetics of said OspC antigens, said OspC variants or OspC mimetics having a structure that is sufficiently similar to native OspC to induce the production of protective immunity; and
(b) a physiologically-acceptable excipient therefore, wherein said amount is sufficient to elicit, in a mammal susceptible to Lyme borreliosis, an immune response that is protective against Lyme borreliosis.

2. An immunogenic composition comprising
(a) an amount of material comprising (i) two or more OspC antigens of Lyme disease Borrelia from each one of the 20 currently recognized OspC families of Figure 11 or (ii) OspC variants or OspC mimetics of said OspC antigens, said OspC variants or OspC mimetics having a structure that is sufficiently similar to native OspC to induce the production of protective immunity; and
(b) a physiologically-acceptable excipient therefore, wherein said amount is sufficient to elicit, in a mammal susceptible to Lyme borreliosis, an immune response that is protective against Lyme borreliosis.

3. An immunogenic composition comprising
(a) an amount of material comprising (i) one or more OspC antigens of Lyme disease Borrelia substantially purified from each one of the Ospc-families of Figure 19 expressed by one or more of the human disease associated (HDA) clones and clonal clusters or (ii) OspC variants or OspC mimetics of said OspC antigens, said OspC variants or OspC mimetics having a structure that is sufficiently similar to native OspC to induce the production of protective immunity; and
(b) a physiologically-acceptable excipient therefore, wherein said amount is sufficient to elicit, in a mammal susceptible to Lyme borreliosis, an immune response that is protective against Lyme borreliosis.

4. An immunogenic composition, comprising
(a) an amount of material comprising (i) two or more OspC antigens of Lyme disease Borrelia from each one of the OspC-families of Figure 19 expressed by one or more of the human disease associated (HDA) clones and clonal clusters or (ii) OspC variants or OspC mimetics of said OspC antigens, said OspC variants or OspC mimetics having a structure that is sufficiently similar to native OspC to induce the production of protective immunity; and
(b) a physiologically-acceptable excipient therefore, wherein said amount is sufficient to elicit, in a mammal susceptible to Lyme borreliosis, an immune response that is protective against Lyme borreliosis.

5. The immunogenic composition of claim 1 or 3 designed to protect against B. burgdorferi strains prevalent within a particular geographic region.

6. The immunogenic composition of claim 5 for use in North America, comprising
(a) an amount of material comprising (i) one or more OspC antigens of Lyme disease Borrelia substantially purified from'each of the OspC-families 2 and 3 or (ii) OspC variants or OspC mimetics of said OspC antigens, said OspC variants or OspC mimetics having a structure that is sufficiently similar to native OspC to induce the production of protective immunity; and
(b) a physiologically-acceptable excipient therefore, wherein said amount is sufficient to elicit, in a mammal susceptible to Lyme borreliosis, an immune response that is protective against Lyme borreliosis.

7. The immunogenic composition of any of the preceding claims, wherein said material comprises additionally an OspA antigen from B. burgdorferi B31.

8. The immunogenic composition of claim 5 for use in Europe, comprising
(a) an amount of material comprising (i) one or more OspC antigens of Lyme disease Borrelia substantially purified from each of the OspC-families 2, 4-7, 9, 10, 12-17 and 19 or (ii) OspC variants or OspC mimetics of said OspC antigens, said OspC variants or OspC mimetics having a structure that is sufficiently similar to native OspC to induce the production of protective immunity; and
(b) a physiologically-acceptable excipient therefore, wherein said amount is sufficient to elicit, in a mammal susceptible to Lyme, borreliosis, an immune response that is protective against Lyme borreliosis.

9. The immunogenic composition of claim 7 for use in Austria, comprising
(a) an amount of material comprising (i) one or more OspC'antigens of Lyme disease Borrelia substantially purified from each of the OspC-families 2, 4-7, 10, 13 and 19 or (ii) OspC variants or OspC mimetics of said OspC antigens, said OspC variants or OspC mimetics having a structure that is sufficiently similar to native OspC to induce the production of protective immunity; and
(b) a physiologically-acceptable excipient therefore, wherein said amount is sufficient to elicit, in a mammal susceptible to Lyme borreliosis, an immune response that is protective against Lyme borreliosis.

10. The immunogenic composition of any one of claims 8 or 9, wherein said material comprises antigens from OspA antigens from Borrelia strains B31, Orth, H4 and KL11.

11. The immunogenic composition of claim 1 or 3, wherein said material comprises antigens from (i) or (ii) purified from Lyme disease Borrelia whole cells.

12. The immunogenic composition of claim 1 or 3, wherein said material comprises antigens from (i) or (ii) which are produced by recombinant techniques in an eucaryotic host and are purified thereof.

13. The immunogenic composition of claim 12, wherein said host is a yeast.

14. The immunogenic composition of claim 13, wherein said host is P. pastoris.

15. An immunogenic composition according to claim 1 or 3 wherein said antigens comprise one or more epitopes with an amino acid sequence selected from the group consisting of 
(1) VKLSESVASLSKAA;
(2) TDNDSKEAILKTNGT;
(3) KELTSPVVAETPKKP;
(4) FVLAVKEVETL;
(5) YAISTLITEKLKAL;
(6) PNLTEISKKITDSNA;
(7) ASANSVKELTSPVV;
(8) SPVVAETPKKP;
(9) GKKIQQNNGLGA; and
(10) SPVVAESPKK,
 or variants or mimetics of such epitopes, such variants or mimetics having a structure that is sufficiently similar to the native epitope to induce the production of protective immunity.

16. An immunogenic composition according to claim 15, wherein each of the epitopes (1) to (10) is represented.

17. The immunogenic composition of claim 15 or 16, further comprising an adjuvant.

18. The immunogenic composition of claim 17, wherein said adjuvant is aluminium hydroxide.

19. The immunogenic composition of any one of the preceding claims, wherein said protective amount is in the range of 1 to 100 micrograms per antigen per dose.

20. The immunogenic composition of claim 19, wherein said protective amount is in the range of 10 to 50 micrograms per antigen per dose.

21. The immunogenic composition of any one of the preceding claims, wherein said mammal is a human.

22. The immunogenic composition of any one of the preceding claims, wherein said protective amount is either preventative or ameliorative of Lyme borreliosis.

23. A recombinant DNA sequence comprising a nucleotide sequence of any one of the OspC gene sequences from strains 28691, 25015, SZ7, 297, SIMON, E61, ACA1, H9, J1, VS461, M57, W, VSDA, NBS23a, 20047, KL10, IP90, NBS1AB, BITS, KL11 and H13 as shown in Fig. 8a.

24. A recombinant DNA sequence encoding for OspC antigens of strains 28691, 25015, SZ7, 297, SIMON, E61, ACA1, H9, J1, VS461, M57, W, VSDA, NBS23a, 20047, KL10, IP90, NBS1AB, BITS, KL11 and H13 as shown in Fig. 9a.

25. A recombinant DNA sequence according to claim 23, wherein said DNA sequence is at least 80% homologous to any one of the OspC gene sequences of Fig.8a.

26. A recombinant expression vector comprising the coding sequence of an OspC antigen of any of the 20 currently recognized OspC families of Figure 11.

27. A recombinant expression vector according to claim 26, wherein said vector comprises a recombinant DNA sequence according to claims 23 to 25.

28. The expression vector of claim 26 or 27, wherein said vector is a shuttle vector for eucaryotic/procaryotic host cells.

29. The expression vector of claim 28, wherein said vector replicates in yeast, preferentially in P, pastoris, and in E. coli.

30. The expression vector according to any one of claims 26 to 29, wherein the expression of the OspC gene is under the transcriptional control of an inducible promoter.

31. The expression vector of claim 30, wherein the expression of the OspC coding sequence is inducible by methanol.

32. A host cell transformed with an expression vector according to any one of claims 26 to 31.

33. OspC antigen as
(i) encoded by any of the sequences according to claims 23 to 25, or (ii) having a homology of at least 80%.

34. The use of a combination of antigens as comprised by the immunogenic compositions of any one of the preceding claims for the manufacture of a vaccine for the treatment or prevention of Lyme borreliosis in a mammal.

35. The use of claim 34 wherein said effective amount is in the range of 1 to 100 micrograms per antigen per dose.

36. The use of claim 35 wherein said effective amount is in the range of 10 to 50 micrograms per antigen per dose.

## Patentansprüche

1. Immunogene Zusammensetzung, enthaltend
(a) eine Menge eines Materials umfassend (i) ein oder mehrere OspC-Antigene von Lyme-Krankheit auslösenden Borrelien, im wesentlichen gereinigt aus jeder der 20 zur Zeit bekannten OspC-Familien aus Fig. 11, oder (ii) OspC-Varianten oder OspC-Nachahmer der OspC-Antigene, wobei die OspC-Varianten oder OspC-Nachahmer eine Struktur haben, die dem nativen OspC genügend ähnlich für die Induktion des Aufbaus einer Schutzimmunität ist; und
(b) einen physiologisch verträglichen Exzipienten dafür, worin die Menge ausreichend ist, in einem für Lyme-Borreliose empfindlichen Säuger eine Immunantwort auszulösen, die Schutz gegen Lyme-Borreliose verleiht.

2. Immunogene Zusammensetzung, enthaltend
(a) eine Menge eines Materials umfassend (i) zwei oder mehrere OspC-Antigene von Lyme-Krankheit auslösenden Borrelien aus jeder der 20 zur Zeit bekannten OspC-Familien aus Fig. 11, oder (ii) OspC-Varianten oder OspC-Nachahmer der OspC-Antigene, wobei die OspC-Varianten oder OspC-Nachahmer eine Struktur haben, die dem nativen OspC genügend ähnlich für die Induktion des Aufbaus einer Schutzimmunität ist; und
(b) einen physiologisch verträglichen Exzipienten dafür, worin die Menge ausreichend ist, in einem für Lyme-Borreliose empfindlichen Säuger eine Immunantwort auszulösen, die Schutz gegen Lyme-Borreliose verleiht.

3. Immunogene Zusammensetzung, enthaltend
(a) eine Menge eines Materials umfassend (i) ein oder mehrere OspC-Antigene von Lyme-Krankheit auslösenden Borrelien, im wesentlichen gereinigt aus jeweils einer der OspC-Familien aus Fig. 19, exprimiert durch ein oder mehrere mit der humanen Erkrankung assoziierter (HDS)-Clone und Clon-Cluster, oder (ii) OspC-Varianten oder OspC-Nachahmer der OspC-Antigene, wobei die OspC-Varianten oder OspC-Nachahmer eine Struktur haben, die dem nativen OspC genügend ähnlich für die Induktion des Aufbaus einer Schutzimmunität ist; und
(b) einen physiologisch verträglichen Exzipienten dafür, worin die Menge ausreichend ist, in einem für Lyme-Borreliose empfindlichen Säuger eine Immunantwort auszulösen, die Schutz gegen Lyme-Borreliose verleiht.

4. Immunogene Zusammensetzung, enthaltend
(a) eine Menge eines Materials umfassend (i) zwei oder mehrere OspC-Antigene von Lyme-Krankheit auslösenden Borrelien aus jeweils einer OspC-Familien aus Fig. 19, oder (ii) OspC-Varianten oder OspC-Nachahmer der OspC-Antigene, wobei die OspC-Varianten oder OspC-Nachahmer eine Struktur haben, die dem nativen OspC genügend ähnlich für die Induktion des Aufbaus einer Schutzimmunität ist; und
(b) einen physiologisch verträglichen Exzipienten dafür, worin die Menge ausreichend ist, in einem für Lyme-Borreliose empfindlichen Säuger eine Immunantwort auszulösen, die Schutz gegen Lyme-Borreliose verleiht.

5. Immunogene Zusammensetzung nach Anspruch 1 oder 3, entwickelt zum Schutz gegen B. burgdorferi-Stämme, die in einer bestimmten geografischen Region vorherrschen.

6. Immunogene Zusammensetzung nach Anspruch 5 zur Verwendung in Nordamerika, enthaltend
(a) eine Menge eines Materials umfassend (i) ein oder mehrere OspC-Antigene von Lyme-Krankheit auslösenden Borrelien, im wesentlichen gereinigt aus jeder der OspC-Familien 2 und 3, oder (ii) OspC-Varianten oder OspC-Nachahmer der OspC-Antigene, wobei die OspC-Varianten oder OspC-Nachahmer eine Struktur haben, die dem nativen OspC genügend ähnlich für die Induktion des Aufbaus einer Schutzimmunität ist; und
(b) einen physiologisch verträglichen Exzipienten dafür, worin die Menge ausreichend ist, in einem für Lyme-Borreliose empfindlichen Säuger eine Immunantwort auszulösen, die Schutz gegen Lyme-Borreliose verleiht.

7. Immunogene Zusammensetzung nach einem der vorhergehenden Ansprüche, worin das Material zusätzlich ein OspA-Antigen aus B. burgdorferi B31 enthält.

8. Immunogene Zusammensetzung nach Anspruch 5 zur Verwendung in Europa, enthaltend
(a) eine Menge eines Materials, umfassend (i) ein oder mehrere OspC-Antigene von Lyme-Krankheit auslösenden Borrelien, im wesentlichen gereinigt aus jeder der OspC-Familien 2, 4-7, 9, 10, 12-17 und 19, oder (ii) OspC-Varianten oder OspC-Nachahmer der OspC-Antigene, wobei die OspC-Varianten oder OspC-Nachahmer eine Struktur haben, die dem nativen OspC genügend ähnlich für die Induktion des Aufbaus einer Schutzimmunität ist; und
(b) einen physiologisch verträglichen Exzipienten dafür, worin die Menge ausreichend ist, in einem für Lyme-Borreliose empfindlichen Säuger eine Immunantwort auszulösen, die Schutz gegen Lyme-Borreliose verleiht.

9. Immunogene Zusammensetzung nach Anspruch 7 zur Verwendung in Österreich, enthaltend
(a) eine Menge eines Materials, umfassend (i) ein oder mehrere OspC-Antigene von Lyme-Krankheit auslösenden Borrelien, im wesentlichen gereinigt aus jeder der OspC-Familien 2, 4-7, 10, 13 und 19, oder OspC-Varianten oder OspC-Nachahmer der OspC-Antigene, wobei die OspC-Varianten oder OspC-Nachahmer eine Struktur haben, die dem nativen OspC genügend ähnlich für die Induktion des Aufbaus einer Schutzimmunität ist; und
(b) einen physiologisch verträglichen Exzipienten dafür, worin die Menge ausreichend ist, in einem für Lyme-Borreliose empfindlichen Säuger eine Immunantwort auszulösen, die Schutz gegen Lyme-Borreliose verleiht.

10. Immunogene Zusammensetzung nach einem der Ansprüche 8 oder 9, worin das Material Antigene aus OspA-Antigenen aus Borrelia-Stämmen B31, Orth, H4 und KL11 enthält.

11. Immunogene Zusammensetzung nach Anspruch 1 oder 3, worin das Material Antigene aus (i) oder (ii), gereinigt aus Lyme-Borrelien-Gesamtzellen enthält.

12. Immunogene Zusammensetzung nach Anspruch 1 oder 3, worin das Material Antigene aus (i) oder (ii) enthält, die gentechnologisch in einem eukaryontischen Wirt hergestellt und hieraus gereinigt werden.

13. Immunogene Zusammensetzung nach Anspruch 12, worin der Wirt eine Hefe ist.

14. Immunogene Zusammensetzung nach Anspruch 13, worin der Wirt P. pastoris ist.

15. Immunogene Zusammensetzung nach Anspruch 1 oder 3, worin die Antigene ein oder mehrere Epitope mit einer Aminosäuresequenz, ausgewählt aus 
(1) VKLSESVASLSKAA;
(2) TDNDSKEAILKTNGT;
(3) KELTSPVVAETPKKR;
 
(4) FVLAVKEVETL;
(5) YAISTLITEKLKAL;
(6) PNLTEISKKITSDSNA;
(7) ASANSVKELTSPVV;
(8) SPVVAETPKKP;
(9) GKKIQQNNGLGA; und
(10) SPVVAESPKK,
 oder Varianten oder Nachahmer solcher Epitope umfassen, wobei die Varianten oder Nachahmer eine solche Struktur haben, daß sie ausreichend ähnlich dem nativen Epitop zur Induktion des Aufbaus einer Schutzimmunität ist.

16. Immunogene Zusammensetzung nach Anspruch 15, worin jedes der Epitope (1) bis (10) vorhanden ist.

17. Immunogene Zusammensetzung nach Anspruch 15 oder 16, die ferner ein Adjuvans enthält.

18. Immunogene Zusammensetzung nach Anspruch 17, worin das Adjuvans Aluminiumhydroxid ist.

19. Immunogene Zusammensetzung nach einem der vorhergehenden Ansprüche, worin die protektive Menge im Bereich von 1 bis 100 µg pro Antigen pro Dosis ist.

20. Immunogene Zusammensetzung nach Anspruch 19, worin die protektive Menge im Bereich von 10 bis 50 µg pro Antigen pro Dosis ist.

21. Immunogene Zusammensetzung nach einem der vorhergehenden Ansprüche, worin der Säuger ein Mensch ist.

22. Immunogene Zusammensetzung nach einem der vorhergehenden Ansprüche, worin die protektive Menge entweder präventativ oder lindernd für Lyme-Borreliose ist.

23. Rekombinante DNA-Sequenz, umfassend eine Nukleotidsequenz aus einer der OspC-Gen-Sequenzen aus den Stämmen 28691, 25015, SZ7, 297, SIMON, E61, ACA1, H9, J1, VS461, M57, W, VSDA, NBS23a, 20047, KL10, IP90, NBS1AB, BITS, KL11 und H13, wie in Fig. 8a gezeigt.

24. Rekombinante DNA-Sequenz kodierend für OspC-Antigene der Stämme 28691, 25015, SZ7, 297, SIMON, E61, ACA1, H9, J1, VS461, M57, W, VSDA, NBS23a, 20047, KL10, IP90, NBS1AB, BITS, KL11 und H13, wie in Fig. 9a gezeigt.

25. Rekombinante DNA-Sequenz gemäß Anspruch 23, worin die DNA-Sequenz mindestens 80 % homolog zu einer der OspC-Gen-Sequenzen der Fig. 8a ist.

26. Rekombinanter Expressionsvektor, umfassend die codierende Sequenz eines OspC-Antigens von einem der 20 zur Zeit bekannten OspC-Familien der Fig. 11.

27. Rekombinanter Expressionsvektor nach Anspruch 26, worin der Vektor eine rekombinante DNA-Sequenz nach einem der Ansprüche 23 bis 25 umfaßt.

28. Expressionsvektor nach Anspruch 26 oder 27, worin der Vektor ein Shuttle-Vektor für eukaryontische/prokaryontische Wirtszellen ist.

29. Expressionsvektor nach Anspruch 28, worin der Vektor sich in Hefe, vorzugsweise in P. pastoris, und in E. coli repliziert.

30. Expressionsvektor nach einem der Ansprüche 26 bis 29, worin die Expression des OspC-Gens unter der transkriptionalen Kontrolle eines induzierbaren Promotors steht.

31. Expressionsvektor nach Anspruch 30, worin die Expression der OspC-codierenden Sequenz durch Methanol induzierbar ist.

32. Wirtszelle, transformiert mit einem Expressionsvektor nach einem der Ansprüche 26 bis 31.

33. OspC-Antigen nach (i) kodiert durch eine der Sequenzen gemäß den Ansprüchen 23 bis 25, oder (ii) mit einer Homologie von mindestens 80 %.

34. Verwendung einer Kombination von Antigenen, wie von den immunogenen Zusammensetzungen nach einem der vorhergehenden Ansprüche umfaßt, zur Herstellung eines Impfstoffes für die Behandlung oder Prävention von Lyme-Borreliose in einem Säuger.

35. Verwendung nach Anspruch 34, worin die wirksame Menge im Bereich von 1 bis 100 µg pro Antigen pro Dosis ist.

36. Verwendung nach Anspruch 35, worin die wirksame Menge im Bereich von 10 bis 50 µg pro Antigen pro Dosis ist.

## Revendications

1. Composition immunogène comprenant :
(a) une quantité de matériau comprenant (i) un ou plusieurs antigènes OspC de Borrelia à l'origine de la maladie de Lyme essentiellement purifiés à partir de chacune des 20 familles d'OspC actuellement reconnues de la Figure 11, ou (ii) des variants d'OspC ou des motifs mimétiques d'OspC desdits antigènes OspC, lesdits variants ou motifs mimétiques d'OspC ayant une structure qui ressemble suffisamment à l'OspC natif pour induire la production d'immunité protectrice; et
(b) un excipient physiologiquement acceptable à cet effet, composition dans laquelle ladite quantité est suffisante pour induire, chez un mammifère sensible à la borréliose de Lyme, une réponse immune qui soit protectrice de la borréliose de Lyme.

2. Composition immunogène comprenant :
(a) une quantité de matériau comprenant (i) deux ou plusieurs antigènes OspC de Borrelia à l'origine de la maladie de Lyme provenant de chacune des 20 familles d'OspC actuellement reconnues de la Figure 11, ou (ii) des variants d'OspC ou des motifs mimétiques d'OspC desdits antigènes OspC, lesdits variants ou motifs mimétiques d'OspC ayant une structure qui ressemble suffisamment à l'OspC natif pour induire la production d'immunité protectrice; et
(b) un excipient physiologiquement acceptable à cet effet, composition dans laquelle ladite quantité est suffisante pour induire, chez un mammifère sensible à la borréliose de Lyme, une réponse immune qui soit protectrice de la borréliose de Lyme.

3. Composition immunogène comprenant :
(a) une quantité de matériau comprenant (i) un ou plusieurs antigènes OspC de Borrelia à l'origine de la maladie de Lyme essentiellement purifiés à partir de chacune des familles d'OspC de la Figure 19 exprimées par un ou plusieurs clones et groupes clonaux associés à la maladie humaine (HDA), ou (ii) des variants d'OspC ou des motifs mimétiques d'OspC desdits antigènes OspC, lesdits variants d'OspC ou motifs mimétiques d'OspC ayant une structure qui ressemble suffisamment à l'OspC natif pour induire la production d'immunité protectrice; et
(b) un excipient physiologiquement acceptable à cet effet, composition dans laquelle ladite quantité est suffisante pour induire, chez un mammifère sensible à la borréliose de Lyme, une réponse immune qui soit protectrice de la borréliose de Lyme.

4. Composition immunogène, comprenant :
(a) une quantité de matériau comprenant (i) deux ou plusieurs antigènes de Borrelia à l'origine de la maladie de Lyme provenant de chacune des familles d'OspC de la Figure 19 exprimées par un ou plusieurs des clones et groupes clonaux associés à la maladie humaine (HDA), ou (ii) des variants d'OspC ou des motifs mimétiques d'OspC desdits antigènes OspC, lesdits variants d'OspC ou motifs mimétiques d'OspC ayant une structure qui ressemble suffisamment à l'OspC natif pour induire la production d'immunité protectrice ; et
(b) un excipient physiologiquement acceptable à cet effet, composition dans laquelle ladite quantité est suffisante pour induire, chez un mammifère sensible à la borréliose de Lyme, une réponse immune qui soit protectrice de la borréliose de Lyme.

5. Composition immunogène selon la revendication 1 ou 3 conçue pour protéger des souches de B. burgdorferi répandues dans une région géographique donnée.

6. Composition immunogène selon la revendication 5 pour utilisation en Amérique du Nord, comprenant
(a) une quantité de matériau comprenant (i) un ou plusieurs antigènes de Borrelia à l'origine de la maladie de Lyme essentiellement purifiés à partir de chacune des familles d'OspC 2 et 3 ou (ii) des variants d'OspC ou des motifs mimétiques d'OspC desdits antigènes OspC, lesdits variants d'OspC ou motifs mimétiques d'OspC ayant une structure qui ressemble suffisamment à l'OspC natif pour induire la production d'immunité protectrice ; et
(b) un excipient physiologiquement acceptable à cet effet, composition dans laquelle ladite quantité est suffisante pour induire, chez un mammifère sensible à la borréliose de Lyme, une réponse immune qui soit protectrice de la borréliose de Lyme.

7. Composition immunogène selon l'une quelconque des revendications précédentes, dans laquelle ledit matériau comprend en outre un antigène OspA dérivé de B. burgdorferi B31.

8. Composition immunogène selon la revendication 5 pour utilisation en Europe, comprenant :
(a) une quantité de matériau comprenant (i) un ou plusieurs antigènes OspC de Borrelia à l'origine de la maladie de Lyme essentiellement purifiés à partir de chacune des familles d'OspC 2, 4 - 7, 9, 10, 12 - 17 et 19 ou (ii) des variants d'OspC ou des motifs mimétiques d'OspC desdits antigènes OspC, lesdits variants d'OspC ou motifs mimétiques d'OspC ayant une structure qui ressemble suffisamment à l'OspC natif pour induire la production d'immunité protectrice; et
(b) un excipient physiologiquement acceptable à cet effet, composition dans laquelle ladite quantité est suffisante pour induire, chez un mammifère sensible à la borréliose de Lyme, une réponse immune qui soit protectrice de la borréliose de Lyme.

9. Composition immunogène selon la revendication 7 pour utilisation en Autriche, comprenant :
(a) une quantité de matériau comprenant (i) un ou plusieurs antigènes OspC de Borrelia à l'origine de la maladie de Lyme essentiellement purifiés à partir de chacune des familles d'OspC 2, 4 - 7, 10, 13 et 19 ou (ii) des variants d'OspC ou motifs mimétiques d'OspC desdits antigènes QspC, lesdits variants d'OspC ou motifs mimétiques d'OspC ayant une structure qui ressemble suffisamment à l'OspC natif pour induire la production d'immunité protectrice; et
(b) un excipient physiologiquement acceptable à cet effet, composition dans laquelle ladite quantité est suffisante pour induire, chez un mammifère sensible à la borréliose de Lyme, une réponse immune qui soit protectrice de la borréliose de Lyme.

10. Composition immunogène selon l'une quelconque des revendications 8 ou 9, dans laquelle ledit matériau comprend des antigènes issus des antigènes OspA dérivés des souches de Borrelia B31, Orth, H4 et KL11.

11. Composition immunogène selon la revendication 1 ou 3, dans laquelle ledit matériau comprend des antigènes issus de (i) ou de (ii) purifiés à partir de cellules entières de Borrelia à l'origine de la maladie de Lyme.

12. Composition immunogène selon la revendication 1 ou 3, dans laquelle ledit matériau comprend des antigènes issus de (i) ou de (ii) qui sont produits par des procédés recombinants dans un hôte eucaryote et sont purifiés à partir de l'hôte en question.

13. Composition immunogène selon la revendication 12, dans laquelle ledit hôte est une levure.

14. Composition immunogène selon la revendication 13, dans laquelle ledit hôte est P. pastoris.

15. Composition immunogène selon la revendication 1 ou 3, dans laquelle lesdits antigènes comprennent un ou plusieurs épitopes répondant à une séquence d'acides aminés sélectionnée dans le groupe constitué de 
(1) VKLSESVASLSKAA;
(2) TDNDSKEAILKTNGT;
(3) KELTSPVVAETPKKP;
(4) FVLAVKEVETL;
(5) YAISTLITEKLKAL;
(6) PNLTEISKKITDSNA;
(7) ASANSVKELTSPVV;
(8) SPVVAETPKKP;
(9) GKKIQQNNGLGA; et
(10) SPVVAESPKK;
 ou des variants ou motifs mimétiques de tels épitopes, de tels variants ou motifs mimétiques ayant une structure qui ressemble suffisamment à l'épitope natif pour induire la production d'immunité protectrice.

16. Composition immunogène selon la revendication 15, dans laquelle chacun des épitopes (1) à (10) est représenté.

17. Composition immunogène selon la revendication 15 ou 16, comprenant en outre un adjuvant.

18. Composition immunogène selon la revendication 17, dans laquelle ledit adjuvant est de l'hydroxyde d'aluminium.

19. Composition immunogène selon l'une quelconque des revendications précédentes, dans laquelle ladite quantité protectrice est comprise dans la gamme de 1 à 100 µg par type d'antigène par dose.

20. Composition immunogène selon la revendication 19, dans laquelle ladite quantité protectrice est comprise dans la gamme de 10 à 50 µg par type d'antigène par dose.

21. Composition immunogène selon l'une quelconque des revendications précédentes, dans laquelle ledit mammifère est l'homme.

22. Composition immunogène selon l'une quelconque des revendications précédentes, dans laquelle ladite quantité protectrice a une action préventive ou thérapeutique sur la borréliose de Lyme.

23. Séquence d'ADN recombinante comprenant une séquence nucléotidique de l'une quelconque des séquences de gènes OspC dérivées des souches 28691, 25015, SZ7, 297, SIMON, E61, ACA1, H9, J1, VS461, M57, W, VSDA, NBS23a, 20047, KL10, IP90, NBS1AB, BITS, KL11, et H13 comme indiqué à la Figure 8a.

24. Séquence d'ADN recombinante codant pour des antigènes OspC des souches 28691, 25015, SZ7, 297, SIMON, E61, ACA1, H9, J1, VS461, M57, W, VSDA, NBS23a, 20047, KL10, IP90, NBS1AB, BITS, KL11 et H13 comme indiqué à la Figure 9a.

25. Séquence d'ADN recombinante selon la revendication 23, dans laquelle ladite séquence d'ADN présente un degré d'homologie au moins égal à 80% à l'une quelconque des séquences de gènes OspC de la Figure 8a.

26. Vecteur d'expression recombinant comprenant la séquence codante d'un antigène OspC de l'une quelconque des 20 familles d'OspC actuellement reconnues de la Figure 11.

27. Vecteur d'expression recombinant selon la revendication 26, dans lequel ledit vecteur d'expression comprend une séquence d'ADN recombinante selon les revendications 23 à 25.

28. Vecteur d'expression selon la revendication 26 ou 27, dans lequel ledit vecteur est un vecteur navette utilisable pour des cellules hôtes eucaryotes/procaryotes.

29. Vecteur d'expression selon la revendication 28, dans lequel ledit vecteur se reproduit dans les levures, de préférence dans P. pastoris et dans E. coli.

30. Vecteur d'expression selon l'une quelconque des revendications 26 à 29, dans lequel l'expression du gène OspC est sous le contrôle transcriptionnel d'un promoteur inductible.

31. Vecteur d'expression selon la revendication 30, dans lequel l'expression de la séquence codante pour OspC est inductible par le méthanol.

32. Cellule hôte transformée par un vecteur d'expression selon l'une quelconque des revendications 26 à 31.

33. Antigène OspC tel que :
(i) encodé par l'une quelconque des séquences selon les revendications 23 à 25, ou
(ii) ayant un degré d'homologie d'au moins 80%.

34. Utilisation d'une combinaison d'antigènes comme ceux compris dans les compositions immunogènes de l'une quelconque des revendications précédentes pour l'obtention d'un vaccin destiné au traitement ou à la prévention de la borréliose de Lyme chez un mammifère.

35. Utilisation selon la revendication 34, dans laquelle ladite quantité efficace est comprise dans la gamme de 1 à 100 µg par type d'antigène par dose.

36. Utilisation selon la revendication 35, dans laquelle ladite quantité efficace est comprise dans la gamme de 10 à 50 µg par type d'antigène par dose.
